# EUROPEAN PATENT SPECIFICATION

(11) **EP 2 352 021 B1**
(45) Date of publication and mention of the grant of the patent: **04.05.2016**
(21) Application number: 09815923.9
(22) Date of filing: 28.09.2009
(51) Int. Cl.: G01N 27/62, C07K 14/47, G01N 33/68

(54) **PEPTIDE FOR USE IN SIMULTANEOUS PROTEIN QUANTIFICATION OF METABOLIZING ENZYMES USING MASS SPECTROMETRIC ANALYSIS APPARATUS**
PEPTID ZUR VERWENDUNG BEI EINER SIMULTANEN PROTEINQUANTIFIZIERUNG VON STOFFWECHSELENZYMEN MIT EINEM MASSENSPEKTROMETRIE-ANALYSEGERÄT
PEPTIDE POUR UNE UTILISATION DANS UNE QUANTIFICATION PROTÉIQUE SIMULTANÉE D'ENZYMES DE MÉTABOLISATION À L'AIDE D'UN APPAREIL D'ANALYSE PAR SPECTROMÉTRIE DE MASSE

(30) Priority: 29.09.2008 JP 2008251212
(43) Date of publication of application: 03.08.2011
(73) Proprietor: Tohoku University, Aoba-ku Sendai-shi Miyagi 980-8577 (JP)
(72) Inventor: KAMIIE, Junichi, Sendai-shi Miyagi 980-8577 (JP); OHTSUKI, Sumio, Sendai-shi Miyagi 980-8577 (JP); TERASAKI, Tetsuya, Sendai-shi Miyagi 980-8577 (JP)
(74) Representative: D Young & Co LLP
(86) International application number: PCT/JP2009/004948
(87) International publication number: WO 2010/035504

(56) References cited:
- WO-A1-2007/055116
- WO-A2-2005/114700
- WO-A2-2007/127767
- WO-A2-2007/140291
- JP-T- 2007 538 262
- US-A1- 2007 092 926
- LANE CATHERINE S ET AL: "Comparative cytochrome P450 proteomics in the livers of immunodeficient mice using 18O stable isotope labeling.", MOLECULAR & CELLULAR PROTEOMICS : MCP JUN 2007 LNKD- PUBMED:17296599, vol. 6, no. 6, June 2007 (2007-06), pages 953-962, XP002668749, ISSN: 1535-9476
- FAGEN ZHANG ET AL.: 'Quantitation of human glutathione S-transferases in complex matrices by liquid chromatography/tandem mass spectrometry with signature peptides' RAPID COMMUNICATIONS IN MASS SPECTROMETRY vol. 18, no. ISS.4, 2004, pages 491 - 498, XP002360988
- XIAOTAO DUAN ET AL.: 'Precolumn derivatization of cysteine residues for quantitative analysis of five major cytochrome P450 isoenzymes by liquid chromatography/tandem mass spectrometry' RAPID COMMUNICATIONS IN MASS SPECTROMETRY vol. 21, no. ISS.20, 2007, pages 3234 - 3244, XP008144332

### SEQUENCE LISTING

<110> TOHOKU UNIVERSITY
<120> Peptide for simultaneous quantitative protein assay of metabolizing
   enzymes by mass spectrometry
<130> P20080035
<150> JP2008-251212 <151> 2008-09-29
<160> 697
<170> PatentIn version 3.1
<210> 1
   <211> 12
   <212> PRT
   <213> Homo sapiens
<400> 1
   Leu Trp Val Asn Pro Ser Glu Phe Leu Pro Glu Arg
   1               5                   10
<210> 2
   <211> 14
   <212> PRT
   <213> Homo sapiens
<400> 2
   Gln Leu Asp Glu Asn Ala Asn Val Gln Leu Ser Asp Glu Lys
   1               5                   10
<210> 3
   <211> 11
   <212> PRT
   <213> Homo sapiens
<400> 3
   Tyr Leu Pro Asn Pro Ser Leu Asn Ala Phe Lys
   1 5 10
<210> 4
   <211> 10
   <212> PRT
   <213> Homo sapiens
<400> 4
   Phe Leu Thr Pro Asp Gly Ala Ile Asp Lys
   1 5 10
<210> 5
   <211> 8
   <212> PRT
   <213> Homo sapiens
<400> 5
   Ala Ser Arg Pro Gln Val Pro Lys
   1 5
<210> 6
   <211> 11
   <212> PRT
   <213> Homo sapiens
<400> 6
   Thr Val Gln Glu His Tyr Gln Asp Phe Asp Lys
   1 5 10
<210> 7
   <211> 9
   <212> PRT
   <213> Homo sapiens
<400> 7
   Tyr Leu Pro Asn Pro Ala Leu Gln Arg
   1 5
<210> 8
   <211> 7
   <212> PRT
   <213> Homo sapiens
<400> 8
   Phe Leu Trp Phe Leu Gln Lys
   1 5
<210> 9
   <211> 7
   <212> PRT
   <213> Homo sapiens
<400> 9
   Tyr Pro Asp Val Gln Thr Arg
   1 5
<210> 10
   <211> 12
   <212> PRT
   <213> Homo sapiens
<400> 10
   Gln Val Leu Glu Gly His Val Leu Ser Glu Ala Arg
   1 5 10
<210> 11
   <211> 8
   <212> PRT
   <213> Homo sapiens
<400> 11
   Glu Leu Val Ala Leu Leu Val Arg
   1 5
<210> 12
   <211> 9
   <212> PRT
   <213> Homo sapiens
<400> 12
   Tyr Ser His Asp Asp Pro Glu Phe Arg
   1 5
<210> 13
   <211> 8
   <212> PRT
   <213> Homo sapiens
<400> 13
   Ala Asn Pro Asn Glu Pro Ala Lys
   1 5
<210> 14
   <211> 11
   <212> PRT
   <213> Homo sapiens
<400> 14
   Glu Leu Leu Ser His Asn Glu Glu Phe Gly Arg
   1               5                   10
<210> 15
   <211> 15
   <212> PRT
   <213> Homo sapiens
<400> 15
   Gly Thr Gly Gly Ala Asn Ile Asp Pro Thr Phe Phe Leu Ser Arg
   1               5                   10                  15
<210> 16
   <211> 11
   <212> PRT
   <213> Homo sapiens
<400> 16
   Gly Tyr Gly Val Val Phe Ser Asn Gly Glu Arg
   1               5                   10
<210> 17
   <211> 13
   <212> PRT
   <213> Homo sapiens
<400> 17
   Ile Gln Glu Glu Ala Gly Phe Leu Ile Asp Ala Leu Arg
   1               5                   10
<210> 18
<211> 13
   <212> PRT
   <213> Homo sapiens
<400> 18
   Ile Gln Glu Glu Ser Gly Phe Leu Ile Glu Ala Ile Arg
   1               5                   10
<210> 19
   <211> 7
   <212> PRT
   <213> Homo sapiens
<400> 19
   Phe Ala Ile Ala Thr Leu Arg
   1 5
<210> 20
   <211> 15
   <212> PRT
   <213> Homo sapiens
<400> 20
   Gly Thr His Gly Ala Asn Ile Asp Pro Thr Phe Phe Leu Ser Arg
   1 5 10 15
<210> 21
   <211> 11
   <212> PRT
   <213> Homo sapiens
<400> 21
   Gly Glu Gln Ala Thr Phe Asp Trp Leu Phe Lys
   1 5 10
<210> 22
   <211> 7
   <212> PRT
   <213> Homo sapiens
<400> 22
   Ala Glu Ala Phe Ser Gly Arg
   1 5
<210> 23
   <211> 7
   <212> PRT
   <213> Homo sapiens
<400> 23
   Tyr Pro His Val Ala Glu Arg
   1 5
<210> 24
   <211> 7
   <212> PRT
<213> Homo sapiens
<400> 24
   Tyr Phe Pro Gly Ala His Arg
   1 5
<210> 25
   <211> 11
   <212> PRT
   <213> Homo sapiens
<400> 25
   Gly Tyr Gly Val Ile Phe Ala Asn Gly Asn Arg
   1 5 10
<210> 26
   <211> 11
   <212> PRT
   <213> Homo sapiens
<400> 26
   Val Gln Glu Glu Ile Asp His Val Ile Gly Arg
   1 5 10
<210> 27
   <211> 12
   <212> PRT
   <213> Homo sapiens
<400> 27
   Glu His Gln Ala Ser Leu Asp Val Asn Asn Pro Arg
   1 5 10
<210> 28
   <211> 8
   <212> PRT
   <213> Homo sapiens
<400> 28
   Gly Asn Ser Pro Ile Ser Gln Arg
   1 5
<210> 29
   <211> 13
   <212> PRT
   <213> Homo sapiens
<400> 29
   Glu Ala Leu Ile Asp Asn Gly Glu Glu Phe Ser Gly Arg
   1 5 10
<210> 30
   <211> 8
   <212> PRT
   <213> Homo sapiens
<400> 30
   Gly Ile Phe Pro Leu Ala Glu Arg
   1 5
<210> 31
   <211> 16
   <212> PRT
   <213> Homo sapiens
<400> 31
   Gly Thr Thr Ile Leu Ile Ser Leu Thr Ser Val Leu His Asp Asn Lys
   1 5 10 15
<210> 32
   <211> 9
   <212> PRT
   <213> Homo sapiens
<400> 32
   Ile Ala Glu Asn Phe Ala Tyr Ile Lys
   1 5
<210> 33
   <211> 13
   <212> PRT
   <213> Homo sapiens
<400> 33
   Glu Ala Leu Ile Asp His Gly Glu Glu Phe Ser Gly Arg
   1 5 10
<210> 34
   <211> 7
   <212> PRT
   <213> Homo sapiens
<400> 34
   Tyr Pro Glu Val Thr Ala Lys
   1 5
<210> 35
   <211> 8
   <212> PRT
   <213> Homo sapiens
<400> 35
   Gly His Phe Pro Leu Ala Glu Arg
   1 5
<210> 36
   <211> 14
   <212> PRT
   <213> Homo sapiens
<400> 36
   Ile Tyr Gly Pro Val Phe Thr Leu Tyr Phe Gly Leu Glu Arg
   1 5 10
<210> 37
   <211> 16
   <212> PRT
   <213> Homo sapiens
<400> 37
   Gly Thr Thr Ile Leu Thr Ser Leu Thr Ser Val Leu His Asp Asn Lys
   1 5 10 15
<210> 38
   <211> 8
   <212> PRT
   <213> Homo sapiens
<400> 38
   Ser Gln Gly Val Phe Leu Ala Arg
   1 5
<210> 39
   <211> 8
   <212> PRT
   <213> Homo sapiens
<400> 39
   Asp Ile Glu Val Gln Gly Phe Arg
   1 5
<210> 40
   <211> 15
   <212> PRT
   <213> Homo sapiens
<400> 40
   Phe Ile Thr Leu Val Pro Ser Asn Leu Pro His Glu Ala Thr Arg
   1 5 10 15
<210> 41
   <211> 7
   <212> PRT
   <213> Homo sapiens
<400> 41
   Glu Ala Leu Leu Asp Tyr Lys
   1 5
<210> 42
   <211> 7
   <212> PRT
   <213> Homo sapiens
<400> 42
   Tyr Pro Glu Ile Glu Glu Lys
   1 5
<210> 43
   <211> 10
   <212> PRT
   <213> Homo sapiens
<400> 43
   Gly Asp Leu Pro Ala Phe His Ala His Arg
   1 5 10
<210> 44
   <211> 11
   <212> PRT
   <213> Homo sapiens
<400> 44
   Gly Ile Ile Phe Asn Asn Gly Pro Thr Trp Lys
   1 5 10
<210> 45
   <211> 12
   <212> PRT
   <213> Homo sapiens
<400> 45
   Phe Lys Pro Glu His Phe Leu Asn Glu Asn Gly Lys
   1 5 10
<210> 46
   <211> 7
   <212> PRT
   <213> Homo sapiens
<400> 46
   Leu His Glu Glu Ile Asp Arg
   1 5
<210> 47
   <211> 11
   <212> PRT
   <213> Homo sapiens
<400> 47
   Val Gln Glu Glu Ile Asp Leu Val Val Gly Arg
   1 5 10
<210> 48
   <211> 13
   <212> PRT
   <213> Homo sapiens
<400> 48
   Val Ile Gly Gln Gly Gln Gln Pro Ser Thr Ala Ala Arg
   1 5 10
<210> 49
   <211> 8
   <212> PRT
   <213> Homo sapiens
<400> 49
   Asp Phe Ile Asp Ala Tyr Leu Lys
   1 5
<210> 50
   <211> 15
   <212> PRT
   <213> Homo sapiens
<400> 50
   Glu Val Thr Val Asp Thr Thr Leu Ala Gly Tyr His Leu Pro Lys
   1 5 10 15
<210> 51
   <211> 13
   <212> PRT
   <213> Homo sapiens
<400> 51
   Ile Gln Glu Glu Ala Gln His Leu Thr Glu Ala Ile Lys
   1 5 10
<210> 52
   <211> 12
   <212> PRT
   <213> Homo sapiens
<400> 52
   Glu Glu Asn Gly Gln Pro Phe Asp Pro His Phe Lys
   1 5 10
<210> 53
   <211> 12
   <212> PRT
   <213> Homo sapiens
<400> 53
   Leu Leu Asp Glu Val Thr Tyr Leu Glu Ala Ser Lys
   1 5 10
<210> 54
   <211> 10
   <212> PRT
   <213> Homo sapiens
<400> 54
   Phe Thr Phe Arg Pro Pro Asn Asn Glu Lys
   1 5 10
<210> 55
   <211> 13
<212> PRT
   <213> Homo sapiens
<400> 55
   Phe Glu Tyr Gln Asp Ser Trp Phe Gln Gln Leu Leu Lys
   1 5 10
<210> 56
   <211> 16
   <212> PRT
   <213> Homo sapiens
<400> 56
   Phe His Leu His Phe Pro His Glu Leu Val Pro Asp Leu Lys Pro Arg
   1 5 10 15
<210> 57
   <211> 7
   <212> PRT
   <213> Homo sapiens
<400> 57
   Gly Arg Pro Phe Asp Phe Lys
   1 5
<210> 58
   <211> 9
   <212> PRT
   <213> Homo sapiens
<400> 58
   Asp Pro Glu Val Phe His Pro Glu Arg
   1 5
<210> 59
   <211> 16
   <212> PRT
   <213> Homo sapiens
<400> 59
   Gly Thr Thr Val Ile Thr Asn Leu Tyr Ser Val His Phe Asp Glu Lys
   1 5 10 15
<210> 60
   <211> 7
   <212> PRT
   <213> Homo sapiens
<400> 60
   Tyr Phe Gly Tyr Gly Gln Lys
   1 5
<210> 61
   <211> 9
   <212> PRT
   <213> Homo sapiens
<400> 61
   Asn Asp Pro Ser Ser Thr Phe Ser Lys
   1               5
<210> 62
   <211> 10
   <212> PRT
   <213> Homo sapiens
<400> 62
   Phe Phe Asn Asp Ala Ile Glu Thr Tyr Lys
   1               5                   10
<210> 63
   <211> 11
   <212> PRT
   <213> Homo sapiens
<400> 63
   Asn Ala Ala Val Val Tyr Asp Phe Leu Ser Arg
   1               5                   10
<210> 64
   <211> 7
   <212> PRT
   <213> Homo sapiens
<400> 64
   Glu Phe Gln Ala Val Val Arg
   1               5
<210> 65
   <211> 9
   <212> PRT
   <213> Homo sapiens
<400> 65
   Asp Leu Val Asp Ala Phe Leu Leu Lys
   1               5
<210> 66
   <211> 9
   <212> PRT
   <213> Homo sapiens
<400> 66
   His Pro Glu Glu Phe Asn Pro Asp Arg
   1 5
<210> 67
   <211> 13
   <212> PRT
   <213> Homo sapiens
<400> 67
   Glu Ala Leu Gly Gly Gln Ala Glu Glu Phe Ser Gly Arg
   1 5 10
<210> 68
   <211> 13
   <212> PRT
   <213> Homo sapiens
<400> 68
   Glu Leu Gly Ala Gly Gln Ala Pro Ser Leu Gly Asp Arg
   1 5 10
<210> 69
   <211> 15
   <212> PRT
   <213> Homo sapiens
<400> 69
   Gln Leu Leu His His Val Ser Thr Leu Ala Ala Phe Thr Val Arg
   1 5 10 15
<210> 70
   <211> 7
   <212> PRT
   <213> Homo sapiens
<400> 70
   Phe Leu Asp Ala Asp Gly Arg
   1 5
<210> 71
   <211> 16
   <212> PRT
   <213> Homo sapiens
<400> 71
   Gln Val Gln Gln His Gln Gly Asn Leu Asp Ala Ser Gly Pro Ala Arg
   1 5 10 15
<210> 72
   <211> 13
   <212> PRT
   <213> Homo sapiens
<400> 72
   Leu Pro Tyr Thr Asp Ala Val Leu His Glu Ala Gln Arg
   1 5 10
<210> 73
   <211> 9
   <212> PRT
   <213> Homo sapiens
<400> 73
   Ala Leu His Ser Leu Gly Val Gly Arg
   1 5
<210> 74
   <211> 11
   <212> PRT
   <213> Homo sapiens
<400> 74
   Gly Gly Gly Ile Phe Phe Ser Ser Gly Ala Arg
   1 5 10
<210> 75
   <211> 10
   <212> PRT
   <213> Homo sapiens
<400> 75
   Phe Ile Thr Leu Leu Pro His Val Pro Arg
   1 5 10
<210> 76
   <211> 11
   <212> PRT
   <213> Homo sapiens
<400> 76
   Thr Val Val Leu Thr Gly Phe Glu Ala Val Lys
   1 5 10
<210> 77
   <211> 7
   <212> PRT
   <213> Homo sapiens
<400> 77
   His Pro Asp Val Gln Gly Arg
   1 5
<210> 78
   <211> 13
   <212> PRT
   <213> Homo sapiens
<400> 78
   Thr Glu Leu Phe Leu Leu Phe Ala Gly Leu Leu Gln Arg
   1 5 10
<210> 79
   <211> 11
   <212> PRT
   <213> Homo sapiens
<400> 79
   Tyr Gly Pro Val Phe Thr Val His Leu Gly Arg
   1 5 10
<210> 80
   <211> 11
   <212> PRT
   <213> Homo sapiens
<400> 80
   Ser Leu Leu Ser Pro Thr Phe Thr Ser Gly Lys
   1 5 10
<210> 81
   <211> 12
   <212> PRT
   <213> Homo sapiens
<400> 81
   Leu Gln Glu Glu Ile Asp Ala Val Leu Pro Asn Lys
   1 5 10
<210> 82
   <211> 7
   <212> PRT
   <213> Homo sapiens
<400> 82
   Asp Gly Thr Leu Ser Gly Glu
   1               5
<210> 83
   <211> 8
   <212> PRT
   <213> Homo sapiens
<400> 83
   Asp Thr Ile Asn Phe Leu Ser Lys
   1               5
<210> 84
   <211> 12
   <212> PRT
   <213> Homo sapiens
<400> 84
   Tyr Trp Thr Glu Pro Glu Glu Phe Arg Pro Glu Arg
   1               5                   10
<210> 85
   <211> 12
   <212> PRT
   <213> Homo sapiens
<400> 85
   Phe Asn Pro Leu Asp Pro Phe Val Leu Ser Ile Lys
   1               5                   10
<210> 86
<211> 7
   <212> PRT
   <213> Homo sapiens
<400> 86
   Asp Glu Thr Val Ser Gly Ala
   1               5
<210> 87
   <211> 9
   <212> PRT
   <213> Homo sapiens
<400> 87
   Glu Ile Asp Thr Val Leu Pro Asn Lys
   1 5
<210> 88
   <211> 14
   <212> PRT
   <213> Homo sapiens
<400> 88
   Phe Gly Gly Leu Leu Leu Thr Glu Lys Pro Ile Val Leu Lys
   1 5 10
<210> 89
   <211> 7
   <212> PRT
   <213> Homo sapiens
<400> 89
   Leu Phe Pro Val Val Ser Arg
   1 5
<210> 90
   <211> 11
   <212> PRT
   <213> Homo sapiens
<400> 90
   Thr Leu Leu Ser Pro Ala Phe Thr Ser Val Lys
   1 5 10
<210> 91
   <211> 9
   <212> PRT
   <213> Homo sapiens
<400> 91
   Tyr Ile Pro Phe Gly Ala Gly Pro Arg
   1 5
<210> 92
   <211> 7
   <212> PRT
<213> Homo sapiens
<400> 92
   Asp Ser Ile Asp Leu Tyr Arg
   1 5
<210> 93
   <211> 7
   <212> PRT
   <213> Homo sapiens
<400> 93
   Gly Leu Trp Asn Phe Asp Arg
   1 5
<210> 94
   <211> 7
   <212> PRT
   <213> Homo sapiens
<400> 94
   Gln Glu Ala Glu Asn Ser Lys
   1 5
<210> 95
   <211> 7
   <212> PRT
   <213> Homo sapiens
<400> 95
   Phe Ala Leu Thr Asn Ile Lys
   1 5
<210> 96
   <211> 16
   <212> PRT
   <213> Homo sapiens
<400> 96
   Leu Asp Asn Leu Pro Ile Leu Gln Pro Glu Lys Pro Ile Val Leu Lys
   1 5 10 15
<210> 97
   <211> 8
   <212> PRT
   <213> Homo sapiens
<400> 97
   Ala Val Gln Leu Tyr Leu His Arg
   1 5
<210> 98
   <211> 9
   <212> PRT
   <213> Homo sapiens
<400> 98
   Val Ala Thr Ala Leu Thr Leu Leu Arg
   1               5
<210> 99
   <211> 9
   <212> PRT
   <213> Homo sapiens
<400> 99
   Phe Glu Leu Leu Pro Asp Pro Thr Arg
   1               5
<210> 100
   <211> 7
   <212> PRT
   <213> Homo sapiens
<400> 100
   Ile Pro Ile Pro Ile Ala Arg
   1               5
<210> 101
   <211> 12
   <212> PRT
   <213> Homo sapiens
<400> 101
   Glu Leu Ser Thr Pro Val Thr Phe Pro Asp Gly Arg
   1               5                   10
<210> 102
   <211> 16
   <212> PRT
   <213> Homo sapiens
<400> 102
   Asn Gly Phe His Leu His Leu Lys Pro Leu Gly Pro Gly Ser Gly Lys
   1               5                   10                  15
<210> 103
   <211> 16
   <212> PRT
   <213> Homo sapiens
<400> 103
   Glu Ile Leu Gly Asp Gln Asp Phe Phe Gln Trp Asp Asp Leu Gly Lys
   1 5 10 15
<210> 104
   <211> 10
   <212> PRT
   <213> Homo sapiens
<400> 104
   Leu Tyr Pro Pro Val Pro Gln Val Tyr Arg
   1 5 10
<210> 105
   <211> 15
   <212> PRT
   <213> Homo sapiens
<400> 105
   Asn Ser Ala Val Trp Pro Asp Pro Glu Val Phe Asp Ser Leu Arg
   1 5 10 15
<210> 106
   <211> 8
   <212> PRT
   <213> Homo sapiens
<400> 106
   Gly Asp Thr Gly Leu Gly His Arg
   1 5
<210> 107
   <211> 9
   <212> PRT
   <213> Homo sapiens
<400> 107
   Phe Glu Phe Ser Leu Asp Pro Ser Arg
   1 5
<210> 108
   <211> 7
   <212> PRT
   <213> Homo sapiens
<400> 108
   Leu Ser Asp Ala Asp Leu Arg
   1 5
<210> 109
   <211> 9
   <212> PRT
   <213> Homo sapiens
<400> 109
   Gly Leu Leu Val Leu Glu Gly Pro Lys
   1 5
<210> 110
   <211> 14
   <212> PRT
   <213> Homo sapiens
<400> 110
   Ala Pro Asp Val Tyr Asp Phe Phe Leu Gln Trp Ile Gly Arg
   1 5 10
<210> 111
   <211> 12
   <212> PRT
   <213> Homo sapiens
<400> 111
   Gln Leu Ser Lys Pro Val Thr Phe Val Asp Gly Arg
   1 5 10
<210> 112
   <211> 7
   <212> PRT
   <213> Homo sapiens
<400> 112
   Phe Asp Pro Glu Asn Ile Lys
   1 5
<210> 113
   <211> 12
   <212> PRT
   <213> Homo sapiens
<400> 113
   His Val Thr Gln Asp Ile Val Leu Pro Asp Gly Arg
   1 5 10
<210> 114
   <211> 14
   <212> PRT
   <213> Homo sapiens
<400> 114
   Val Leu Thr Gln Leu Val Ala Thr Tyr Pro Gln Gly Phe Lys
   1               5                   10
<210> 115
   <211> 10
   <212> PRT
   <213> Homo sapiens
<400> 115
   Leu His Pro Pro Val Pro Ala Val Ser Arg
   1               5                   10
<210> 116
   <211> 8
   <212> PRT
   <213> Homo sapiens
<400> 116
   Ala Glu Asp Gly Leu Trp Leu Arg
   1               5
<210> 117
   <211> 10
   <212> PRT
   <213> Homo sapiens
<400> 117
   Leu His Pro Pro Ile Pro Thr Phe Ala Arg
   1               5                   10
<210> 118
   <211> 15
   <212> PRT
   <213> Homo sapiens
<400> 118
   Val Leu Thr Gln Leu Val Ala Thr Tyr Pro Gln Gly Phe Val Arg
   1               5                   10                  15
<210> 119
   <211> 14
   <212> PRT
   <213> Homo sapiens
<400> 119
   Thr Leu Asp Phe Ile Asp Val Leu Leu Leu Ser Glu Asp Lys
   1 5 10
<210> 120
   <211> 11
   <212> PRT
   <213> Homo sapiens
<400> 120
   Val Val Leu Ala Leu Thr Leu Leu His Phe Arg
   1 5 10
<210> 121
   <211> 7
   <212> PRT
   <213> Homo sapiens
<400> 121
   Phe Asn Gln Glu Asn Ile Lys
   1 5
<210> 122
   <211> 14
   <212> PRT
   <213> Homo sapiens
<400> 122
   Thr Leu Thr Gln Leu Val Thr Thr Tyr Pro Gln Gly Phe Lys
   1 5 10
<210> 123
   <211> 7
<212> PRT
   <213> Homo sapiens
<400> 123
   Phe Asp Pro Glu Asn Ser Lys
   1 5
<210> 124
   <211> 8
   <212> PRT
   <213> Homo sapiens
<400> 124
   Ala Leu Ser Asp Glu Asp Ile Arg
   1               5
<210> 125
   <211> 11
   <212> PRT
   <213> Homo sapiens
<400> 125
   Leu Val His Asp Phe Thr Asp Ala Val Ile Arg
   1               5                   10
<210> 126
   <211> 8
   <212> PRT
   <213> Homo sapiens
<400> 126
   Ser Tyr Ile Thr Ile Phe Asn Lys
   1               5
<210> 127
   <211> 10
   <212> PRT
   <213> Homo sapiens
<400> 127
   Leu His Pro Pro Ala Pro Phe Ile Ser Arg
   1               5                   10
<210> 128
   <211> 9
   <212> PRT
   <213> Homo sapiens
<400> 128
   Gln Gln Gly Ala Glu Ala Trp Leu Lys
   1               5
<210> 129
   <211> 8
   <212> PRT
   <213> Homo sapiens
<400> 129
   Thr Glu Asn Gly Leu Trp Leu Lys
   1               5
<210> 130
   <211> 9
   <212> PRT
   <213> Homo sapiens
<400> 130
   Leu Leu His Leu Leu Gly Leu Glu Lys
   1               5
<210> 131
   <211> 9
   <212> PRT
   <213> Homo sapiens
<400> 131
   Val Val Val Ala Leu Thr Leu Leu Arg
   1               5
<210> 132
   <211> 13
   <212> PRT
   <213> Homo sapiens
<400> 132
   Ser Pro Leu Ala Tyr Val Pro Phe Ser Ala Gly Pro Arg
   1               5                   10
<210> 133
   <211> 11
   <212> PRT
   <213> Homo sapiens
<400> 133
   Leu His His Tyr Leu Asp Phe Ile Tyr Tyr Arg
   1               5                   10
<210> 134
   <211> 10
   <212> PRT
   <213> Homo sapiens
<400> 134
   Gln Tyr Pro Pro Val Thr Leu Val Ser Arg
   1               5                   10
<210> 135
   <211> 12
   <212> PRT
   <213> Homo sapiens
<400> 135
   Thr Leu Asp Phe Ile Asp Val Leu Leu Leu Ala Arg
   1 5 10
<210> 136
   <211> 9
   <212> PRT
   <213> Homo sapiens
<400> 136
   Phe Asp Pro Asp Asn Pro Gln Gln Arg
   1 5
<210> 137
   <211> 13
   <212> PRT
   <213> Homo sapiens
<400> 137
   His Pro Tyr Ala Tyr Val Pro Phe Ser Ala Gly Pro Arg
   1 5 10
<210> 138
   <211> 9
   <212> PRT
   <213> Homo sapiens
<400> 138
   Phe Phe Pro Glu Asn Ala Gln Gly Arg
   1 5
<210> 139
   <211> 7
   <212> PRT
   <213> Homo sapiens
<400> 139
   Leu Ser His Glu Asp Ile Arg
   1 5
<210> 140
   <211> 10
   <212> PRT
   <213> Homo sapiens
<400> 140
   Leu Phe Pro Ser Val Pro Leu Phe Ala Arg
   1 5 10
<210> 141
   <211> 13
   <212> PRT
   <213> Homo sapiens
<400> 141
   Ile Leu His Thr Phe Thr Asn Ser Val Ile Ala Glu Arg
   1 5 10
<210> 142
   <211> 13
   <212> PRT
   <213> Homo sapiens
<400> 142
   Gly Thr Glu Ala Val Ile Ile Pro Tyr Ala Leu His Arg
   1 5 10
<210> 143
   <211> 12
   <212> PRT
   <213> Homo sapiens
<400> 143
   Tyr Phe Pro Asn Pro Glu Glu Phe Gln Pro Glu Arg
   1 5 10
<210> 144
   <211> 14
   <212> PRT
   <213> Homo sapiens
<400> 144
   Asn Ile Gly Ala Gln Ser Asn Asp Asp Ser Glu Tyr Val Arg
   1 5 10
<210> 145
   <211> 9
   <212> PRT
   <213> Homo sapiens
<400> 145
   Gly Leu Ala Ala Leu Asp Gly Pro Lys
   1               5
<210> 146
   <211> 9
   <212> PRT
   <213> Homo sapiens
<400> 146
   Phe Ser Gln Glu Asn Ser Asp Gln Arg
   1               5
<210> 147
   <211> 10
   <212> PRT
   <213> Homo sapiens
<400> 147
   Leu Ile Pro Ala Val Pro Ser Ile Ser Arg
   1               5                   10
<210> 148
   <211> 13
   <212> PRT
   <213> Homo sapiens
<400> 148
   His Pro Tyr Ala Tyr Leu Pro Phe Ser Ala Gly Ser Arg
   1               5                   10
<210> 149
   <211> 7
   <212> PRT
   <213> Homo sapiens
<400> 149
   Leu Glu Glu Ile Ile Glu Lys
   1               5
<210> 150
   <211> 8
   <212> PRT
   <213> Homo sapiens
<400> 150
   Phe Ser Pro Pro Leu Leu Gly Lys
   1 5
<210> 151
   <211> 7
   <212> PRT
   <213> Homo sapiens
<400> 151
   Ala Ile Phe Glu Leu Ser Lys
   1 5
<210> 152
   <211> 7
   <212> PRT
   <213> Homo sapiens
<400> 152
   Leu Ser Pro Gln Gly Tyr Arg
   1 5
<210> 153
   <211> 13
   <212> PRT
   <213> Homo sapiens
<400> 153
   Leu Tyr Ser Leu Leu Tyr His Ser Asp Ile Ile Phe Lys
   1 5 10
<210> 154
<211> 11
   <212> PRT
   <213> Homo sapiens
<400> 154
   Val Thr Ile Ala Leu Ile Leu Leu His Phe Arg
   1 5 10
<210> 155
   <211> 8
   <212> PRT
   <213> Homo sapiens
<400> 155
   Ile Leu Glu Ser Trp Val Gly Arg
   1 5
<210> 156
   <211> 12
   <212> PRT
   <213> Homo sapiens
<400> 156
   Leu Leu Asp Lys Pro Ile Thr Phe Pro Asp Gly Arg
   1 5 10
<210> 157
   <211> 7
   <212> PRT
   <213> Homo sapiens
<400> 157
   Glu Phe Glu Val Tyr His Lys
   1 5
<210> 158
   <211> 10
   <212> PRT
   <213> Homo sapiens
<400> 158
   Trp Glu Glu His Ile Ala Gln Asn Ser Arg
   1 5 10
<210> 159
   <211> 10
   <212> PRT
   <213> Homo sapiens
<400> 159
   Phe Ser Ser Gln Gly Gln Ile Phe Ser Lys
   1 5 10
<210> 160
   <211> 9
   <212> PRT
<213> Homo sapiens
<400> 160
   Gly Leu Val Thr Leu Asp Gly Ser Lys
   1 5
<210> 161
   <211> 12
   <212> PRT
   <213> Homo sapiens
<400> 161
   Asp Phe Ser Glu Ala Asp Leu Gln Ala Glu Val Lys
   1 5 10
<210> 162
   <211> 11
   <212> PRT
   <213> Homo sapiens
<400> 162
   Phe Asn Gln Glu Leu His Gln Phe Thr Glu Lys
   1 5 10
<210> 163
   <211> 13
   <212> PRT
   <213> Homo sapiens
<400> 163
   Leu Ala Pro Asp His Ser Arg Pro Pro Gln Pro Val Arg
   1 5 10
<210> 164
   <211> 9
   <212> PRT
   <213> Homo sapiens
<400> 164
   Val Ala Val Ala Leu Thr Leu Leu Arg
   1 5
<210> 165
   <211> 14
   <212> PRT
   <213> Homo sapiens
<400> 165
   Ile His Pro Tyr Ala Phe Ile Pro Phe Ser Ala Gly Leu Arg
   1 5 10
<210> 166
   <211> 8
   <212> PRT
   <213> Homo sapiens
<400> 166
   Leu Phe Ala Ile His Glu Ile Lys
   1 5
<210> 167
   <211> 13
   <212> PRT
   <213> Homo sapiens
<400> 167
   Tyr Val His Phe Ile Thr Asn Pro Leu Ser Tyr His Lys
   1 5 10
<210> 168
   <211> 7
   <212> PRT
   <213> Homo sapiens
<400> 168
   Tyr Leu Asp Glu Asn Gly Lys
   1 5
<210> 169
   <211> 15
   <212> PRT
   <213> Homo sapiens
<400> 169
   Ala Gly Leu Gly Ile Leu Pro Pro Leu Asn Asp Ile Glu Phe Lys
   1 5 10 15
<210> 170
   <211> 11
   <212> PRT
   <213> Homo sapiens
<400> 170
   Ala His Ile Leu Asn Asn Leu Asp Asn Phe Lys
   1 5 10
<210> 171
   <211> 9
   <212> PRT
   <213> Homo sapiens
<400> 171
   Leu Ser Ser Ala Ser Leu Asn Ile Arg
   1 5
<210> 172
   <211> 9
   <212> PRT
   <213> Homo sapiens
<400> 172
   Arg Pro Gly Glu Pro Pro Leu Ile Lys
   1 5
<210> 173
   <211> 13
   <212> PRT
   <213> Homo sapiens
<400> 173
   Gln His Gly Asp Thr Phe Thr Val Leu Leu Gly Gly Lys
   1 5 10
<210> 174
   <211> 15
   <212> PRT
   <213> Homo sapiens
<400> 174
   Leu Leu Phe Gly Ile Gln Tyr Pro Asp Ser Asp Val Leu Phe Arg
   1 5 10 15
<210> 175
   <211> 16
   <212> PRT
   <213> Homo sapiens
<400> 175
   Tyr Ile Thr Phe Ile Leu Asp Pro Phe Gln Tyr Gln Leu Val Ile Lys
   1 5 10 15
<210> 176
   <211> 9
   <212> PRT
   <213> Homo sapiens
<400> 176
   Ala Phe Ser Ile Ser Gln Leu Gln Lys
   1 5
<210> 177
   <211> 9
   <212> PRT
   <213> Homo sapiens
<400> 177
   Leu Ser Ser Tyr Ser Thr Thr Ile Arg
   1 5
<210> 178
   <211> 9
   <212> PRT
   <213> Homo sapiens
<400> 178
   Gln Val Phe Glu Pro Gln Leu Leu Lys
   1 5
<210> 179
   <211> 8
   <212> PRT
   <213> Homo sapiens
<400> 179
   Phe Phe Ala Leu Ser Glu Val Lys
   1 5
<210> 180
   <211> 7
   <212> PRT
   <213> Homo sapiens
<400> 180
   Ala Ala Pro Thr Leu Leu Arg
   1 5
<210> 181
   <211> 7
   <212> PRT
   <213> Homo sapiens
<400> 181
   Glu Trp Leu Glu Val Gly Arg
   1 5
<210> 182
   <211> 11
   <212> PRT
   <213> Homo sapiens
<400> 182
   Glu Glu Ala Thr Gln Val Leu Gly Glu Ala Arg
   1               5                   10
<210> 183
   <211> 9
   <212> PRT
   <213> Homo sapiens
<400> 183
   Leu Val His Glu Asp Tyr Thr Leu Lys
   1               5
<210> 184
   <211> 9
   <212> PRT
   <213> Homo sapiens
<400> 184
   Phe Val Tyr Ser Leu Leu Trp Pro Arg
   1               5
<210> 185
   <211> 9
   <212> PRT
   <213> Homo sapiens
<400> 185
   Arg Pro Trp Glu Pro Pro Leu Asp Lys
   1               5
<210> 186
   <211> 11
   <212> PRT
   <213> Homo sapiens
<400> 186
   Leu Gln Gln Val Asp Ser Leu His Pro His Arg
   1               5                   10
<210> 187
   <211> 9
   <212> PRT
   <213> Homo sapiens
<400> 187
   Asn Pro Asn Val Gln Gln Ala Leu Arg
   1 5
<210> 188
   <211> 13
   <212> PRT
   <213> Homo sapiens
<400> 188
   His Leu Gln Val Glu Thr Leu Thr Gln Glu Asp Ile Lys
   1 5 10
<210> 189
   <211> 7
   <212> PRT
   <213> Homo sapiens
<400> 189
   Ala Gln Ala Leu Gly Thr Arg
   1 5
<210> 190
   <211> 13
   <212> PRT
   <213> Homo sapiens
<400> 190
   Leu Asn Pro Glu Val Leu Ser Pro Asn Ala Val Gln Arg
   1 5 10
<210> 191
   <211> 10
<212> PRT
   <213> Homo sapiens
<400> 191
   Asn Pro Ala Leu Phe Pro Arg Pro Glu Arg
   1 5 10
<210> 192
   <211> 9
   <212> PRT
   <213> Homo sapiens
<400> 192
   Ala Glu Leu Ser Leu Glu Ala Ile Lys
   1 5
<210> 193
   <211> 9
   <212> PRT
   <213> Homo sapiens
<400> 193
   Leu Asn Pro Asp Val Leu Ser Pro Lys
   1 5
<210> 194
   <211> 7
   <212> PRT
   <213> Homo sapiens
<400> 194
   Tyr Asn Leu Gly Gly Pro Arg
   1 5
<210> 195
   <211> 10
   <212> PRT
   <213> Homo sapiens
<400> 195
   Asn Ala Ala Leu Phe Pro Arg Pro Glu Arg
   1 5 10
<210> 196
   <211> 16
   <212> PRT
   <213> Homo sapiens
<400> 196
   Gly Thr Glu Val Ile Ile Asn Leu Trp Ala Leu His His Asn Glu Lys
   1 5 10 15
<210> 197
   <211> 12
   <212> PRT
   <213> Homo sapiens
<400> 197
   Thr Thr Val Ile Val Gly His His Gln Leu Ala Lys
   1 5 10
<210> 198
   <211> 8
   <212> PRT
   <213> Homo sapiens
<400> 198
   Glu Ala Gln Ala Glu Gly Ser Thr
   1               5
<210> 199
   <211> 14
   <212> PRT
   <213> Homo sapiens
<400> 199
   Ala Asn Val Asp Ser Ser Ile Gly Glu Phe Ala Val Asp Lys
   1               5                   10
<210> 200
   <211> 11
   <212> PRT
   <213> Homo sapiens
<400> 200
   Asp Ser Leu Val Asp Leu Val Pro Trp Leu Lys
   1               5                   10
<210> 201
   <211> 11
   <212> PRT
   <213> Homo sapiens
<400> 201
   Ile Pro Leu Asp Glu Ser Ala Ile Val Val Lys
   1               5                   10
<210> 202
   <211> 9
   <212> PRT
   <213> Homo sapiens
<400> 202
   Glu Ile Gln Thr Val Ile Gly Glu Arg
   1               5
<210> 203
   <211> 13
   <212> PRT
   <213> Homo sapiens
<400> 203
   Val Trp Ile Ser Gly Glu Glu Thr Leu Ile Ile Ser Lys
   1 5 10
<210> 204
   <211> 9
   <212> PRT
   <213> Homo sapiens
<400> 204
   Ala Leu Ser Gly Pro Gly Leu Val Arg
   1 5
<210> 205
   <211> 12
   <212> PRT
   <213> Homo sapiens
<400> 205
   Ile His Asp Leu Ser Leu His Pro Asp Glu Thr Lys
   1 5 10
<210> 206
   <211> 10
   <212> PRT
   <213> Homo sapiens
<400> 206
   His Pro Asn Val Glu Glu Ala Ile Ile Lys
   1 5 10
<210> 207
   <211> 12
   <212> PRT
   <213> Homo sapiens
<400> 207
   Gly Ile Ile Phe Asn Asn Asn Pro Glu Leu Trp Lys
   1 5 10
<210> 208
   <211> 7
   <212> PRT
   <213> Homo sapiens
<400> 208
   Leu Leu Trp Asn Trp Trp Lys
   1 5
<210> 209
   <211> 8
   <212> PRT
   <213> Homo sapiens
<400> 209
   Ser Ala Leu Leu Leu Gly Ile Arg
   1 5
<210> 210
   <211> 9
   <212> PRT
   <213> Homo sapiens
<400> 210
   Glu Ser Leu Val Ala Gly Gln Trp Arg
   1 5
<210> 211
   <211> 11
   <212> PRT
   <213> Homo sapiens
<400> 211
   Ala Thr Val Leu Gly Glu Tyr Ala Leu Pro Lys
   1 5 10
<210> 212
   <211> 7
   <212> PRT
   <213> Homo sapiens
<400> 212
   Leu Glu Ile Lys Pro Trp Lys
   1 5
<210> 213
   <211> 7
   <212> PRT
   <213> Homo sapiens
<400> 213
   Asn Pro Gln Val Gln Gln Lys
   1 5
<210> 214
   <211> 10
   <212> PRT
   <213> Homo sapiens
<400> 214
   Ser Leu Ala Ala Phe Leu Gln Gln Leu Arg
   1 5 10
<210> 215
   <211> 12
   <212> PRT
   <213> Homo sapiens
<400> 215
   Gly His Val Glu Asp Leu Tyr Ser Glu Leu Asn Lys
   1 5 10
<210> 216
   <211> 13
   <212> PRT
   <213> Homo sapiens
<400> 216
   Leu Val Thr Ser Thr Ala Tyr Thr Ser Pro Gln Pro Arg
   1 5 10
<210> 217
   <211> 16
   <212> PRT
   <213> Homo sapiens
<400> 217
   Asn Leu Phe Ser Leu Pro Ile Asp Val Pro Phe Ser Gly Leu Tyr Arg
   1 5 10 15
<210> 218
   <211> 11
   <212> PRT
   <213> Homo sapiens
<400> 218
   Leu Val Ser Val His Trp Pro Ala Ser Val Arg
   1 5 10
<210> 219
   <211> 8
   <212> PRT
   <213> Homo sapiens
<400> 219
   Phe Thr His Phe His Gly Glu Ile
   1 5
<210> 220
   <211> 8
   <212> PRT
   <213> Homo sapiens
<400> 220
   Ile Phe Thr Val Glu Leu Ala Arg
   1 5
<210> 221
   <211> 11
   <212> PRT
   <213> Homo sapiens
<400> 221
   Phe Ser Phe Ile Pro Phe Gly Gly Gly Leu Arg
   1 5 10
<210> 222
<211> 10
   <212> PRT
   <213> Homo sapiens
<400> 222
   Leu Asn Pro Pro Val Pro Gly Gly Phe Arg
   1 5 10
<210> 223
   <211> 10
   <212> PRT
   <213> Homo sapiens
<400> 223
   Thr His Leu Phe Gly Arg Pro Thr Val Arg
   1 5 10
<210> 224
   <211> 11
   <212> PRT
   <213> Homo sapiens
<400> 224
   Thr Phe Glu Leu Asn Gly Tyr Gln Ile Pro Lys
   1 5 10
<210> 225
   <211> 13
   <212> PRT
   <213> Homo sapiens
<400> 225
   Asp Ala Leu Gln Leu Leu Ile Glu His Ser Trp Glu Arg
   1 5 10
<210> 226
   <211> 14
   <212> PRT
   <213> Homo sapiens
<400> 226
   Thr Ser Pro Thr Val Tyr Pro Val Asp Asn Leu Pro Ala Arg
   1 5 10
<210> 227
   <211> 16
   <212> PRT
   <213> Homo sapiens
<400> 227
   Ile Thr Leu Val Pro Val Leu His Pro Val Asp Gly Leu Ser Val Lys
   1 5 10 15
<210> 228
   <211> 13
   <212> PRT
   <213> Homo sapiens
<400> 228
   Ile Phe Ser His Glu Ala Leu Glu Ser Tyr Leu Pro Lys
   1 5 10
<210> 229
   <211> 10
   <212> PRT
   <213> Homo sapiens
<400> 229
   Asp Thr His Asp Thr Ala Pro Val Phe Lys
   1               5                   10
<210> 230
   <211> 9
   <212> PRT
   <213> Homo sapiens
<400> 230
   Asp Val Asn Val Phe Asp Pro Asp Arg
   1               5
<210> 231
   <211> 10
   <212> PRT
   <213> Homo sapiens
<400> 231
   Thr His Leu Leu Gly Arg Pro Leu Ile Arg
   1               5                   10
<210> 232
   <211> 8
   <212> PRT
   <213> Homo sapiens
<400> 232
   Leu Asp Thr Leu Ser Gly Leu Arg
   1               5
<210> 233
   <211> 15
   <212> PRT
   <213> Homo sapiens
<400> 233
   Thr Val Leu Gln Thr Phe Glu Leu Asp Gly Phe Gln Ile Pro Lys
   1               5                   10                  15
<210> 234
   <211> 11
   <212> PRT
   <213> Homo sapiens
<400> 234
   Phe His Tyr Leu Pro Phe Gly Gly Gly Val Arg
   1 5 10
<210> 235
   <211> 10
   <212> PRT
   <213> Homo sapiens
<400> 235
   Ile Gln Leu Val Ile Gln Asp Thr Leu Arg
   1 5 10
<210> 236
   <211> 11
   <212> PRT
   <213> Homo sapiens
<400> 236
   Val Leu Ala Val Glu Leu Ala Ser Thr Ser Arg
   1 5 10
<210> 237
   <211> 7
   <212> PRT
   <213> Homo sapiens
<400> 237
   His Pro Thr Val Leu Glu Lys
   1 5
<210> 238
   <211> 10
   <212> PRT
   <213> Homo sapiens
<400> 238
   Leu Phe Thr Pro Ile Ser Gly Gly Tyr Arg
   1 5 10
<210> 239
   <211> 10
   <212> PRT
   <213> Homo sapiens
<400> 239
   Glu Glu Leu Val Ala Gln Gly Leu Gly Arg
   1 5 10
<210> 240
   <211> 16
   <212> PRT
   <213> Homo sapiens
<400> 240
   Ala Ala Glu Pro Gly Asp Ala Leu Asp Leu Ile Ile His Ser Ala Arg
   1 5 10 15
<210> 241
   <211> 11
   <212> PRT
   <213> Homo sapiens
<400> 241
   Thr Phe Glu Leu Asp Gly Tyr Gln Ile Pro Lys
   1 5 10
<210> 242
   <211> 8
   <212> PRT
   <213> Homo sapiens
<400> 242
   Thr Ile Leu Leu Gly Glu His Arg
   1 5
<210> 243
   <211> 10
   <212> PRT
   <213> Homo sapiens
<400> 243
   Ser Pro Pro Glu Gly Phe Asp Pro Glu Arg
   1 5 10
<210> 244
   <211> 10
   <212> PRT
   <213> Homo sapiens
<400> 244
   Thr His Leu Leu Gly Arg Pro Val Ile Arg
   1 5 10
<210> 245
   <211> 10
   <212> PRT
   <213> Homo sapiens
<400> 245
   Asp Thr His Glu Thr Ala Ala Val Tyr Arg
   1               5                   10
<210> 246
   <211> 8
   <212> PRT
   <213> Homo sapiens
<400> 246
   Ala Ser Thr Leu Pro Leu Pro Lys
   1               5
<210> 247
   <211> 10
   <212> PRT
   <213> Homo sapiens
<400> 247
   Leu Leu Pro Pro Val Ser Gly Gly Tyr Arg
   1               5                   10
<210> 248
   <211> 9
   <212> PRT
   <213> Homo sapiens
<400> 248
   His Leu Glu Gly Ala Ile Ser Glu Lys
   1               5
<210> 249
   <211> 8
   <212> PRT
   <213> Homo sapiens
<400> 249
   Val Ser Gly Ala Glu Asn Val Arg
   1               5
<210> 250
   <211> 11
   <212> PRT
   <213> Homo sapiens
<400> 250
   Ala Ala Ile Pro Ala Ala Leu Pro Ser Asp Lys
   1               5                   10
<210> 251
   <211> 7
   <212> PRT
   <213> Homo sapiens
<400> 251
   Ser Leu Glu Glu Ile Pro Arg
   1               5
<210> 252
   <211> 8
   <212> PRT
   <213> Homo sapiens
<400> 252
   Asn Ser Gln Pro Ala Thr Pro Arg
   1               5
<210> 253
   <211> 11
   <212> PRT
   <213> Homo sapiens
<400> 253
   Glu Ile Glu Val Asp Gly Phe Leu Phe Pro Lys
   1               5                   10
<210> 254
   <211> 15
   <212> PRT
   <213> Homo sapiens
<400> 254
   Asp Pro Thr Ala Phe Ser Glu Pro Glu Ser Phe Gln Pro His Arg
   1               5                   10                  15
<210> 255
   <211> 11
   <212> PRT
   <213> Homo sapiens
<400> 255
   Val Val Leu Ala Pro Glu Thr Gly Glu Leu Lys
   1               5                   10
<210> 256
   <211> 11
   <212> PRT
   <213> Homo sapiens
<400> 256
   Ser Ile Pro Glu Asp Thr Val Thr Phe Val Arg
   1 5 10
<210> 257
   <211> 11
   <212> PRT
   <213> Homo sapiens
<400> 257
   Asp Ile His Val Gly Asp Tyr Ile Ile Pro Lys
   1 5 10
<210> 258
   <211> 15
   <212> PRT
   <213> Homo sapiens
<400> 258
   Thr Val Tyr Val Ala Ala Pro Ala Leu Val Glu Glu Leu Leu Arg
   1 5 10 15
<210> 259
   <211> 9
<212> PRT
   <213> Homo sapiens
<400> 259
   Gly Thr Gly Pro Pro Ala Leu Val Arg
   1 5
<210> 260
   <211> 9
   <212> PRT
   <213> Homo sapiens
<400> 260
   His Leu Val Pro Gly Pro Trp Gly Arg
   1 5
<210> 261
   <211> 15
   <212> PRT
   <213> Homo sapiens
<400> 261
   Ser Leu Leu Ala Pro Leu Leu Leu Arg Pro Gln Ala Ala Ala Arg
   1 5 10 15
<210> 262
   <211> 14
   <212> PRT
   <213> Homo sapiens
<400> 262
   Asp Leu Glu Ser Gly Ala His Leu Thr His Phe Leu Phe Arg
   1 5 10
<210> 263
   <211> 12
   <212> PRT
   <213> Homo sapiens
<400> 263
   Trp Ala Pro Glu Leu Gly Ala Ser Leu Gly Tyr Arg
   1 5 10
<210> 264
   <211> 9
   <212> PRT
   <213> Homo sapiens
<400> 264
   Ser Ile Asn Leu Gln Phe Leu Asp Arg
   1 5
<210> 265
   <211> 14
   <212> PRT
   <213> Homo sapiens
<400> 265
   Phe Gly Leu Glu Gly Ile Ala Ala Val Leu Leu Gly Ser Arg
   1 5 10
<210> 266
   <211> 10
   <212> PRT
   <213> Homo sapiens
<400> 266
   His Pro Glu Val Gln Gln Thr Val Tyr Arg
   1 5 10
<210> 267
   <211> 14
   <212> PRT
   <213> Homo sapiens
<400> 267
   Val Asp Asn Phe Gly Ser Ile Pro Phe Gly His Gly Val Arg
   1 5 10
<210> 268
   <211> 9
   <212> PRT
   <213> Homo sapiens
<400> 268
   Phe Ser Gln Ile His Val Asp Asn Lys
   1 5
<210> 269
   <211> 11
   <212> PRT
   <213> Homo sapiens
<400> 269
   Thr Ser Ser Gln Thr Asn Ala Val His Ala Lys
   1 5 10
<210> 270
   <211> 10
   <212> PRT
   <213> Homo sapiens
<400> 270
   Leu Phe Pro Val Leu Pro Gly Asn Gly Arg
   1 5 10
<210> 271
   <211> 14
   <212> PRT
   <213> Homo sapiens
<400> 271
   Thr His Gly Leu Leu Thr Pro Gly Gly Pro Ile His Val Arg
   1 5 10
<210> 272
   <211> 7
   <212> PRT
   <213> Homo sapiens
<400> 272
   Ser Trp Asp Gly Leu Phe Lys
   1 5
<210> 273
   <211> 15
   <212> PRT
   <213> Homo sapiens
<400> 273
   Val Thr Gln Glu Asp Leu Val Ile Gly Gly Tyr Leu Ile Pro Lys
   1 5 10 15
<210> 274
   <211> 11
   <212> PRT
   <213> Homo sapiens
<400> 274
   Trp Leu Arg Pro Phe Ile Pro Lys Pro Trp Arg
   1 5 10
<210> 275
   <211> 10
   <212> PRT
   <213> Homo sapiens
<400> 275
   His Val Pro Thr Ala Ala Asp Val Pro Lys
   1 5 10
<210> 276
   <211> 13
   <212> PRT
   <213> Homo sapiens
<400> 276
   Val Ser Glu Asp Asp Leu Glu Asn Leu Leu Leu Ile Lys
   1 5 10
<210> 277
   <211> 9
   <212> PRT
   <213> Homo sapiens
<400> 277
   Phe Gly Pro Gly Ala Pro Lys Pro Arg
   1 5
<210> 278
   <211> 11
   <212> PRT
   <213> Homo sapiens
<400> 278
   Phe Thr Tyr Phe Pro Phe Ser Leu Gly His Arg
   1 5 10
<210> 279
   <211> 11
   <212> PRT
   <213> Homo sapiens
<400> 279
   Ala Glu Gln Leu Val Glu Ile Leu Glu Ala Lys
   1 5 10
<210> 280
   <211> 9
   <212> PRT
   <213> Homo sapiens
<400> 280
   Ala Leu Gln Thr Val Phe Gly Glu Arg
   1 5
<210> 281
   <211> 10
   <212> PRT
   <213> Homo sapiens
<400> 281
   Leu Tyr Pro Pro Ala Trp Gly Thr Phe Arg
   1 5 10
<210> 282
   <211> 7
   <212> PRT
   <213> Homo sapiens
<400> 282
   Gln Pro Glu Ile Val Ala Arg
   1 5
<210> 283
   <211> 12
   <212> PRT
   <213> Homo sapiens
<400> 283
   Leu Leu Glu Glu Glu Thr Leu Ile Asp Gly Val Arg
   1 5 10
<210> 284
   <211> 9
   <212> PRT
   <213> Homo sapiens
<400> 284
   Leu Gln Tyr Leu Ser Gln Val Leu Lys
   1 5
<210> 285
   <211> 7
   <212> PRT
   <213> Homo sapiens
<400> 285
   Asp Leu Asn Leu Leu Asp Arg
   1 5
<210> 286
   <211> 7
   <212> PRT
   <213> Homo sapiens
<400> 286
   Leu Asp Phe Asn Pro Asp Arg
   1 5
<210> 287
   <211> 8
   <212> PRT
   <213> Homo sapiens
<400> 287
   Leu Thr Thr Pro Val Phe Gly Lys
   1 5
<210> 288
   <211> 8
   <212> PRT
   <213> Homo sapiens
<400> 288
   Gln His Val Ser Ile Ile Glu Lys
   1 5
<210> 289
   <211> 9
   <212> PRT
   <213> Homo sapiens
<400> 289
   Ser Gly Leu Asn Ile Ala His Phe Lys
   1 5
<210> 290
<211> 10
   <212> PRT
   <213> Homo sapiens
<400> 290
   Phe Ala Tyr Val Pro Phe Gly Ala Gly Arg
   1 5 10
<210> 291
   <211> 11
   <212> PRT
   <213> Homo sapiens
<400> 291
   Tyr Leu Gln Asp Asn Pro Ala Ser Gly Glu Lys
   1 5 10
<210> 292
   <211> 12
   <212> PRT
   <213> Homo sapiens
<400> 292
   Asn Glu Asp Leu Asn Ala Glu Asp Val Tyr Ser Arg
   1 5 10
<210> 293
   <211> 12
   <212> PRT
   <213> Homo sapiens
<400> 293
   Ser Pro Ile Glu Phe Leu Glu Asn Ala Tyr Glu Lys
   1 5 10
<210> 294
   <211> 12
   <212> PRT
   <213> Homo sapiens
<400> 294
   Glu Tyr Phe Glu Ser Trp Gly Glu Ser Gly Glu Lys
   1 5 10
<210> 295
   <211> 14
   <212> PRT
   <213> Homo sapiens
<400> 295
   Leu Ala Ala Gly His Leu Val Gln Leu Pro Ala Gly Val Lys
   1 5 10
<210> 296
   <211> 14
   <212> PRT
<213> Homo sapiens
<400> 296
   Ile Asp Asp Ile Leu Gln Thr Leu Leu Asp Ala Thr Tyr Lys
   1 5 10
<210> 297
   <211> 16
   <212> PRT
   <213> Homo sapiens
<400> 297
   Gly His Glu Ile Val Val Leu Ala Pro Asp Ala Ser Leu Tyr Ile Arg
   1 5 10 15
<210> 298
   <211> 11
   <212> PRT
   <213> Homo sapiens
<400> 298
   Tyr Leu Ser Ile Pro Thr Val Phe Phe Leu Arg
   1 5 10
<210> 299
   <211> 17
   <212> PRT
   <213> Homo sapiens
<400> 299
   Glu Val Ser Val Val Asp Ile Leu Ser His Ala Ser Val Trp Leu Phe
   1 5 10 15
   Arg
<210> 300
   <211> 11
   <212> PRT
   <213> Homo sapiens
<400> 300
   Tyr Leu Ser Ile Pro Ala Val Phe Phe Trp Arg
   1 5 10
<210> 301
   <211> 14
   <212> PRT
   <213> Homo sapiens
<400> 301
   Phe Phe Thr Leu Thr Ala Tyr Ala Val Pro Trp Thr Gln Lys
   1 5 10
<210> 302
<211> 11
   <212> PRT
   <213> Homo sapiens
<400> 302
   Tyr Leu Ser Ile Pro Ala Val Phe Phe Leu Arg
   1 5 10
<210> 303
   <211> 9
   <212> PRT
   <213> Homo sapiens
<400> 303
   Asp Ile Val Glu Val Leu Ser Asp Arg
   1 5
<210> 304
   <211> 10
   <212> PRT
   <213> Homo sapiens
<400> 304
   Ser Pro Asp Pro Val Ser Tyr Ile Pro Arg
   1 5 10
<210> 305
   <211> 11
   <212> PRT
   <213> Homo sapiens
<400> 305
   Ser Phe Leu Thr Ala Pro Gln Thr Glu Tyr Arg
   1 5 10
<210> 306
   <211> 6
   <212> PRT
   <213> Homo sapiens
<400> 306
   Trp Thr Ala Pro Leu Arg
   1 5
<210> 307
   <211> 13
   <212> PRT
   <213> Homo sapiens
<400> 307
   Thr Tyr Ser Thr Ser Tyr Thr Leu Glu Asp Gln Asp Arg
   1 5 10
<210> 308
   <211> 11
   <212> PRT
<213> Homo sapiens
<400> 308
   Tyr Phe Ser Leu Pro Ser Val Val Phe Thr Arg
   1 5 10
<210> 309
   <211> 7
   <212> PRT
   <213> Homo sapiens
<400> 309
   Ala Glu Ile Trp Leu Ile Arg
   1 5
<210> 310
   <211> 14
   <212> PRT
   <213> Homo sapiens
<400> 310
   Ala Asn Leu Ile Ala Ser Ala Leu Ala Gln Ile Pro Gln Lys
   1 5 10
<210> 311
   <211> 10
   <212> PRT
   <213> Homo sapiens
<400> 311
   Ala Asn Val Ile Ala Ser Ala Leu Ala Lys
   1 5 10
<210> 312
   <211> 7
   <212> PRT
   <213> Homo sapiens
<400> 312
   Ala Asp Val Trp Leu Ile Arg
   1 5
<210> 313
   <211> 9
   <212> PRT
   <213> Homo sapiens
<400> 313
   Thr Ile Leu Asp Glu Leu Ile Gln Arg
   1               5
<210> 314
   <211> 6
   <212> PRT
   <213> Homo sapiens
<400> 314
   Asn Ser Trp Asn Phe Lys
   1               5
<210> 315
   <211> 10
   <212> PRT
   <213> Homo sapiens
<400> 315
   Phe Glu Val Tyr Pro Thr Ser Leu Thr Lys
   1               5                   10
<210> 316
   <211> 9
   <212> PRT
   <213> Homo sapiens
<400> 316
   Ser Val Ile Asn Asp Pro Ile Tyr Lys
   1               5
<210> 317
   <211> 12
   <212> PRT
   <213> Homo sapiens
<400> 317
   Trp Ile Pro Gln Asn Asp Leu Leu Gly Leu Pro Lys
   1               5                   10
<210> 318
   <211> 7
   <212> PRT
   <213> Homo sapiens
<400> 318
   Leu His Tyr Phe Asn Ala Arg
   1               5
<210> 319
   <211> 7
   <212> PRT
   <213> Homo sapiens
<400> 319
   Leu Ala Leu Ile Gln Glu Lys
   1               5
<210> 320
   <211> 7
   <212> PRT
   <213> Homo sapiens
<400> 320
   Leu His Tyr Phe Asn Gly Arg
   1               5
<210> 321
   <211> 7
   <212> PRT
   <213> Homo sapiens
<400> 321
   Leu His Tyr Pro Asn Gly Arg
   1               5
<210> 322
   <211> 7
   <212> PRT
   <213> Homo sapiens
<400> 322
   Tyr Phe Pro Val Phe Glu Lys
   1               5
<210> 323
   <211> 8
   <212> PRT
   <213> Homo sapiens
<400> 323
   Thr Val Tyr Asn Ile Phe Arg Pro
   1 5
<210> 324
   <211> 7
   <212> PRT
   <213> Homo sapiens
<400> 324
   Leu His Tyr Ser Asn Ala Arg
   1 5
<210> 325
   <211> 11
   <212> PRT
   <213> Homo sapiens
<400> 325
   His His Leu Gln Ile Pro Ile His Phe Pro Lys
   1 5 10
<210> 326
   <211> 7
   <212> PRT
   <213> Homo sapiens
<400> 326
   Gly Leu Ala His Ala Ile Arg
   1 5
<210> 327
   <211> 8
   <212> PRT
   <213> Homo sapiens
<400> 327
   Gln Pro Trp Phe Leu Gly Asp Lys
   1 5
<210> 328
   <211> 9
   <212> PRT
   <213> Homo sapiens
<400> 328
   Phe Leu Pro Arg Pro Val Phe Thr Lys
   1 5
<210> 329
   <211> 7
   <212> PRT
   <213> Homo sapiens
<400> 329
   Ser Gln Trp Leu Asp Val Lys
   1 5
<210> 330
   <211> 9
   <212> PRT
   <213> Homo sapiens
<400> 330
   Phe Leu Pro Lys Pro Leu Tyr Thr Arg
   1 5
<210> 331
   <211> 6
   <212> PRT
   <213> Homo sapiens
<400> 331
   Ser Ser Gln Phe Leu Arg
   1 5
<210> 332
   <211> 7
   <212> PRT
   <213> Homo sapiens
<400> 332
   Thr Leu Gly Leu Tyr Gly Lys
   1 5
<210> 333
   <211> 8
   <212> PRT
   <213> Homo sapiens
<400> 333
   Asn Asp Ile Pro Phe Glu Leu Arg
   1 5
<210> 334
   <211> 10
   <212> PRT
   <213> Homo sapiens
<400> 334
   Asp Phe Pro Pro Ala Asp Pro Thr Ile Lys
   1               5                   10
<210> 335
   <211> 8
   <212> PRT
   <213> Homo sapiens
<400> 335
   Asn Gly Ile Pro Leu Glu Leu Arg
   1               5
<210> 336
   <211> 6
   <212> PRT
   <213> Homo sapiens
<400> 336
   Leu Phe Val Gly Ala Arg
   1               5
<210> 337
   <211> 14
   <212> PRT
   <213> Homo sapiens
<400> 337
   Val Thr Pro Pro Ala Val Thr Gly Ser Pro Glu Phe Glu Arg
   1               5                   10
<210> 338
   <211> 8
   <212> PRT
   <213> Homo sapiens
<400> 338
   Ser Gly Leu Gly Ser Gly Pro Lys
   1               5
<210> 339
   <211> 6
<212> PRT
   <213> Homo sapiens
<400> 339
   Asn Thr Pro Ala Pro Arg
   1 5
<210> 340
   <211> 6
   <212> PRT
   <213> Homo sapiens
<400> 340
   Ala Pro Ile Tyr Val Arg
   1 5
<210> 341
   <211> 9
   <212> PRT
   <213> Homo sapiens
<400> 341
   Asn Leu Asn Asp Glu Ile Leu Asp Arg
   1 5
<210> 342
   <211> 7
   <212> PRT
   <213> Homo sapiens
<400> 342
   Gly Thr Val Gly Asp Trp Lys
   1 5
<210> 343
   <211> 7
   <212> PRT
   <213> Homo sapiens
<400> 343
   His Ala Phe Leu Glu Leu Lys
   1 5
<210> 344
   <211> 7
   <212> PRT
   <213> Homo sapiens
<400> 344
   Leu Thr Phe His Phe Gln Phe
   1 5
<210> 345
   <211> 7
   <212> PRT
   <213> Homo sapiens
<400> 345
   Glu Asp Val Ile Phe Asn Arg
   1               5
<210> 346
   <211> 7
   <212> PRT
   <213> Homo sapiens
<400> 346
   Gly Val Ser Gly Asp Trp Lys
   1               5
<210> 347
   <211> 10
   <212> PRT
   <213> Homo sapiens
<400> 347
   Asp Pro Gly Thr Pro Asp Gln Phe Leu Arg
   1               5                   10
<210> 348
   <211> 8
   <212> PRT
   <213> Homo sapiens
<400> 348
   Tyr Phe Glu Phe His Gly Val Arg
   1               5
<210> 349
   <211> 7
   <212> PRT
   <213> Homo sapiens
<400> 349
   Gln Gln Phe Leu Ile Leu Arg
   1               5
<210> 350
   <211> 7
   <212> PRT
   <213> Homo sapiens
<400> 350
   Tyr Ser Leu Asp Val Trp Ser
   1               5
<210> 351
   <211> 7
   <212> PRT
   <213> Homo sapiens
<400> 351
   Glu Ser Ser Phe Val Glu Lys
   1               5
<210> 352
   <211> 8
   <212> PRT
   <213> Homo sapiens
<400> 352
   Glu Glu Leu Ala Gln Phe His Arg
   1               5
<210> 353
   <211> 9
   <212> PRT
   <213> Homo sapiens
<400> 353
   Ile Gln Thr Leu Thr Ser Ser Val Arg
   1               5
<210> 354
   <211> 9
   <212> PRT
   <213> Homo sapiens
<400> 354
   Tyr Tyr Ser Ile Ala Ser Ser Ser Lys
   1               5
<210> 355
   <211> 9
   <212> PRT
   <213> Homo sapiens
<400> 355
   Phe Ala Val Phe Gly Leu Gly Asn Lys
   1 5
<210> 356
   <211> 12
   <212> PRT
   <213> Homo sapiens
<400> 356
   Ser Tyr Glu Asn Gln Lys Pro Pro Phe Asp Ala Lys
   1 5 10
<210> 357
   <211> 13
   <212> PRT
   <213> Homo sapiens
<400> 357
   Asp Gly Ala Leu Thr Gln Leu Asn Val Ala Phe Ser Arg
   1 5 10
<210> 358
   <211> 14
   <212> PRT
   <213> Homo sapiens
<400> 358
   Thr Ala Leu Thr Tyr Tyr Leu Asp Ile Thr Asn Pro Pro Arg
   1 5 10
<210> 359
   <211> 14
   <212> PRT
   <213> Homo sapiens
<400> 359
   Gln Tyr Glu Leu Val Val His Thr Asp Ile Asp Ala Ala Lys
   1 5 10
<210> 360
   <211> 10
   <212> PRT
   <213> Homo sapiens
<400> 360
   Glu Trp Leu Leu Ala His Glu Gly His Arg
   1 5 10
<210> 361
   <211> 9
   <212> PRT
   <213> Homo sapiens
<400> 361
   Thr Thr Leu Asp Ser Pro Leu Gly Lys
   1 5
<210> 362
   <211> 9
   <212> PRT
   <213> Homo sapiens
<400> 362
   Ala Gln Leu Ala Ser Leu Asn Gly Lys
   1 5
<210> 363
   <211> 8
   <212> PRT
   <213> Homo sapiens
<400> 363
   Ser Thr Phe Val Asn Ile Leu Lys
   1 5
<210> 364
   <211> 7
   <212> PRT
   <213> Homo sapiens
<400> 364
   Ala Val Ser Glu Gly Tyr Lys
   1 5
<210> 365
   <211> 10
   <212> PRT
   <213> Homo sapiens
<400> 365
   Asp Val Val Val Ser Gln Ser Gln Leu Arg
   1 5 10
<210> 366
   <211> 8
   <212> PRT
   <213> Homo sapiens
<400> 366
   Gly Phe Leu Glu Ala Leu Gly Arg
   1               5
<210> 367
   <211> 9
   <212> PRT
   <213> Homo sapiens
<400> 367
   Ala Val Asn Pro Asn His Ser Leu Arg
   1               5
<210> 368
   <211> 8
   <212> PRT
   <213> Homo sapiens
<400> 368
   Ala Pro Ser Ile Asp Glu Pro Arg
   1               5
<210> 369
   <211> 8
   <212> PRT
   <213> Homo sapiens
<400> 369
   Gly Phe Leu Glu Asp Leu Gly Arg
   1               5
<210> 370
<211> 9
   <212> PRT
   <213> Homo sapiens
<400> 370
   Leu Pro Ser Ser Ser Thr Ser Ser Arg
   1               5
<210> 371
   <211> 9
   <212> PRT
   <213> Homo sapiens
<400> 371
   Ile Tyr Glu Gln Glu Gly Leu Glu Lys
   1               5
<210> 372
   <211> 9
   <212> PRT
   <213> Homo sapiens
<400> 372
   Asp Ser Gln Pro Leu Glu Tyr Ser Arg
   1               5
<210> 373
   <211> 7
   <212> PRT
   <213> Homo sapiens
<400> 373
   Asp Pro Ile Gly Tyr Leu Lys
   1               5
<210> 374
   <211> 11
   <212> PRT
   <213> Homo sapiens
<400> 374
   Gly Ile Tyr Pro Ala Ser Thr Gln Leu Asp Arg
   1               5                   10
<210> 375
   <211> 10
   <212> PRT
   <213> Homo sapiens
<400> 375
   Val Phe Leu Ala Thr Asn Ser Asp Tyr Lys
   1               5                   10
<210> 376
   <211> 10
   <212> PRT
<213> Homo sapiens
<400> 376
   Gln Ala Gly Val Tyr His Pro Asn Val Lys
   1               5                   10
<210> 377
   <211> 7
   <212> PRT
   <213> Homo sapiens
<400> 377
   Val Asp Glu Leu Leu Glu Lys
   1               5
<210> 378
   <211> 9
   <212> PRT
   <213> Homo sapiens
<400> 378
   Glu Gly Tyr Glu Asn Phe Phe Asp Lys
   1               5
<210> 379
   <211> 9
   <212> PRT
   <213> Homo sapiens
<400> 379
   Gly Glu Leu Ile His Val Phe Asn Lys
   1               5
<210> 380
   <211> 9
   <212> PRT
   <213> Homo sapiens
<400> 380
   Asn Thr Glu Tyr Phe Asn Gln Leu Lys
   1               5
<210> 381
   <211> 12
   <212> PRT
   <213> Homo sapiens
<400> 381
   Ser His Gly Leu Leu Val Gln Gln Ala Leu Pro Lys
   1 5 10
<210> 382
   <211> 7
   <212> PRT
   <213> Homo sapiens
<400> 382
   Ile Gly Tyr Leu Asn Asp Arg
   1 5
<210> 383
   <211> 12
   <212> PRT
   <213> Homo sapiens
<400> 383
   Thr Val Val Leu Gly Asp Leu Leu Ile Asp Asp Lys
   1 5 10
<210> 384
   <211> 14
   <212> PRT
   <213> Homo sapiens
<400> 384
   Tyr Phe Gly Pro Asp Phe Leu Glu Gln Ile Val Leu Thr Arg
   1 5 10
<210> 385
   <211> 12
   <212> PRT
   <213> Homo sapiens
<400> 385
   Gly Ala Ala Gly Gly Leu Gly Leu Ala Gly Gly Arg
   1 5 10
<210> 386
   <211> 9
   <212> PRT
   <213> Homo sapiens
<400> 386
   Phe Pro Ile Leu Ser Ala Asn Ile Lys
   1 5
<210> 387
   <211> 11
   <212> PRT
   <213> Homo sapiens
<400> 387
   Val Pro Val Val Gln Ala Tyr Ala Phe Gly Lys
   1               5                   10
<210> 388
   <211> 8
   <212> PRT
   <213> Homo sapiens
<400> 388
   Val Pro Ser Tyr Asp Pro Leu Lys
   1               5
<210> 389
   <211> 10
   <212> PRT
   <213> Homo sapiens
<400> 389
   His Pro Asp Tyr Ala Ile Leu Ala Ala Arg
   1               5                   10
<210> 390
   <211> 9
   <212> PRT
   <213> Homo sapiens
<400> 390
   Leu Asn Ser Ala Ile Ile Tyr Asp Arg
   1               5
<210> 391
   <211> 10
   <212> PRT
   <213> Homo sapiens
<400> 391
   Ser Thr Leu Asp Ile Val Leu Ala Asn Lys
   1               5                   10
<210> 392
   <211> 10
   <212> PRT
   <213> Homo sapiens
<400> 392
   Ser Asn Gln Gln Asn Leu Gly Thr Ile Lys
   1 5 10
<210> 393
   <211> 11
   <212> PRT
   <213> Homo sapiens
<400> 393
   Glu Asn Thr Pro Pro Ala Leu Ser Gly Thr Arg
   1 5 10
<210> 394
   <211> 9
   <212> PRT
   <213> Homo sapiens
<400> 394
   Ile Phe Gln Glu Pro Thr Glu Pro Lys
   1 5
<210> 395
   <211> 8
   <212> PRT
   <213> Homo sapiens
<400> 395
   Phe Leu Ile Asp Gly Phe Pro Arg
   1 5
<210> 396
   <211> 8
   <212> PRT
   <213> Homo sapiens
<400> 396
   Leu Ile Ile Gln Ala Gly Ile Lys
   1 5
<210> 397
   <211> 7
   <212> PRT
   <213> Homo sapiens
<400> 397
   Phe Pro Ser Ile Asn His Asp
   1 5
<210> 398
   <211> 12
   <212> PRT
   <213> Homo sapiens
<400> 398
   Gly Ile Ile Ala Ser Ser Val Gly Thr Ile Leu Lys
   1 5 10
<210> 399
   <211> 8
   <212> PRT
   <213> Homo sapiens
<400> 399
   Ile Gln Ala Ile Ala Trp Ala Arg
   1 5
<210> 400
   <211> 8
   <212> PRT
   <213> Homo sapiens
<400> 400
   Ile Tyr Gln Tyr Val Ile Asn Lys
   1 5
<210> 401
   <211> 10
   <212> PRT
   <213> Homo sapiens
<400> 401
   Phe Ser Asp Ser Tyr Ala Ser Val Ile Lys
   1 5 10
<210> 402
   <211> 10
   <212> PRT
   <213> Homo sapiens
<400> 402
   Phe Val Gly Gln Asp Val Glu Gly Glu Arg
   1 5 10
<210> 403
   <211> 7
   <212> PRT
   <213> Homo sapiens
<400> 403
   Leu Ser His Tyr Leu Gln Lys
   1 5
<210> 404
   <211> 8
   <212> PRT
   <213> Homo sapiens
<400> 404
   Asp Asn Asn Leu Thr Thr Gly Lys
   1 5
<210> 405
   <211> 7
   <212> PRT
   <213> Homo sapiens
<400> 405
   Leu Asp Leu Pro Ile Glu Arg
   1 5
<210> 406
   <211> 9
   <212> PRT
   <213> Homo sapiens
<400> 406
   Ile Thr Glu Thr Glu Asp Pro Val Lys
   1 5
<210> 407
   <211> 7
<212> PRT
   <213> Homo sapiens
<400> 407
   Val Pro Val Asp Gly Asn Lys
   1 5
<210> 408
   <211> 8
   <212> PRT
   <213> Homo sapiens
<400> 408
   Asp Asn Asn Ile Thr Thr Gly Lys
   1               5
<210> 409
   <211> 13
   <212> PRT
   <213> Homo sapiens
<400> 409
   Ala Asp Gly Ile Leu Val Pro Gly Gly Phe Gly Ile Arg
   1               5                   10
<210> 410
   <211> 9
   <212> PRT
   <213> Homo sapiens
<400> 410
   Phe Glu Val Asn Pro Asn Leu Ile Lys
   1               5
<210> 411
   <211> 8
   <212> PRT
   <213> Homo sapiens
<400> 411
   Phe Leu Asp Ile Asn Leu Tyr Lys
   1               5
<210> 412
   <211> 12
   <212> PRT
   <213> Homo sapiens
<400> 412
   Gly Ile Ile Ala Ser Ser Ile Gly Thr Ile Leu Lys
   1               5                   10
<210> 413
   <211> 9
   <212> PRT
   <213> Mus musculus
<400> 413
   Tyr Thr Val Asn Asp Leu Val Leu Arg
   1               5
<210> 414
   <211> 10
   <212> PRT
   <213> Mus musculus
<400> 414
   Asp Pro Gly Phe Phe Pro Asn Pro Asn Lys
   1               5                   10
<210> 415
   <211> 10
   <212> PRT
   <213> Mus musculus
<400> 415
   Gly Ala Gly Thr Ser Thr Ser Ser Pro Arg
   1               5                   10
<210> 416
   <211> 10
   <212> PRT
   <213> Mus musculus
<400> 416
   Leu His Pro Ile Ser Val Thr Leu Gln Arg
   1               5                   10
<210> 417
   <211> 9
   <212> PRT
   <213> Mus musculus
<400> 417
   Tyr Leu Gly Phe Gly Trp Gly Val Arg
   1               5
<210> 418
   <211> 13
   <212> PRT
   <213> Mus musculus
<400> 418
   Ala Asp Glu Tyr Thr Gln Asn Phe Tyr Trp Asp Leu Arg
   1 5 10
<210> 419
   <211> 12
   <212> PRT
   <213> Mus musculus
<400> 419
   Asp His Ala Ala Ala Trp Asp Val Ile Phe Asn Lys
   1 5 10
<210> 420
   <211> 14
   <212> PRT
   <213> Mus musculus
<400> 420
   Phe Ser Phe Glu Ser Ile Ser Ser Val Ile Phe Gly Glu Arg
   1 5 10
<210> 421
   <211> 13
   <212> PRT
   <213> Mus musculus
<400> 421
   Leu Gly Thr Leu Glu Ser Val Tyr Ile Val Asp Pro Lys
   1 5 10
<210> 422
   <211> 7
   <212> PRT
   <213> Mus musculus
<400> 422
   Ile Glu Val Gln Asn Leu Arg
   1 5
<210> 423
   <211> 9
   <212> PRT
   <213> Mus musculus
<400> 423
   Val Val Phe Leu Ile Asp Pro Phe Lys
   1 5
<210> 424
   <211> 9
   <212> PRT
   <213> Mus musculus
<400> 424
   Phe Asp Phe Asp Val Ser Asp Asp Lys
   1 5
<210> 425
   <211> 10
   <212> PRT
   <213> Mus musculus
<400> 425
   Glu Ile Asp Gln Tyr Val Gly Phe Ser Arg
   1 5 10
<210> 426
   <211> 8
   <212> PRT
   <213> Mus musculus
<400> 426
   Asp Leu Ser Thr Leu Ile Phe Lys
   1 5
<210> 427
   <211> 7
   <212> PRT
   <213> Mus musculus
<400> 427
   Thr Pro Ser Phe Asn Asp Arg
   1 5
<210> 428
   <211> 8
   <212> PRT
   <213> Mus musculus
<400> 428
   Tyr Gly Pro Ile Tyr Ser Leu Arg
   1 5
<210> 429
   <211> 14
   <212> PRT
   <213> Mus musculus
<400> 429
   Ala Asn Ile Asp Ser Ser Ile Gly Glu Phe Ala Ile Pro Lys
   1 5 10
<210> 430
   <211> 9
   <212> PRT
   <213> Mus musculus
<400> 430
   Ala Leu Thr Gly Pro Gly Leu Val Arg
   1 5
<210> 431
   <211> 10
   <212> PRT
   <213> Mus musculus
<400> 431
   Tyr Phe Gln Pro Phe Gly Phe Gly Pro Arg
   1 5 10
<210> 432
   <211> 9
   <212> PRT
   <213> Mus musculus
<400> 432
   Glu Ile His Thr Val Val Gly Asp Arg
   1 5
<210> 433
   <211> 13
   <212> PRT
   <213> Mus musculus
<400> 433
   Val Trp Ile Ser Gly Glu Glu Thr Leu Ile Ile Ser Lys
   1 5 10
<210> 434
   <211> 10
   <212> PRT
   <213> Mus musculus
<400> 434
   His Leu Val Glu Ile Ile Phe Ser Pro Arg
   1               5                   10
<210> 435
   <211> 8
   <212> PRT
   <213> Mus musculus
<400> 435
   Ile Glu Asp Ile Gln Asn Leu Lys
   1               5
<210> 436
   <211> 7
   <212> PRT
   <213> Mus musculus
<400> 436
   His Ser His Tyr Ile Ser Arg
   1               5
<210> 437
   <211> 11
   <212> PRT
   <213> Mus musculus
<400> 437
   Asp Thr Ser Leu Asn Gly Phe Tyr Ile Pro Lys
   1               5                   10
<210> 438
<211> 8
   <212> PRT
   <213> Mus musculus
<400> 438
   Glu Ala Asn Tyr Leu Val Ser Lys
   1               5
<210> 439
   <211> 12
   <212> PRT
   <213> Mus musculus
<400> 439
   Thr Phe Asn Asp Asn Phe Val Leu Phe Leu Gln Lys
   1 5 10
<210> 440
   <211> 10
   <212> PRT
   <213> Mus musculus
<400> 440
   Asp Asn Gly Gly Leu Ile Pro Glu Glu Lys
   1 5 10
<210> 441
   <211> 11
   <212> PRT
   <213> Mus musculus
<400> 441
   Thr Val Gln Glu His Tyr Gln Asp Phe Asn Lys
   1 5 10
<210> 442
   <211> 11
   <212> PRT
   <213> Mus musculus
<400> 442
   Asp Thr Ser Leu Asn Gly Phe His Ile Pro Lys
   1 5 10
<210> 443
   <211> 8
   <212> PRT
   <213> Mus musculus
<400> 443
   Glu Ala Asn His Leu Val Ser Lys
   1 5
<210> 444
   <211> 9
   <212> PRT
<213> Mus musculus
<400> 444
   Leu Glu Leu Phe Val Val Leu Ala Arg
   1               5
<210> 445
   <211> 9
   <212> PRT
   <213> Mus musculus
<400> 445
   Thr Ile Glu Glu Ala Leu Ile Gln Lys
   1               5
<210> 446
   <211> 10
   <212> PRT
   <213> Mus musculus
<400> 446
   Leu Gly Pro Gly Ser Gln Leu Leu Tyr Arg
   1               5                   10
<210> 447
   <211> 11
   <212> PRT
   <213> Mus musculus
<400> 447
   Ala Ser Ser Ile Ser Gly Tyr Asp Ile Pro Lys
   1               5                   10
<210> 448
   <211> 11
   <212> PRT
   <213> Mus musculus
<400> 448
   Ala Gln Ala Gly Thr Pro Val Ala Ile His Lys
   1               5                   10
<210> 449
   <211> 10
   <212> PRT
   <213> Mus musculus
<400> 449
   Leu Thr Pro Ser Val Pro Phe Thr Thr Arg
   1               5                   10
<210> 450
   <211> 7
   <212> PRT
   <213> Mus musculus
<400> 450
   Asn Pro Gln Val Gln Gln Arg
   1               5
<210> 451
   <211> 10
   <212> PRT
   <213> Mus musculus
<400> 451
   Ile Gln Asp Leu Tyr Ser Glu Leu Asn Lys
   1               5                   10
<210> 452
   <211> 14
   <212> PRT
   <213> Mus musculus
<400> 452
   Asp Thr Glu Glu Glu Ala Leu Thr Phe Ile Ala Ala Ile Lys
   1               5                   10
<210> 453
   <211> 8
   <212> PRT
   <213> Mus musculus
<400> 453
   Thr Glu Ser Ala His Pro Gln Arg
   1               5
<210> 454
   <211> 7
   <212> PRT
   <213> Mus musculus
<400> 454
   Asp Leu Gly Gln Pro Pro Arg
   1               5
<210> 455
   <211> 14
   <212> PRT
   <213> Mus musculus
<400> 455
   Val Trp Gln Ala His Thr Leu Ala Trp Asp Thr Ile Phe Lys
   1               5                   10
<210> 456
   <211> 10
   <212> PRT
   <213> Mus musculus
<400> 456
   Leu Asn Pro Pro Val Pro Gly Gly Phe Arg
   1               5                   10
<210> 457
   <211> 11
   <212> PRT
   <213> Mus musculus
<400> 457
   Phe Ser Phe Ile Pro Phe Gly Gly Gly Leu Arg
   1               5                   10
<210> 458
   <211> 11
   <212> PRT
   <213> Mus musculus
<400> 458
   Thr Phe Glu Leu Asn Gly Tyr Gln Ile Pro Lys
   1               5                   10
<210> 459
   <211> 11
   <212> PRT
   <213> Mus musculus
<400> 459
   Leu Val Ser Val His Trp Pro Ala Ser Val Arg
   1               5                   10
<210> 460
   <211> 8
   <212> PRT
   <213> Mus musculus
<400> 460
   Ile Phe Thr Val Glu Leu Ala Arg
   1 5
<210> 461
   <211> 14
   <212> PRT
   <213> Mus musculus
<400> 461
   Thr Ser Pro Thr Val Tyr Pro Val Asp Asn Leu Pro Ala Arg
   1 5 10
<210> 462
   <211> 7
   <212> PRT
   <213> Mus musculus
<400> 462
   Glu Glu Phe Asn Pro Asp Arg
   1 5
<210> 463
   <211> 11
   <212> PRT
   <213> Mus musculus
<400> 463
   Phe Ile Val Pro His Pro Glu Asp Ala Ser Arg
   1 5 10
<210> 464
   <211> 13
   <212> PRT
   <213> Mus musculus
<400> 464
   Asp Ala Leu Gln Leu Leu Ile Glu His Ser Trp Glu Arg
   1 5 10
<210> 465
   <211> 12
   <212> PRT
   <213> Mus musculus
<400> 465
   Ala Gln Leu Gln Glu Thr Gly Pro Asp Gly Val Arg
   1 5 10
<210> 466
   <211> 10
   <212> PRT
   <213> Mus musculus
<400> 466
   Leu Tyr Pro Val Val Pro Thr Asn Ser Arg
   1 5 10
<210> 467
   <211> 10
   <212> PRT
   <213> Mus musculus
<400> 467
   Glu Val Thr Gly Val Val Pro Phe Gly Lys
   1 5 10
<210> 468
   <211> 11
   <212> PRT
   <213> Mus musculus
<400> 468
   Glu Thr Glu Ile Asn Gly Phe Leu Phe Pro Lys
   1 5 10
<210> 469
   <211> 10
   <212> PRT
   <213> Mus musculus
<400> 469
   Ser Pro Glu Ile Gln Glu Ala Leu His Lys
   1 5 10
<210> 470
   <211> 7
   <212> PRT
   <213> Mus musculus
<400> 470
   Trp Thr Leu Leu Asp Pro Arg
   1 5
<210> 471
   <211> 9
   <212> PRT
   <213> Mus musculus
<400> 471
   Ser Ile Asn Leu Gln Phe Val Asp Arg
   1 5
<210> 472
   <211> 10
   <212> PRT
   <213> Mus musculus
<400> 472
   Leu Tyr Pro Val Val Pro Gly Asn Ser Arg
   1 5 10
<210> 473
   <211> 13
   <212> PRT
   <213> Mus musculus
<400> 473
   Tyr Gly Pro Ile Trp Ser Gly Ser Phe Gly Thr Leu Arg
   1 5 10
<210> 474
   <211> 7
   <212> PRT
   <213> Mus musculus
<400> 474
   Thr Val Leu Val Pro Glu Arg
   1 5
<210> 475
   <211> 7
<212> PRT
   <213> Mus musculus
<400> 475
   Gly Ser Glu Ser Val Leu Arg
   1 5
<210> 476
   <211> 14
   <212> PRT
   <213> Mus musculus
<400> 476
   Phe Gly Leu Glu Ser Ile Gly Ala Val Leu Leu Gly Ser Arg
   1 5 10
<210> 477
   <211> 14
   <212> PRT
   <213> Mus musculus
<400> 477
   Ile His Leu Pro Leu Gln Leu Asp Ala Ser Leu Gly Ser Arg
   1 5 10
<210> 478
   <211> 11
   <212> PRT
   <213> Mus musculus
<400> 478
   Leu His Glu Leu Gln Val His Gly Ala Ala Arg
   1 5 10
<210> 479
   <211> 8
   <212> PRT
   <213> Mus musculus
<400> 479
   Ser Phe Ser Ile Ala Thr Leu Arg
   1 5
<210> 480
   <211> 11
   <212> PRT
   <213> Mus musculus
<400> 480
   Gly Tyr Gly Val Val Phe Ser Ser Gly Glu Arg
   1 5 10
<210> 481
   <211> 13
   <212> PRT
   <213> Mus musculus
<400> 481
   Glu Ala Leu Val Asn Gln Ala Glu Ala Phe Ser Gly Arg
   1 5 10
<210> 482
   <211> 12
   <212> PRT
   <213> Mus musculus
<400> 482
   Phe Thr Asp Leu Ala Pro Ile Gly Leu Pro His Lys
   1 5 10
<210> 483
   <211> 12
   <212> PRT
   <213> Mus musculus
<400> 483
   Thr Pro Pro Gln Tyr Gln Ile His Phe Leu Ser Arg
   1 5 10
<210> 484
   <211> 11
   <212> PRT
   <213> Mus musculus
<400> 484
   Gly Phe Gly Val Val Phe Ser Asn Gly Asn Arg
   1 5 10
<210> 485
   <211> 11
   <212> PRT
   <213> Mus musculus
<400> 485
   Phe Ile Asn Leu Val Pro Asn Asn Ile Pro Arg
   1 5 10
<210> 486
   <211> 11
   <212> PRT
   <213> Mus musculus
<400> 486
   Val Gln Glu Glu Ile Asp His Val Ile Gly Arg
   1 5 10
<210> 487
   <211> 13
   <212> PRT
   <213> Mus musculus
<400> 487
   Glu Ala Leu Ile Asp His Gly Glu Glu Phe Ser Asp Arg
   1               5                   10
<210> 488
   <211> 14
   <212> PRT
   <213> Mus musculus
<400> 488
   Ile Asn Asn Gly Leu Gly Ile Val Phe Ser Asn Gly Asn Arg
   1               5                   10
<210> 489
   <211> 13
   <212> PRT
   <213> Mus musculus
<400> 489
   Glu Ala Leu Ile Asp Gln Gly Asp Glu Phe Ser Asp Lys
   1               5                   10
<210> 490
   <211> 11
   <212> PRT
   <213> Mus musculus
<400> 490
   Ile Gln Glu Glu Ile Ala His Val Ile Gly Arg
   1               5                   10
<210> 491
   <211> 8
   <212> PRT
   <213> Mus musculus
<400> 491
   Thr Asp Ser Ser Leu Leu Ser Arg
   1               5
<210> 492
   <211> 10
   <212> PRT
   <213> Mus musculus
<400> 492
   Phe Asp Pro Gly His Phe Leu Asp Glu Lys
   1 5 10
<210> 493
   <211> 11
   <212> PRT
   <213> Mus musculus
<400> 493
   Phe Gly Asp Ile Ala Pro Leu Asn Leu Pro Arg
   1 5 10
<210> 494
   <211> 8
   <212> PRT
   <213> Mus musculus
<400> 494
   Phe Gln Asp Phe Phe Ile Pro Lys
   1 5
<210> 495
   <211> 10
   <212> PRT
   <213> Mus musculus
<400> 495
   Leu Asn Ser Phe Ile Ala Leu Val Asn Lys
   1 5 10
<210> 496
   <211> 10
   <212> PRT
   <213> Mus musculus
<400> 496
   Gly Asn Pro Glu Ser Ser Phe Asn Asp Lys
   1 5 10
<210> 497
   <211> 12
   <212> PRT
   <213> Mus musculus
<400> 497
   Gly Val Ile Leu Ala Pro Tyr Gly Pro Glu Trp Arg
   1 5 10
<210> 498
   <211> 10
   <212> PRT
   <213> Mus musculus
<400> 498
   Thr Thr Trp Asp Pro Val Gln Ala Pro Arg
   1 5 10
<210> 499
   <211> 14
   <212> PRT
   <213> Mus musculus
<400> 499
   Ser Phe Ile Ala Ile Leu Asp Asn Leu Leu Thr Glu Asn Arg
   1 5 10
<210> 500
   <211> 7
   <212> PRT
   <213> Mus musculus
<400> 500
   Tyr Pro Glu Ile Glu Glu Lys
   1 5
<210> 501
   <211> 10
   <212> PRT
   <213> Mus musculus
<400> 501
   Ser Gln Asp Leu Leu Thr Ser Leu Thr Lys
   1 5 10
<210> 502
   <211> 13
   <212> PRT
   <213> Mus musculus
<400> 502
   Leu Leu Thr Ile Ile His Phe Ile Asn Asp Asn Phe Lys
   1 5 10
<210> 503
   <211> 11
   <212> PRT
   <213> Mus musculus
<400> 503
   Gly Asn Gly Ile Ala Phe Ser Asp Gly Glu Arg
   1 5 10
<210> 504
   <211> 12
   <212> PRT
   <213> Mus musculus
<400> 504
   Glu His Gln Asp Ser Leu Asp Pro Asn Ser Pro Arg
   1 5 10
<210> 505
   <211> 7
   <212> PRT
   <213> Mus musculus
<400> 505
   Phe Asp Tyr Asp Asp Glu Arg
   1 5
<210> 506
<211> 13
   <212> PRT
   <213> Mus musculus
<400> 506
   Leu Pro Phe Val Gly Asn Phe Phe Gln Ile Asp Thr Lys
   1 5 10
<210> 507
   <211> 12
   <212> PRT
   <213> Mus musculus
<400> 507
   Asn Gly Leu Val Val Ser Asn Gly Gln Thr Trp Lys
   1 5 10
<210> 508
   <211> 12
   <212> PRT
   <213> Mus musculus
<400> 508
   Glu Glu Gly Gly Gln Pro Phe Asn Pro His Leu Lys
   1 5 10
<210> 509
   <211> 8
   <212> PRT
   <213> Mus musculus
<400> 509
   Phe Asn Gly Phe His Leu Pro Lys
   1 5
<210> 510
   <211> 7
   <212> PRT
   <213> Mus musculus
<400> 510
   Glu Val Thr Val Asp Thr Lys
   1 5
<210> 511
   <211> 13
   <212> PRT
   <213> Mus musculus
<400> 511
   Ile Gln Glu Glu Thr His His Leu Val Glu Ala Ile Arg
   1 5 10
<210> 512
   <211> 11
   <212> PRT
<213> Mus musculus
<400> 512
   Asn Ala Asp Val Val Tyr Asp Phe Leu Ser Arg
   1 5 10
<210> 513
   <211> 7
   <212> PRT
   <213> Mus musculus
<400> 513
   Gly Gly Pro Phe Asp Leu Lys
   1 5
<210> 514
   <211> 9
   <212> PRT
   <213> Mus musculus
<400> 514
   Asp Val Val Asp Ala Phe Leu Leu Lys
   1 5
<210> 515
   <211> 8
   <212> PRT
   <213> Mus musculus
<400> 515
   Gly Thr Leu Ala Thr Leu Asp Lys
   1 5
<210> 516
   <211> 13
   <212> PRT
   <213> Mus musculus
<400> 516
   Leu Pro Tyr Thr Asp Ala Val Leu His Glu Ala Gln Arg
   1 5 10
<210> 517
   <211> 8
   <212> PRT
   <213> Mus musculus
<400> 517
   Tyr Ala Leu Leu Leu Leu Leu Arg
   1 5
<210> 518
   <211> 7
   <212> PRT
   <213> Mus musculus
<400> 518
   Tyr Pro Gln Val Gln Gln Arg
   1 5
<210> 519
   <211> 10
   <212> PRT
   <213> Mus musculus
<400> 519
   Gln Asp Pro Gly Thr Glu Phe Thr Glu Lys
   1 5 10
<210> 520
   <211> 11
   <212> PRT
   <213> Mus musculus
<400> 520
   Glu Glu Leu Ile Gln Glu Leu Gly Pro Gly Arg
   1 5 10
<210> 521
   <211> 13
   <212> PRT
   <213> Mus musculus
<400> 521
   Glu Ala Leu Gly Gly Gln Ala Glu Glu Phe Ser Gly Arg
   1 5 10
<210> 522
   <211> 9
   <212> PRT
   <213> Mus musculus
<400> 522
   Val Trp Pro Thr Gly Asp Gln Ser Arg
   1 5
<210> 523
   <211> 8
   <212> PRT
   <213> Mus musculus
<400> 523
   Phe Gln Thr Ser Gly Pro Ala Arg
   1 5
<210> 524
   <211> 7
   <212> PRT
   <213> Mus musculus
<400> 524
   Ala Pro Ser Leu Ser Asp Arg
   1 5
<210> 525
   <211> 7
   <212> PRT
   <213> Mus musculus
<400> 525
   Phe Leu Asp Glu Asp Gly Arg
   1 5
<210> 526
   <211> 14
   <212> PRT
   <213> Mus musculus
<400> 526
   Thr Phe Asp Gly His Gly Val Phe Phe Ala Asn Gly Glu Arg
   1 5 10
<210> 527
   <211> 7
   <212> PRT
   <213> Mus musculus
<400> 527
   Glu Phe Tyr Gln Gln Phe Lys
   1 5
<210> 528
   <211> 7
   <212> PRT
   <213> Mus musculus
<400> 528
   Leu Tyr Pro Ile Ala Asn Arg
   1 5
<210> 529
   <211> 13
   <212> PRT
   <213> Mus musculus
<400> 529
   Gln Pro Val Leu Ala Ile Thr Asp Pro Asp Ile Ile Lys
   1 5 10
<210> 530
   <211> 13
   <212> PRT
   <213> Mus musculus
<400> 530
   Gln Gly Leu Leu Gln Pro Glu Asn Pro Leu Leu Leu Lys
   1 5 10
<210> 531
   <211> 12
   <212> PRT
   <213> Mus musculus
<400> 531
   Asp Val Ile Ser Phe Phe Thr Thr Ser Val Glu Arg
   1 5 10
<210> 532
   <211> 12
   <212> PRT
   <213> Mus musculus
<400> 532
   Thr Asp Val Glu Ile Asn Gly Leu Phe Ile Pro Lys
   1 5 10
<210> 533
   <211> 7
   <212> PRT
   <213> Mus musculus
<400> 533
   Glu Thr Glu Ile Pro Leu Lys
   1 5
<210> 534
   <211> 7
   <212> PRT
   <213> Mus musculus
<400> 534
   Leu Tyr Pro Ile Ala Gly Arg
   1 5
<210> 535
   <211> 13
   <212> PRT
   <213> Mus musculus
<400> 535
   Ala Leu Leu Ser Pro Thr Phe Thr Ser Gly Asn Leu Lys
   1 5 10
<210> 536
   <211> 7
   <212> PRT
   <213> Mus musculus
<400> 536
   Leu Tyr Pro Ile Thr Asn Arg
   1 5
<210> 537
   <211> 7
   <212> PRT
   <213> Mus musculus
<400> 537
   Leu Tyr Pro Ile Ala Ile Arg
   1 5
<210> 538
   <211> 7
   <212> PRT
   <213> Mus musculus
<400> 538
   Phe Ala Leu Ile Ser Ile Lys
   1 5
<210> 539
   <211> 9
   <212> PRT
   <213> Mus musculus
<400> 539
   Ala Leu Gln Thr Val Phe Gly Glu Arg
   1 5
<210> 540
   <211> 9
   <212> PRT
   <213> Mus musculus
<400> 540
   Leu Gln Tyr Leu Ser Gln Val Leu Lys
   1 5
<210> 541
   <211> 12
   <212> PRT
   <213> Mus musculus
<400> 541
   Leu Leu Glu Glu Glu Thr Leu Ile Asp Gly Val Arg
   1 5 10
<210> 542
   <211> 11
   <212> PRT
   <213> Mus musculus
<400> 542
   Ala Glu Gln Leu Val Glu Ile Leu Glu Ala Lys
   1 5 10
<210> 543
   <211> 10
<212> PRT
   <213> Mus musculus
<400> 543
   Leu Tyr Pro Pro Ala Trp Gly Thr Phe Arg
   1 5 10
<210> 544
   <211> 7
   <212> PRT
   <213> Mus musculus
<400> 544
   Gln Pro Glu Ile Val Ala Arg
   1 5
<210> 545
   <211> 11
   <212> PRT
   <213> Mus musculus
<400> 545
   Phe Thr Tyr Phe Pro Phe Ser Leu Gly His Arg
   1 5 10
<210> 546
   <211> 10
   <212> PRT
   <213> Mus musculus
<400> 546
   Val Leu Leu Tyr Asp Pro Asp Tyr Val Lys
   1 5 10
<210> 547
   <211> 10
   <212> PRT
   <213> Mus musculus
<400> 547
   Leu Tyr Pro Pro Val Ile Ser Val Ser Arg
   1 5 10
<210> 548
   <211> 9
   <212> PRT
   <213> Mus musculus
<400> 548
   Asn Ser Leu Ser Asp Glu Asp Leu Arg
   1 5
<210> 549
   <211> 11
   <212> PRT
   <213> Mus musculus
<400> 549
   Glu Leu Gln Gln Ile Leu Ile Trp Val Glu Lys
   1               5                   10
<210> 550
   <211> 7
   <212> PRT
   <213> Mus musculus
<400> 550
   Ile Pro Val Pro Ile Ala Arg
   1               5
<210> 551
   <211> 10
   <212> PRT
   <213> Mus musculus
<400> 551
   Ala Leu Asp Ser Phe Pro Gly Pro Pro Lys
   1               5                   10
<210> 552
   <211> 7
   <212> PRT
   <213> Mus musculus
<400> 552
   Val Pro Gln Leu Ile Leu Arg
   1               5
<210> 553
   <211> 9
   <212> PRT
   <213> Mus musculus
<400> 553
   Phe Glu Phe Ser Pro Asp Pro Ser Lys
   1               5
<210> 554
   <211> 12
   <212> PRT
   <213> Mus musculus
<400> 554
   His Trp Leu Phe Gly His Ala Leu Glu Ile Gln Lys
   1               5                   10
<210> 555
   <211> 10
   <212> PRT
   <213> Mus musculus
<400> 555
   Ile Leu Ile Gln Ile Tyr Ala Ala Tyr Arg
   1 5 10
<210> 556
   <211> 12
   <212> PRT
   <213> Mus musculus
<400> 556
   Thr Leu Pro Asp Gln Gly Leu Asp Asp Phe Leu Lys
   1 5 10
<210> 557
   <211> 13
   <212> PRT
   <213> Mus musculus
<400> 557
   Thr Leu Leu Leu Leu Gly Ala Ser Trp Ile Leu Ala Arg
   1 5 10
<210> 558
   <211> 8
   <212> PRT
   <213> Mus musculus
<400> 558
   Leu Ile Ser Asp Gly Ser Ala Arg
   1 5
<210> 559
   <211> 10
   <212> PRT
   <213> Mus musculus
<400> 559
   Val Phe Pro Ser Val Pro Leu Phe Ala Arg
   1 5 10
<210> 560
   <211> 12
   <212> PRT
   <213> Mus musculus
<400> 560
   Ala Glu Asn Val Glu Val Ile Leu Thr Ser Ser Lys
   1 5 10
<210> 561
   <211> 13
   <212> PRT
   <213> Mus musculus
<400> 561
   Leu Trp Ile Gly Pro Val Pro Leu Val Ala Leu Tyr Lys
   1 5 10
<210> 562
   <211> 9
   <212> PRT
   <213> Mus musculus
<400> 562
   Phe Phe Pro Glu Asn Ser Gln Gly Arg
   1 5
<210> 563
   <211> 13
   <212> PRT
   <213> Mus musculus
<400> 563
   His Pro Tyr Ala Tyr Val Pro Phe Ser Ala Gly Pro Arg
   1 5 10
<210> 564
   <211> 7
   <212> PRT
   <213> Mus musculus
<400> 564
   Ser Ile Pro Ser Val Ala Arg
   1 5
<210> 565
   <211> 13
   <212> PRT
   <213> Mus musculus
<400> 565
   Gly Thr Glu Ala Ile Ile Ile Pro Tyr Ala Leu His Arg
   1 5 10
<210> 566
   <211> 7
   <212> PRT
   <213> Mus musculus
<400> 566
   Leu Ser Gln Glu Asp Ile Arg
   1 5
<210> 567
   <211> 12
   <212> PRT
   <213> Mus musculus
<400> 567
   Asp Glu Leu Asn Gly Phe Phe Asn Thr Leu Ile Arg
   1 5 10
<210> 568
   <211> 9
   <212> PRT
   <213> Mus musculus
<400> 568
   Leu Thr Ile Leu Gln Val Leu His Lys
   1 5
<210> 569
   <211> 7
   <212> PRT
   <213> Mus musculus
<400> 569
   Leu Gly Leu Leu Val Val Lys
   1 5
<210> 570
   <211> 7
   <212> PRT
   <213> Mus musculus
<400> 570
   Asp Ala Phe Asn Ile Gln Arg
   1 5
<210> 571
   <211> 9
   <212> PRT
   <213> Mus musculus
<400> 571
   Ala Leu Asp Phe Gln Ser Tyr Ala Lys
   1 5
<210> 572
   <211> 9
   <212> PRT
   <213> Mus musculus
<400> 572
   Val Val Gln Glu Asp Tyr Val Leu Lys
   1 5
<210> 573
   <211> 11
   <212> PRT
   <213> Mus musculus
<400> 573
   Ala Gly Phe Leu Ser Leu Phe Gly Tyr Thr Lys
   1 5 10
<210> 574
<211> 9
   <212> PRT
   <213> Mus musculus
<400> 574
   Phe Val Tyr Ser Leu Leu Gly Pro Arg
   1 5
<210> 575
   <211> 10
   <212> PRT
   <213> Mus musculus
<400> 575
   Glu Trp Val Glu Val Ser Gln Leu Gln Arg
   1               5                   10
<210> 576
   <211> 12
   <212> PRT
   <213> Mus musculus
<400> 576
   Val Phe Gly Tyr Gln Ser Val Asp Gly Asp His Arg
   1               5                   10
<210> 577
   <211> 10
   <212> PRT
   <213> Mus musculus
<400> 577
   Val Pro Pro Leu Ile Ala Ser Phe Val Arg
   1               5                   10
<210> 578
   <211> 14
   <212> PRT
   <213> Mus musculus
<400> 578
   Gly Ser Ala Pro Pro Gly Pro Val Pro Glu Gly Gln Ile Arg
   1               5                   10
<210> 579
   <211> 8
   <212> PRT
   <213> Mus musculus
<400> 579
   Ala Leu Trp Ser Ser Ser Glu Lys
   1               5
<210> 580
   <211> 9
   <212> PRT
<213> Mus musculus
<400> 580
   Thr Glu Asn Ile Glu Thr Ile Asp Arg
   1 5
<210> 581
   <211> 10
   <212> PRT
   <213> Mus musculus
<400> 581
   Ala Pro Phe Phe Glu Tyr Asn Ile His Lys
   1 5 10
<210> 582
   <211> 12
   <212> PRT
   <213> Mus musculus
<400> 582
   Glu Leu Ser Glu Glu Asp Val Asp Ala Val Val Arg
   1 5 10
<210> 583
   <211> 8
   <212> PRT
   <213> Mus musculus
<400> 583
   Asp Glu Tyr Leu Leu Asn Phe Lys
   1 5
<210> 584
   <211> 13
   <212> PRT
   <213> Mus musculus
<400> 584
   Ile Phe Ser Ser His Ile Pro Tyr Tyr Leu Val Pro Lys
   1 5 10
<210> 585
   <211> 8
   <212> PRT
   <213> Mus musculus
<400> 585
   Ala Asn Tyr Glu His Ile Ile Lys
   1 5
<210> 586
   <211> 9
   <212> PRT
   <213> Mus musculus
<400> 586
   Gly Leu Ala Leu Pro Gln Asp Phe Arg
   1 5
<210> 587
   <211> 10
   <212> PRT
   <213> Mus musculus
<400> 587
   Asp Ser Ser Asp Val Tyr Ser Thr Gln Lys
   1 5 10
<210> 588
   <211> 12
   <212> PRT
   <213> Mus musculus
<400> 588
   Gly Ala Ser Ala Ala Gly Ser Ala Leu Val Tyr Arg
   1 5 10
<210> 589
   <211> 13
   <212> PRT
   <213> Mus musculus
<400> 589
   Gln Leu Glu Phe Leu Gln Pro Leu Val Glu Asp Pro Arg
   1 5 10
<210> 590
   <211> 7
   <212> PRT
   <213> Mus musculus
<400> 590
   Ile Val Ala Phe Ala Gly Lys
   1 5
<210> 591
   <211> 8
   <212> PRT
   <213> Mus musculus
<400> 591
   Asp Ala Ala Ser Leu Thr Asn Arg
   1               5
<210> 592
   <211> 8
   <212> PRT
   <213> Mus musculus
<400> 592
   Leu Ser Ala Glu Leu Gly Leu Arg
   1               5
<210> 593
   <211> 7
   <212> PRT
   <213> Mus musculus
<400> 593
   Thr Val Phe Phe Gln Gln Arg
   1               5
<210> 594
   <211> 8
   <212> PRT
   <213> Mus musculus
<400> 594
   Ser Ile Ser Leu Leu Glu Glu Arg
   1               5
<210> 595
   <211> 7
   <212> PRT
   <213> Mus musculus
<400> 595
   Asp Leu Val His Ser Leu Lys
   1               5
<210> 596
   <211> 11
   <212> PRT
   <213> Mus musculus
<400> 596
   Thr Ala His Leu His Val Leu Ala Val His Arg
   1 5 10
<210> 597
   <211> 9
   <212> PRT
   <213> Mus musculus
<400> 597
   Gly Ala Ala Val Ala Leu Asn Ile Arg
   1 5
<210> 598
   <211> 13
   <212> PRT
   <213> Mus musculus
<400> 598
   Tyr Val Thr Leu Ile Tyr Thr Asn Tyr Glu Asn Gly Lys
   1 5 10
<210> 599
   <211> 7
   <212> PRT
   <213> Mus musculus
<400> 599
   Gly Leu Thr His Ser Ile Arg
   1 5
<210> 600
   <211> 9
   <212> PRT
   <213> Mus musculus
<400> 600
   Ala Ile Leu Ser Tyr Leu Ala Ala Lys
   1 5
<210> 601
   <211> 7
   <212> PRT
   <213> Mus musculus
<400> 601
   Leu Tyr Tyr Phe Asn Gly Arg
   1 5
<210> 602
   <211> 10
   <212> PRT
   <213> Mus musculus
<400> 602
   Glu Glu Ser Tyr Asp Leu Ile Leu Ser Arg
   1               5                   10
<210> 603
   <211> 7
   <212> PRT
   <213> Mus musculus
<400> 603
   Tyr Phe Pro Val Phe Glu Lys
   1               5
<210> 604
   <211> 9
   <212> PRT
   <213> Mus musculus
<400> 604
   Ala Ile Leu Asn Tyr Ile Ala Ser Lys
   1               5
<210> 605
   <211> 7
   <212> PRT
   <213> Mus musculus
<400> 605
   Tyr Phe Pro Ala Phe Glu Lys
   1               5
<210> 606
   <211> 9
   <212> PRT
   <213> Mus musculus
<400> 606
   Ile Ala Ala Phe Leu Gln Ser Asp Arg
   1               5
<210> 607
   <211> 8
   <212> PRT
   <213> Mus musculus
<400> 607
   Gln Phe Ser Leu Phe Leu Gly Lys
   1 5
<210> 608
   <211> 8
   <212> PRT
   <213> Mus musculus
<400> 608
   Phe Thr Trp Phe Ala Gly Glu Lys
   1 5
<210> 609
   <211> 7
   <212> PRT
   <213> Mus musculus
<400> 609
   Ser Gln Trp Leu Asp Val Lys
   1 5
<210> 610
   <211> 9
   <212> PRT
   <213> Mus musculus
<400> 610
   Asn Glu Asp Leu Ile Asn Gly Ile Lys
   1 5
<210> 611
   <211> 8
<212> PRT
   <213> Mus musculus
<400> 611
   Asn His Phe Pro Glu Ala Leu Arg
   1 5
<210> 612
   <211> 8
   <212> PRT
   <213> Mus musculus
<400> 612
   Ser Phe Ser Glu Phe Val Glu Lys
   1               5
<210> 613
   <211> 7
   <212> PRT
   <213> Mus musculus
<400> 613
   Glu Asp Ala Ile Phe Asn Arg
   1               5
<210> 614
   <211> 9
   <212> PRT
   <213> Mus musculus
<400> 614
   Asp Asp Val Asp Leu Val Glu Phe Lys
   1               5
<210> 615
   <211> 12
   <212> PRT
   <213> Mus musculus
<400> 615
   Tyr Val Asn Glu Glu Glu Ile Glu Asp Val Leu Lys
   1               5                   10
<210> 616
   <211> 10
   <212> PRT
   <213> Mus musculus
<400> 616
   Asp Gln Pro Gln Val Pro Ala Ile Phe Arg
   1               5                   10
<210> 617
   <211> 10
   <212> PRT
   <213> Mus musculus
<400> 617
   Asp Tyr Ile Val Asp Ala Gly Phe Gly Arg
   1 5 10
<210> 618
   <211> 9
   <212> PRT
   <213> Mus musculus
<400> 618
   Asp Asn Val Asp Leu Val Glu Phe Lys
   1 5
<210> 619
   <211> 12
   <212> PRT
   <213> Mus musculus
<400> 619
   Ser Leu Thr Glu Glu Glu Ile Glu Asp Val Leu Arg
   1 5 10
<210> 620
   <211> 7
   <212> PRT
   <213> Mus musculus
<400> 620
   Ile Gly Tyr Gln Ser Thr Arg
   1 5
<210> 621
   <211> 9
   <212> PRT
   <213> Mus musculus
<400> 621
   Glu Glu Glu Ile Glu Glu Val Leu Lys
   1 5
<210> 622
   <211> 9
   <212> PRT
   <213> Mus musculus
<400> 622
   Asp Asn Ile Asp Leu Val Glu Phe Lys
   1 5
<210> 623
   <211> 12
   <212> PRT
   <213> Mus musculus
<400> 623
   Asp Gln Pro Gln Val Pro Ala Ile Phe His Leu Arg
   1 5 10
<210> 624
   <211> 10
   <212> PRT
   <213> Mus musculus
<400> 624
   Ser Val Phe Gly Ile His Leu Glu Thr Lys
   1 5 10
<210> 625
   <211> 11
   <212> PRT
   <213> Mus musculus
<400> 625
   Asp Val Val Val Ser Tyr Tyr Asn Phe Tyr Lys
   1 5 10
<210> 626
   <211> 11
   <212> PRT
   <213> Mus musculus
<400> 626
   Val Ser Tyr Gly Ser Trp Tyr Gln His Val Lys
   1 5 10
<210> 627
   <211> 8
   <212> PRT
   <213> Mus musculus
<400> 627
   Thr Tyr Ala Phe Pro Tyr Thr Lys
   1 5
<210> 628
   <211> 13
   <212> PRT
   <213> Mus musculus
<400> 628
   Glu Glu Tyr Gln Gln Glu Ile Leu Ser Asp Ile Glu Lys
   1               5                   10
<210> 629
   <211> 8
   <212> PRT
   <213> Mus musculus
<400> 629
   Gly Glu Asp Phe Phe Thr Leu Lys
   1               5
<210> 630
   <211> 9
   <212> PRT
   <213> Mus musculus
<400> 630
   Asn Thr Gly Val Tyr Leu Ile Ser Arg
   1               5
<210> 631
   <211> 12
   <212> PRT
   <213> Mus musculus
<400> 631
   Asp Ile Ala Pro Ser Asn His Tyr Ser Leu Gln Arg
   1               5                   10
<210> 632
   <211> 10
   <212> PRT
   <213> Mus musculus
<400> 632
   Thr Thr Tyr Trp Ile Asp Tyr Ile Leu Arg
   1               5                   10
<210> 633
   <211> 10
   <212> PRT
   <213> Mus musculus
<400> 633
   Ile Gly Val Ser Phe Leu Val Leu Pro Lys
   1               5                   10
<210> 634
   <211> 7
   <212> PRT
   <213> Mus musculus
<400> 634
   Tyr Asn Leu Leu Pro Ala Lys
   1               5
<210> 635
   <211> 9
   <212> PRT
   <213> Mus musculus
<400> 635
   Tyr Leu Ser Glu Asp Ile Ala Asn Lys
   1               5
<210> 636
   <211> 9
   <212> PRT
   <213> Mus musculus
<400> 636
   Thr Leu Ala Ser Ala Leu His Glu Arg
   1               5
<210> 637
   <211> 7
   <212> PRT
   <213> Mus musculus
<400> 637
   Leu Ala Gly Ala Leu Gly Arg
   1               5
<210> 638
   <211> 12
   <212> PRT
   <213> Mus musculus
<400> 638
   Gly His His Thr Val Leu Leu Leu Ser Glu Gly Arg
   1 5 10
<210> 639
   <211> 11
   <212> PRT
   <213> Mus musculus
<400> 639
   Tyr Phe Ser Leu Pro Ser Val Val Phe Ser Arg
   1 5 10
<210> 640
   <211> 8
   <212> PRT
   <213> Mus musculus
<400> 640
   Ile Phe Ile Asp Ala Gln Trp Lys
   1 5
<210> 641
   <211> 13
   <212> PRT
   <213> Mus musculus
<400> 641
   Thr Tyr Ala Val Ser His Thr Gln Glu Asp Leu Asn Arg
   1 5 10
<210> 642
<211> 9
   <212> PRT
   <213> Mus musculus
<400> 642
   Asp Ile Ser Glu Glu Val Leu Asn Lys
   1 5
<210> 643
   <211> 11
   <212> PRT
   <213> Mus musculus
<400> 643
   Val Leu Trp Gly Ser Trp Tyr Asp His Val Lys
   1 5 10
<210> 644
   <211> 12
   <212> PRT
   <213> Mus musculus
<400> 644
   Val Phe Asp Val Ala Val Leu Ala Pro Leu Leu Lys
   1 5 10
<210> 645
   <211> 9
   <212> PRT
   <213> Mus musculus
<400> 645
   Thr Ala Leu Thr Phe Gln Gln Ile Lys
   1 5
<210> 646
   <211> 10
   <212> PRT
   <213> Mus musculus
<400> 646
   Tyr Glu Asp Leu Val Gly Asp Pro Val Lys
   1 5 10
<210> 647
   <211> 7
   <212> PRT
   <213> Mus musculus
<400> 647
   Asp Pro Ala Leu Asp Leu Lys
   1 5
<210> 648
   <211> 8
   <212> PRT
<213> Mus musculus
<400> 648
   Val Asn Ser Pro Glu Glu Val Lys
   1 5
<210> 649
   <211> 14
   <212> PRT
   <213> Mus musculus
<400> 649
   Leu Tyr Pro Gly Asp Ala Val Ser Leu Gln Gly Ala Ala Arg
   1 5 10
<210> 650
   <211> 7
   <212> PRT
   <213> Mus musculus
<400> 650
   Glu Ser Leu Gln Val Val Arg
   1 5
<210> 651
   <211> 13
   <212> PRT
   <213> Mus musculus
<400> 651
   Ile Ala Ser Gly Leu Gly Leu Ala Trp Ile Ile Gly Arg
   1 5 10
<210> 652
   <211> 9
   <212> PRT
   <213> Mus musculus
<400> 652
   Asn Leu Asp Glu Asp Val Val Asp Lys
   1 5
<210> 653
   <211> 9
   <212> PRT
   <213> Mus musculus
<400> 653
   Ile Val Leu Glu Thr Ser Phe Glu Lys
   1 5
<210> 654
   <211> 10
   <212> PRT
   <213> Mus musculus
<400> 654
   Asn His Phe Thr Val Ala Gln Asn Glu Arg
   1 5 10
<210> 655
   <211> 11
   <212> PRT
   <213> Mus musculus
<400> 655
   Val Ser Trp Gly Ser Trp Phe Asp His Val Lys
   1 5 10
<210> 656
   <211> 11
   <212> PRT
   <213> Mus musculus
<400> 656
   Leu Glu Leu Leu Ala Tyr Leu Leu Gly Glu Lys
   1 5 10
<210> 657
   <211> 11
   <212> PRT
   <213> Mus musculus
<400> 657
   Gln Phe Phe Gln Val Pro Leu Asn Ile Pro Lys
   1 5 10
<210> 658
   <211> 7
   <212> PRT
   <213> Mus musculus
<400> 658
   Ile Ser Thr Gln Gln Val Lys
   1 5
<210> 659
   <211> 8
   <212> PRT
   <213> Mus musculus
<400> 659
   Tyr Gln His Leu Trp Asn Ile Lys
   1 5
<210> 660
   <211> 9
   <212> PRT
   <213> Mus musculus
<400> 660
   Ala Asp Val Gly Leu Val Glu Asp Lys
   1 5
<210> 661
   <211> 12
   <212> PRT
   <213> Mus musculus
<400> 661
   Leu Leu Asp Leu Gly Val Leu Val Pro Leu Leu Arg
   1 5 10
<210> 662
   <211> 11
   <212> PRT
   <213> Mus musculus
<400> 662
   Leu Tyr Ala Gln Pro Gly Asp Pro Gly Glu Arg
   1 5 10
<210> 663
   <211> 9
   <212> PRT
   <213> Mus musculus
<400> 663
   Glu Gly Glu Thr Pro Leu Glu Thr Lys
   1 5
<210> 664
   <211> 9
   <212> PRT
   <213> Mus musculus
<400> 664
   Ser Leu Ala Glu Gly Asn Pro Asp Arg
   1 5
<210> 665
   <211> 7
   <212> PRT
   <213> Mus musculus
<400> 665
   Glu Gln Gly Ala Glu Val Arg
   1 5
<210> 666
   <211> 7
   <212> PRT
   <213> Mus musculus
<400> 666
   Asp Pro Gly Leu Asn Leu Lys
   1 5
<210> 667
   <211> 7
   <212> PRT
   <213> Mus musculus
<400> 667
   Ala Leu Pro Ser Ala Pro Arg
   1 5
<210> 668
   <211> 7
   <212> PRT
   <213> Mus musculus
<400> 668
   Leu Ser Gln Glu Gln Val Arg
   1 5
<210> 669
   <211> 7
   <212> PRT
   <213> Mus musculus
<400> 669
   Glu Ser Ile Gln Val Leu Arg
   1 5
<210> 670
   <211> 7
   <212> PRT
   <213> Mus musculus
<400> 670
   Glu Ala Val His Ala Trp Arg
   1 5
<210> 671
   <211> 10
   <212> PRT
   <213> Mus musculus
<400> 671
   Gln His Ile Tyr Val His Ala Thr Trp Arg
   1 5 10
<210> 672
   <211> 10
   <212> PRT
   <213> Mus musculus
<400> 672
   Asn Pro Ala Ile Ser Leu Val Glu Glu Arg
   1 5 10
<210> 673
   <211> 8
   <212> PRT
   <213> Mus musculus
<400> 673
   Val Pro Glu Val Asn Asp Leu Arg
   1 5
<210> 674
   <211> 8
   <212> PRT
   <213> Mus musculus
<400> 674
   Asn Ser Ala Ala Thr Ala Glu Lys
   1 5
<210> 675
   <211> 7
   <212> PRT
   <213> Mus musculus
<400> 675
   Ala Leu Val Glu Asp Pro Arg
   1               5
<210> 676
   <211> 7
   <212> PRT
   <213> Mus musculus
<400> 676
   Gly Asp Pro Thr Leu Gly Lys
   1               5
<210> 677
   <211> 10
   <212> PRT
   <213> Mus musculus
<400> 677
   Asp Leu Ser Leu Asp Leu Leu Leu Pro Arg
   1               5                   10
<210> 678
   <211> 10
   <212> PRT
   <213> Mus musculus
<400> 678
   Asp Ala Leu Ser Val Ser Gln Ala Trp Arg
   1               5                   10
<210> 679
   <211> 12
<212> PRT
   <213> Mus musculus
<400> 679
   Gln Val Pro Ser Ser Pro Ala Gly Leu Gly Glu Arg
   1               5                   10
<210> 680
   <211> 7
   <212> PRT
   <213> Mus musculus
<400> 680
   Asp Pro Leu Thr Val Ile Arg
   1 5
<210> 681
   <211> 9
   <212> PRT
   <213> Mus musculus
<400> 681
   Ser Val His Ser Glu Leu Glu Gln Arg
   1 5
<210> 682
   <211> 7
   <212> PRT
   <213> Mus musculus
<400> 682
   His Thr Leu Pro Phe Ala Lys
   1 5
<210> 683
   <211> 7
   <212> PRT
   <213> Mus musculus
<400> 683
   Trp Ala Ser Ser Thr Glu Lys
   1 5
<210> 684
   <211> 11
   <212> PRT
   <213> Mus musculus
<400> 684
   Glu Pro Ile Leu Ala Ala Gln Ala Val Pro Arg
   1 5 10
<210> 685
   <211> 9
   <212> PRT
   <213> Mus musculus
<400> 685
   Gln Pro Glu Leu Ser Leu Ser Pro Arg
   1 5
<210> 686
   <211> 7
   <212> PRT
   <213> Mus musculus
<400> 686
   Gln Pro Glu Leu Thr Leu Arg
   1               5
<210> 687
   <211> 12
   <212> PRT
   <213> Mus musculus
<400> 687
   Ala Ile Gln Thr Leu Pro Glu Ala Leu Gln Gln Arg
   1               5                   10
<210> 688
   <211> 8
   <212> PRT
   <213> Mus musculus
<400> 688
   Ala Pro Leu Ala Gln Thr Thr Arg
   1               5
<210> 689
   <211> 8
   <212> PRT
   <213> Mus musculus
<400> 689
   Ser His Gly Phe Val Val Leu Lys
   1               5
<210> 690
   <211> 7
   <212> PRT
   <213> Mus musculus
<400> 690
   Ser Gln Val Asp Ile Val Lys
   1               5
<210> 691
   <211> 7
   <212> PRT
   <213> Mus musculus
<400> 691
   Trp Ser Leu Pro Tyr Glu Lys
   1               5
<210> 692
   <211> 11
   <212> PRT
   <213> Mus musculus
<400> 692
   Val Ile Thr Ser Gly Phe Asn Ala Leu Glu Lys
   1               5                   10
<210> 693
   <211> 8
   <212> PRT
   <213> Mus musculus
<400> 693
   Ile Leu Gln Ser Thr Ala Gly Lys
   1               5
<210> 694
   <211> 13
   <212> PRT
   <213> Mus musculus
<400> 694
   Gly Ile Asp Tyr Glu Ile Val Pro Ile Asn Leu Ile Lys
   1               5                   10
<210> 695
   <211> 14
   <212> PRT
   <213> Mus musculus
<400> 695
   Val Asp Leu Ser Pro Tyr Pro Thr Ile Ser His Ile Asn Lys
   1               5                   10
<210> 696
   <211> 11
   <212> PRT
   <213> Homo sapiens
<400> 696
   Val Ala Leu Thr Leu Asp Leu Asp Leu Glu Lys
   1 5 10
<210> 697
   <211> 8
   <212> PRT
   <213> Homo sapiens
<400> 697
   Gln Glu Leu Ile Ala Trp Glu Lys
   1 5

## Description

### Technical Field

The present invention relates to a peptide for use in simultaneous quantification of metabolizing enzyme proteins in humans or other mammals with a mass spectrometer and a method of using the same. More specifically, the present invention relates to a peptide consisting of a partial amino acid sequence of a human metabolizing enzyme protein or the like for use in simultaneous quantification of absolute amounts of two or more of human or mammal metabolizing enzyme proteins in a biological sample with a mass spectrometer at high sensitivity and a method of using the same.

### Background Art

Metabolizing enzymes occurring in mammals including humans include drug metabolizing enzymes, steroid metabolizing enzymes, amino acid metabolizing enzymes, and nucleic acid metabolizing enzymes. Among these metabolizing enzymes, drug metabolizing enzymes are important enzymes that are involved in effectiveness and occurrence of toxicities of a drug by modifying the drug by metabolism. Examples of such metabolizing enzymes include CYP (P-450), glucuronic acid conjugating enzymes, sulfuric acid conjugating enzymes, and glucuronide conjugating enzymes. These enzyme molecules are subject to induction and inhibition of expressions thereof due to various factors, and such variations in the expressions result in varied effectiveness and toxicities of a drug. As expression profiles of drug-metabolizing enzymes thus determine how a drug is metabolized, these expression profiles are very important information in development of a drug.

An expression profile of a drug metabolizing enzyme can be analyzed at an mRNA level by utilizing PCR, DNA chips, and the like. However, mRNA expression is not necessarily consistent with expression of a protein, which is actual manifestation of an activity of the enzyme. Furthermore, one drug is often metabolized by more than one metabolizing enzyme. In such a case, the magnitude of contribution of each metabolizing enzyme cannot be analyzed by mRNA expression. Achievement of comparison between different metabolizing enzymes by analyzing metabolizing enzymes with a protein expression level and further obtaining an absolute expression level has been awaited.

One example of methods for detecting a metabolizing enzyme protein is a method using an antibody. Although a protein can be detected and quantified by Western blot in this method, it is not only very difficult to prepare a specific antibody, but also it requires considerable time and labor to construct profiles of two or more molecules.

Meanwhile, mass spectrometry techniques nave been dramatically advanced, investigated, and utilized to detect and measure various biomaterials in recent years. Mass spectrometers having various functions have been developed, including a mass spectrometer in use for electrosprayionization (ESI), a mass spectrometer in use for liquid chromatography-mass spectrometry (LC-MS), and an MS/MS or tandem mass (tandem MS) spectrometer, which consists of two connected mass spectrometers. Mass spectrometers having these functions in combination are used in detection, measurement, or quantification of biomaterials (Patent Documents 1 to 3).

US 2007/0092926 discloses analysis of protein isoforms using unique tryptic peptides by mass spectrometry and immunochemistry. WO 2007/140291 discloses expression quantification using mass spectrometry. WO 2007/127767 discloses quantification of enzyme activity by mass spectrometry. Lane Catherine S. et al disclose "Comparative cytochrome P450 proteomics in the livers of immunodeficient mice using 180 stable isotope labelling", Molecular & Cellular Proteomics, vol. 6, June 2007, pages 953-962.

The inventors of the present invention have invented a method for simultaneous quantification of absolute expression levels of 20 or more membrane proteins with a mass spectrometer (Patent Document 4). This invention is a method for obtaining an absolute expression level of a target protein by selecting a highly sensitive peptide from peptides obtained by digesting the target protein with trypsin and using a stable-isotope-labeled peptide that has the same amino acid sequence as the amino acid sequence of the selected peptide to quantify an absolute expression level of a trypsin-digested peptide to be quantified in a biological sample. It is therefore critical to select a highly sensitive peptide in a target protein to achieve quantification at high sensitivity, precision, and reliability.

However, the above-mentioned invention described in Patent Document 4 is an invention related to a method for quantifying an absolute expression level of a cell membrane protein and an amino acid sequence of a peptide of the cell membrane protein, and information on a peptide that can be used for quantification of a metabolizing enzyme, an intracellular protein, was unknown. As an enormous amount of proteins exist in the cell as compared with the cell membrane, quantification of a metabolizing enzyme, an intracellular protein, involves quantification of trace amounts of proteins in a more complicated protein sample, and it was anticipated to be more difficult to select a peptide to be quantified for quantification of a metabolizing enzyme in an intracellular protein sample as compared with selection of a peptide to be quantified in a cell membrane protein.

### Citation List

### Patent Document

Patent Document 1: Japanese Patent Laid-Open No. 2004-28993
Patent Document 2: Japanese Patent Laid-Open No. 2004-77276
Patent Document 3: National Publication of International Patent Application No. 2004-533610
Patent Document 4: International Publication No. WO 07/055116

### Disclosure of the Invention

### Problems to be Solved by the Invention

An object of the present invention is to provide a peptide consisting of an amino acid sequence that can simultaneously quantify absolute protein amounts of metabolizing enzymes in a biological sample at high sensitivity and a method of using the same.

### Means for Solving the Problems

The inventors of the present invention conducted various researches to achieve the foregoing object and found that the presence of a specific amino acid suppresses ionization of peptides and lowers sensitivity, and that the presence of a different specific amino acid increases efficiency of collision-induced dissociation and facilitates ionization of peptides, resulting in high sensitivity. The inventors of the present invention selected a peptide that can be detected with a mass spectrometer at high sensitivity and can simultaneously quantify metabolizing enzymes, intracellular proteins, at high sensitivity, identified an amino acid sequence of the peptide by scoring peptides according to criteria and narrowing down a candidate from two or more peptides by total scores, and thus accomplished the present invention.

Specifically, the present invention relates to (1) a peptide for use in simultaneous protein quantification of metabolizing enzymes with a mass spectrometer, consisting of the partial amino acid sequence of a human metabolizing enzyme protein set forth in any one of SEQ ID NOS: 1 to 412, (2) a peptide for use in simultaneous protein quantification of metabolizing enzymes with a mass spectrometer, consisting of the partial amino acid sequence of a mouse metabolizing enzyme protein set forth in any one of SEQ ID NOS: 413 to 695, (3) a peptide for use in simultaneous protein quantification of metabolizing enzymes with a mass spectrometer, consisting of an amino acid sequence including deletion, substitution, or addition of one or two amino acids in the amino acid sequence set forth in any one of SEQ ID NOS: 1 to 695, and (4) a stable-isotope-labeled peptide, wherein one or more of peptide-constituting amino acids contain any one or more of ¹⁵N, ¹³C, ¹⁸O, and ²H in the peptide according to any one of the above (1) to (3).

Furthermore, the present invention relates to (5) a kit for simultaneous protein quantification of metabolizing enzymes, comprising the peptide according to any one of the above (1) to (3) and the stable-isotope-labeled peptide according to the above (4), and (6) a method of using the peptide according to any one of the above (1) to (3) and the stable-isotope-labeled peptide according to the above (4) as a probe for simultaneous protein quantification of metabolizing enzymes.

Furthermore, the present invention relates to (7) a method for simultaneous protein quantification of metabolizing enzymes with a liquid chromatograph-tandem mass spectrometer (LC-MS/MS) using a stable-isotope-labeled peptide, comprising the steps of: (a) performing a mass spectrometry by liquid chromatograph-tandem mass spectrometer (LC-MS/MS) using the peptide according to any one of the above (1) to (3) and the stable-isotope-labeled peptide according to the above (4) at predetermined concentration levels of these peptides to create a calibration curve; (b) performing a mass spectrometry by LC-MS/MS by adding the stable-isotope-labeled peptide according to the above (4) to peptide fragments obtained by fragmenting metabolizing enzyme proteins to be quantified in a sample with trypsin to calculate a mass spectrum area ratio of a metabolizing enzyme protein peptide to be quantified and the stable-isotope-labeled peptide; and (c) obtaining a quantitative value from the area ratio using the calibration curve.

### Advantageous Effects of Invention

Quantification of metabolizing enzyme proteins, which was difficult by conventional methods, can be achieved conveniently and rapidly at high precision by a method for quantification of metabolizing enzyme proteins by mass spectrometry using a peptide consisting of the amino acid sequence of the present invention. Furthermore, as metabolizing enzyme proteins can be quantified by the method for quantification of metabolizing enzyme proteins of the present invention without using an antibody, the step of preparing an antibody, which conventionally required the longest time, can be omitted, and further metabolizing enzymes for which an antibody cannot be prepared can be quantified. Therefore, a method of wide application for highly precise quantification of metabolizing enzyme proteins, intracellular proteins, can be provided. Accordingly, a method for quantification of metabolizing enzyme proteins utilizing the present invention can be expected to contribute greatly to understanding of drug interactions, individual variations, and toxicities. Particularly in development of a novel drug, information on metabolizing enzymes of which expressions are changed by a novel drug candidate substance and the extents to which these expressions are changed has a very important significance to predict interactions and toxicities of the substance. The method of the present invention can therefore be expected to contribute greatly to promotion of development of a novel drug.

### Brief Description of Drawings

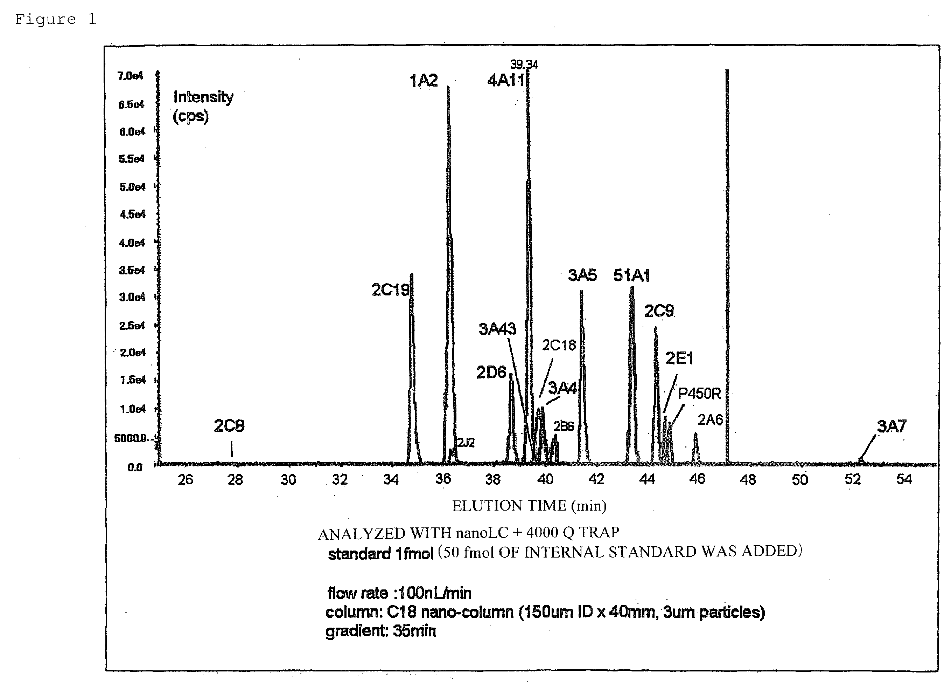

[Figure 1] A diagram showing results of measurement by nanoLC-MS/MS by adding 50 fmol each of ¹³C₆, ¹⁵N-labeled peptides to 1 fmol of non-labeled peptides to be quantified. 1 fmol of selected peptides (CYP) can be detected by nanoLC.

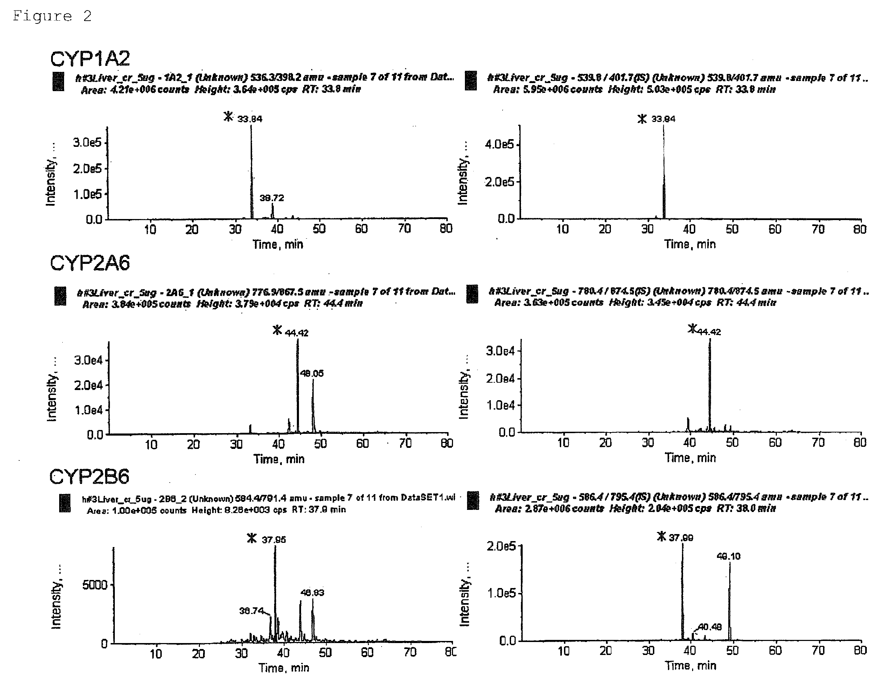

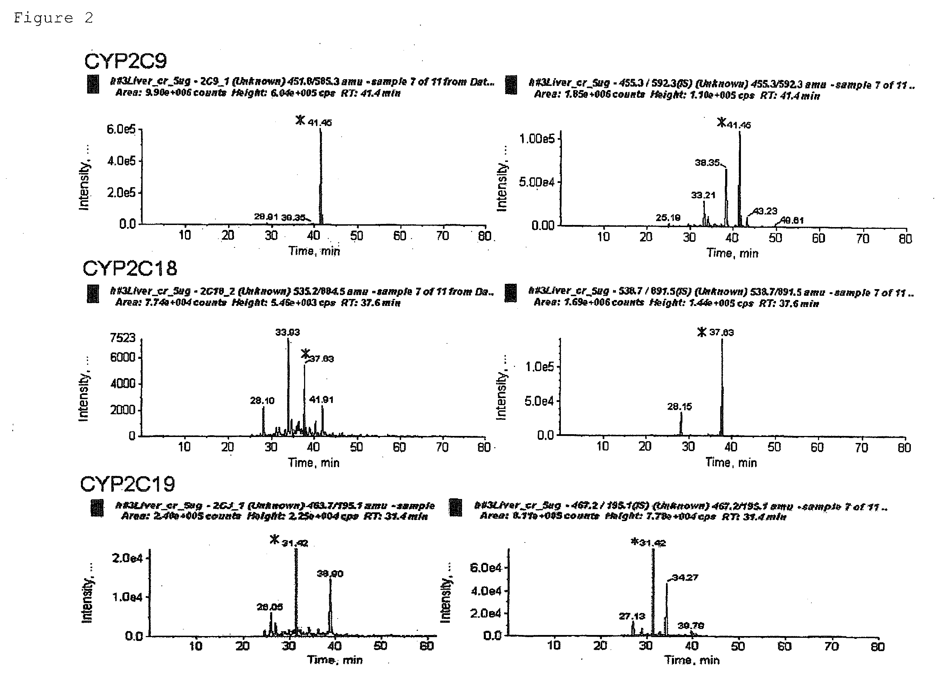

[Figure 2] An example of analysis of absolute expression levels of CYP from the human liver: a peak of a peptide to be quantified (left panel: in red, marked with *) and a peak of a labeled peptide as an internal standard (right panel: in red, marked with *) in the analysis of the human liver. Both the peaks were detected at the same elution time. In the graphs, the horizontal axis represents elution time (min), and the vertical axis represents intensity (cps).

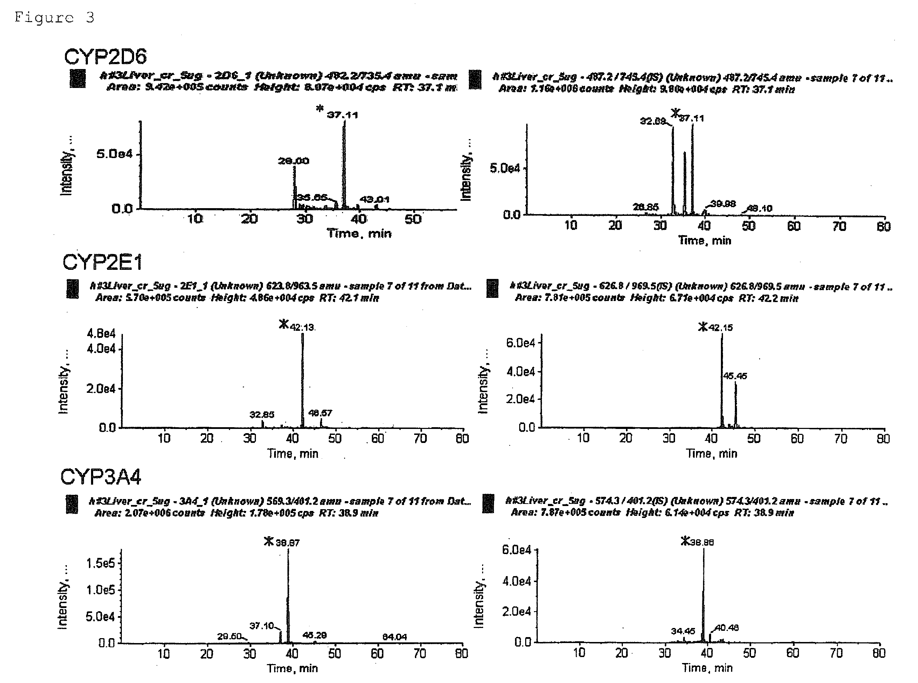

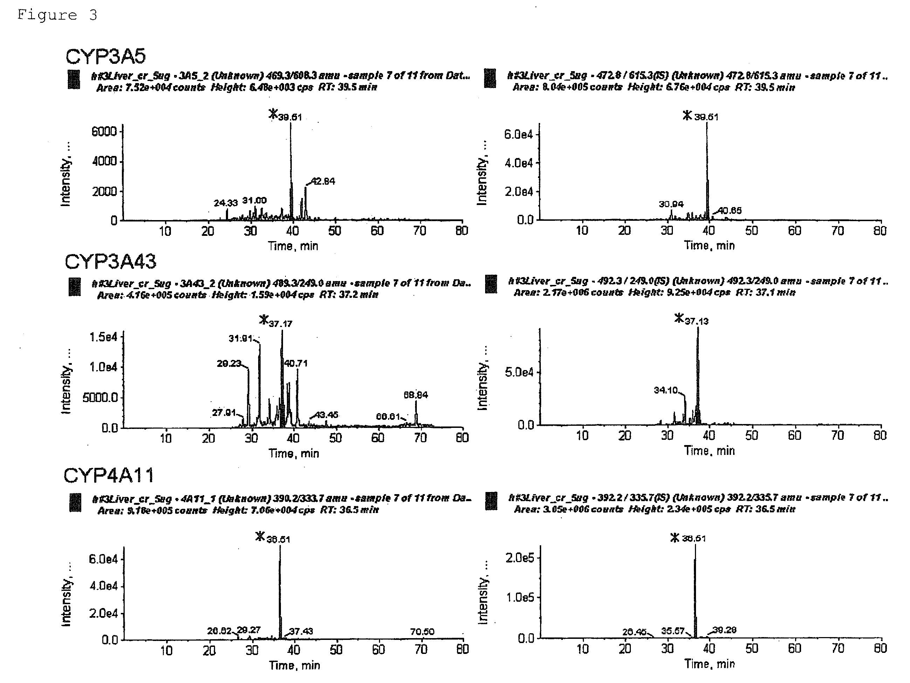

[Figure 3] An example of analysis of absolute expression levels of CYP from the human liver: a peak of a peptide to be quantified (left panel: in red, marked with *) and a peak of a labeled peptide as an internal standard (right panel: in red, marked with *) in the analysis of the human liver. Both the peaks were detected at the same elution time. In the graphs, the horizontal axis represents elution time (min), and the vertical axis represents intensity (cps).

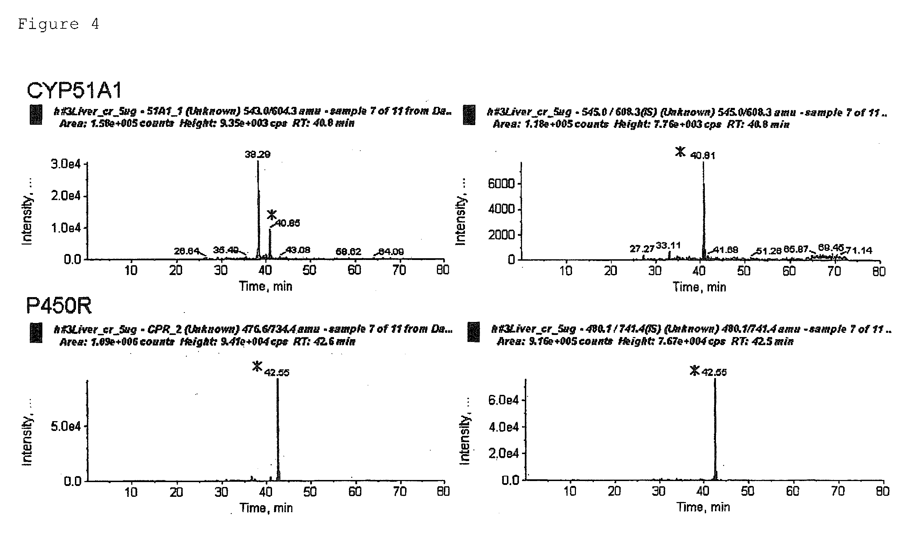

[Figure 4] An example of analysis of absolute expression levels of CYP from the human liver: a peak of a peptide to be quantified (left panel: in red, marked with *) and a peak of a labeled peptide as an internal standard (right panel: in red, marked with *) in the analysis of the human liver. Both the peaks were detected at the same elution time. In the graphs, the horizontal axis represents elution time (min), and the vertical axis represents intensity (cps).

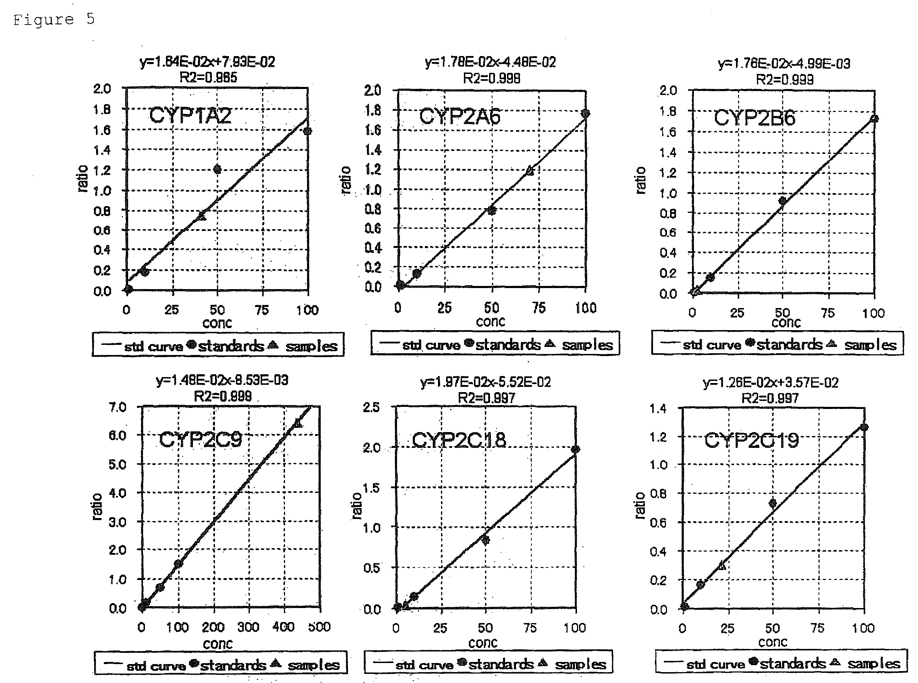

[Figure 5] An example of analysis of absolute expression levels of CYP from the human liver: The calibration curve of each CYP molecule is shown. The horizontal axis represents the amount of a peptide to be quantified (fmol), and the vertical axis represents the peak area ratio of the peptide to be quantified and the labeled peptide. In the graphs, the blue circle represents a reference standard of a synthesized peptide, and the green triangle represents a peptide to be quantified in a sample.

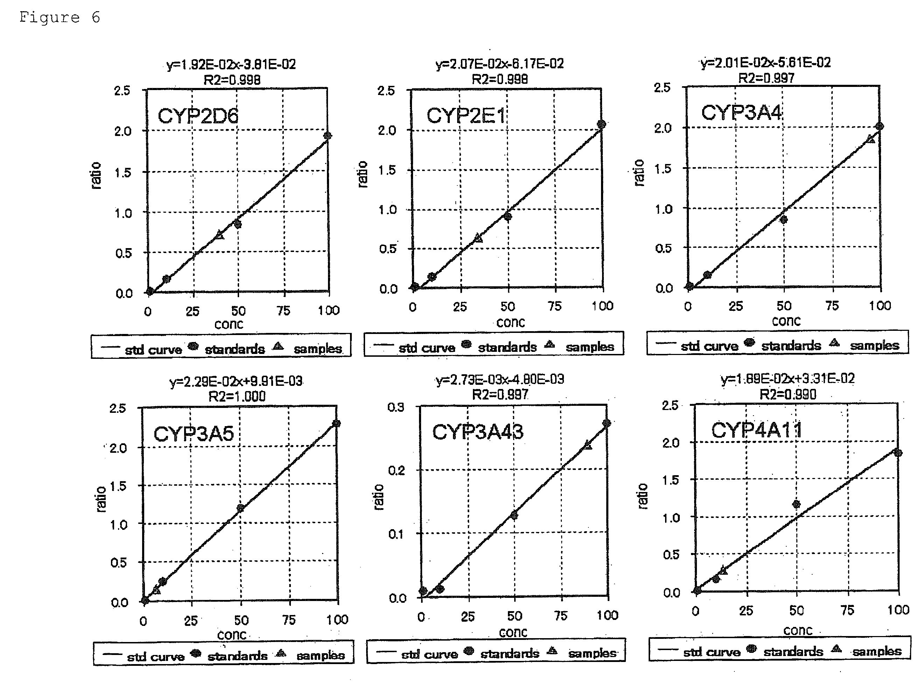

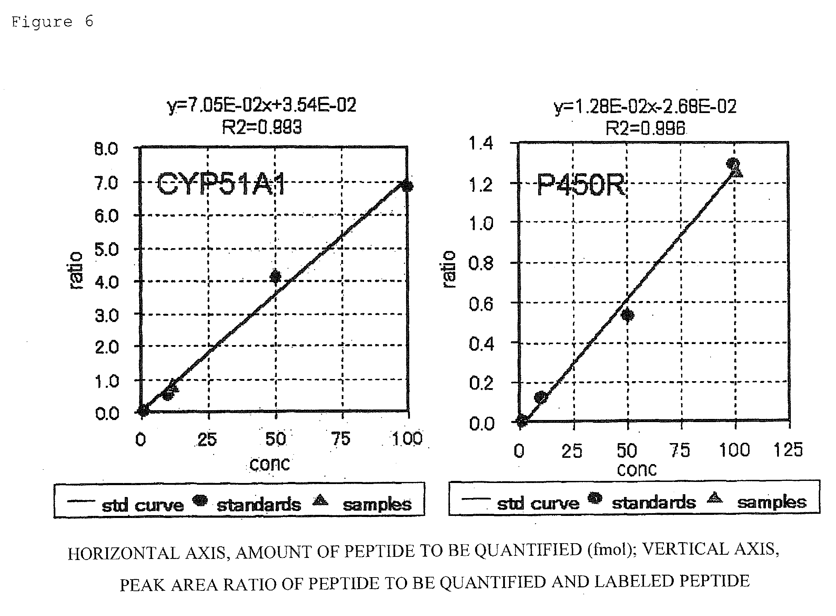

[Figure 6] An example of analysis of absolute expression levels of CYP from the human liver: The calibration curve of each CYP molecule is shown. The horizontal axis represents the amount of a peptide to be quantified (fmol), and the vertical axis represents the peak area ratio of the peptide to be quantified and the labeled peptide. In the graphs, the blue circle represents a reference standard of a synthesized peptide, and the green triangle represents a peptide to be quantified in a sample.

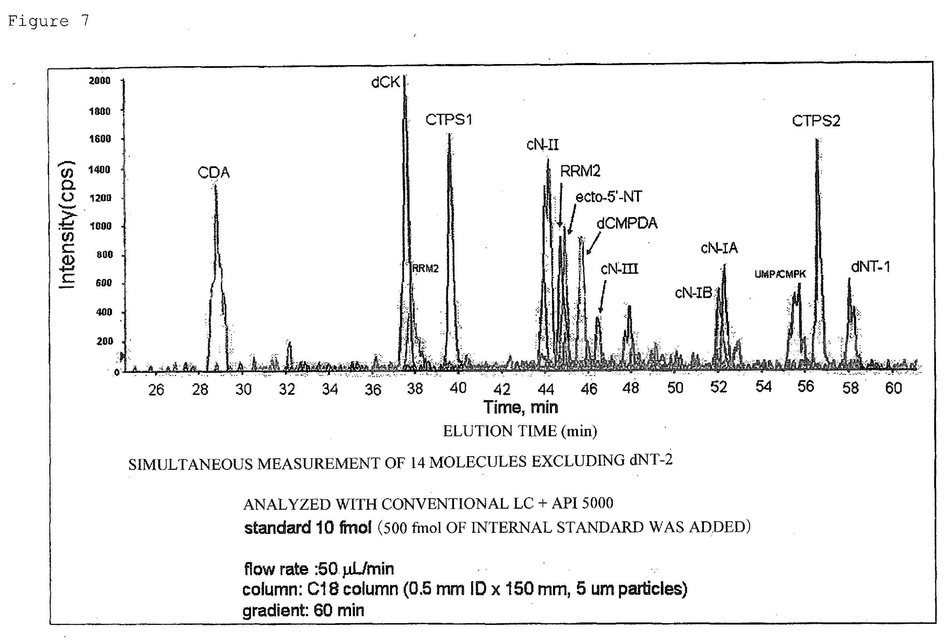

[Figure 7] A diagram showing results of measurement by conventional LC-MS/MS by adding 500 fmol each of ¹³C₆, ¹⁵N-labeled peptides to 10 fmol of non-labeled peptides to be quantified. Conventional HPLC can detect 10 fmol of peptides (Ara-C metabolizing enzymes).

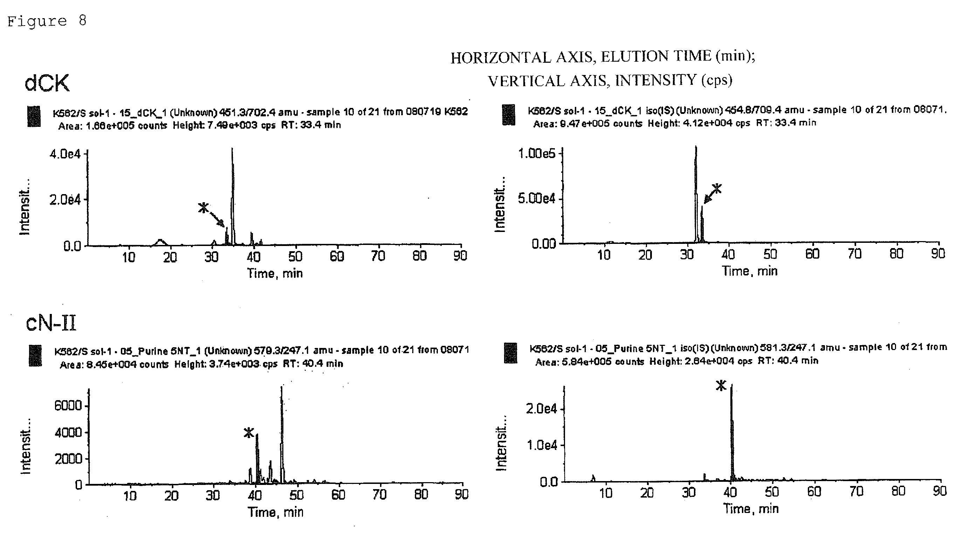

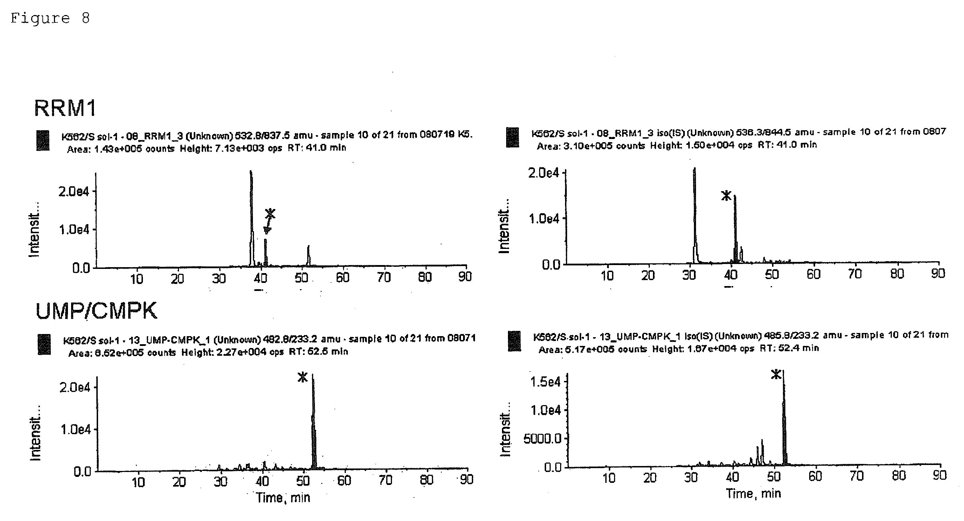

[Figure 8] An example of analysis of absolute expression levels of Ara-C-related metabolizing enzymes from human leukemic cells: a peak of a peptide to be quantified (left panel: in red, marked with *) and a peak of a labeled peptide as an internal standard (right panel: in red, marked with *) in the analysis of human leukemic cells. Both the peaks were detected at the same elution time. In the graphs, the horizontal axis represents elution time (min), and the vertical axis represents intensity (cps).

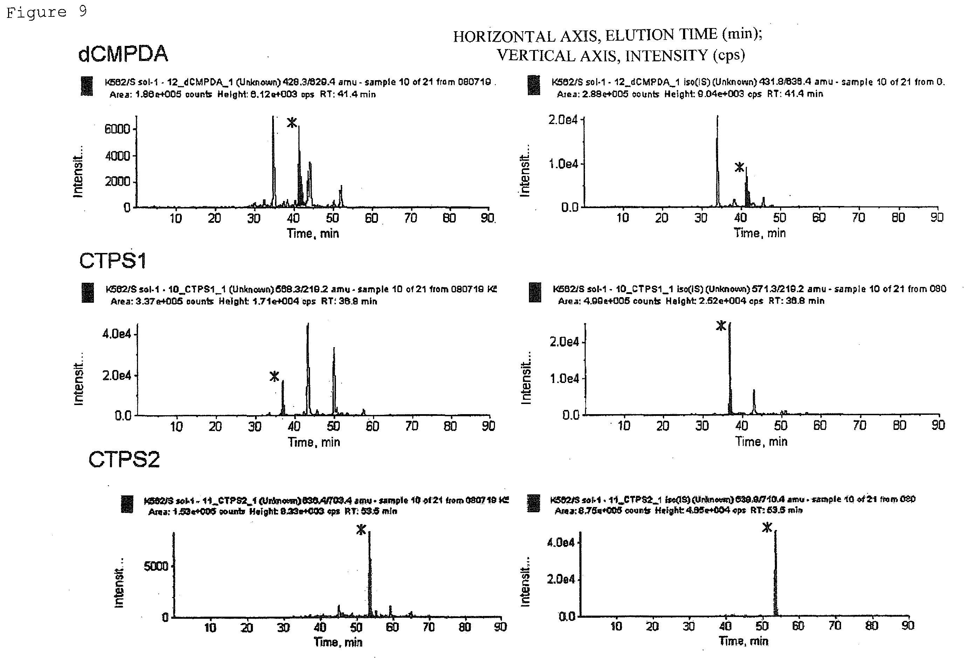

[Figure 9] An example of analysis of absolute expression levels of Ara-C-related metabolizing enzymes from human leukemic cells: a peak of a peptide to be quantified (left panel: in red, marked with *) and a peak of a labeled peptide as an internal standard (right panel: in red, marked with *) in the analysis of human leukemic cells. Both the peaks were detected at the same elution time. In the graphs, the horizontal axis represents elution time (min), and the vertical axis represents intensity (cps).

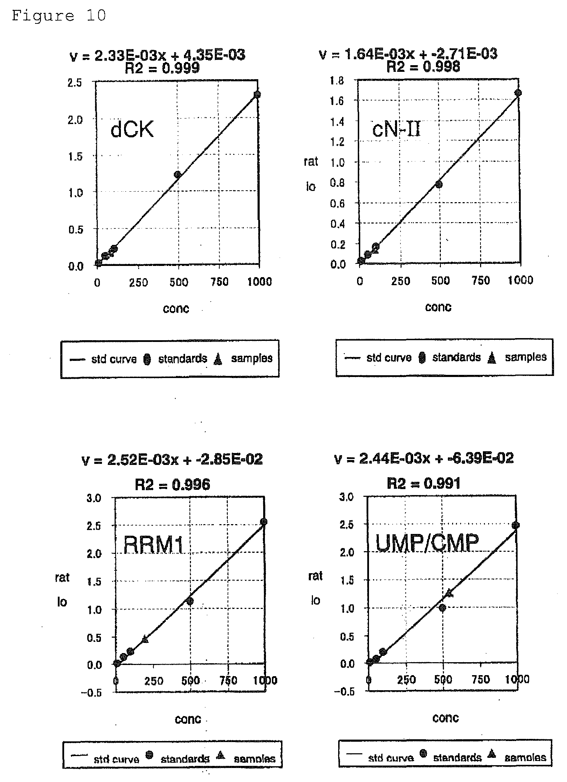

[Figure 10] An example of analysis of expression levels of Ara-C-related metabolizing enzymes from human leukemic cells. The calibration curve of each Ara-C metabolizing enzyme molecule is shown. The horizontal axis represents the amount of a peptide to be quantified (fmol), and the vertical axis represents the peak area ratio of the peptide to be quantified and the labeled peptide. In the graphs, the blue circle represents a reference standard of a synthesized peptide, and the green triangle represents a peptide to be quantified in a sample.

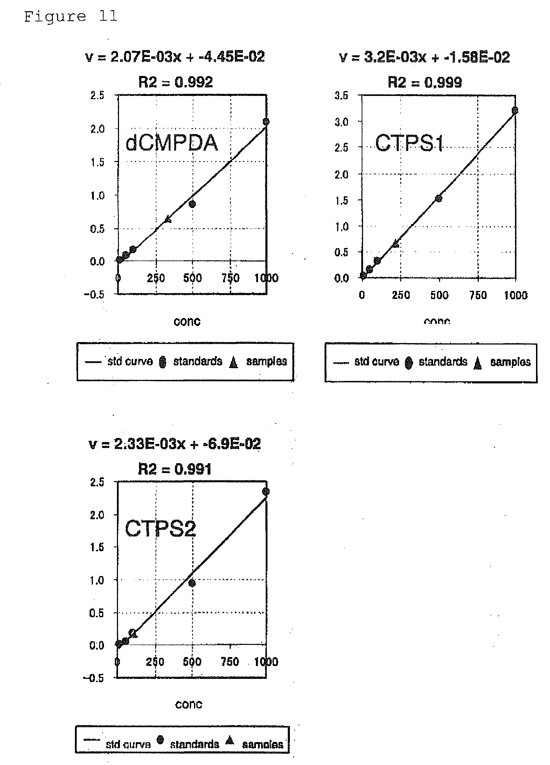

[Figure 11] An example of analysis of expression levels of Ara-C-related metabolizing enzymes from human leukemic cells. The calibration curve of each Ara-C metabolizing enzyme molecule is shown. The horizontal axis represents the amount of a peptide to be quantified (fmol), and the vertical axis represents the peak area ratio of the peptide to be quantified and the labeled peptide. In the graph, the blue circle represents a reference standard of a synthesized peptide, and the green triangle represents a peptide to be quantified in a sample.

### Mode of Carrying Out the Invention

### (Peptides consisting of partial amino acid sequence of human metabolizing enzyme protein)

The peptide of the present invention is a peptide for use in simultaneous protein quantification of metabolizing enzymes with a mass spectrometer, consisting of a partial amino acid sequence of a human metabolizing enzyme protein. Specific examples of the human metabolizing enzyme protein can include CYP1A1 (SwissProt accession number: P04798), CYP1A2 (SwissProt accession number: P05177), CYP1B1 (SwissProt accession number: Q16678), CYP2A6 (SwissProt accession number: P11509), CYP2A7 (SwissProt accession number: P20853), CYP2A13 (SwissProt accession number: Q16696), CYP2B6 (SwissProt accession number: P20813), CYP2C8 (SwissProt accession number: P10632), CYP2C9 (SwissProt accession number: P11712), CYP2C18 (SwissProt accession number: P33260), CYP2C19 (SwissProt accession number: P33261), CYP2D6 (SwissProt accession number: P10635), CYP2E1 (SwissProt accession number: P05181), CYP2F1 (SwissProt accession number: P24903), CYP2J2 (SwissProt accession number: P51589), CYP2R1 (SwissProt accession number: Q6VVX0), CYP2S1 (SwissProt accession number: Q96SQ9), CYP2W1 (SwissProt accession number: Q8TAV3), CYP3A4 (SwissProt accession number: P08684), CYP3A5 (SwissProt accession number: P20815), CYP3A7 (SwissProt accession number: P24462), CYP3A43 (SwissProt accession number: Q9HB55), CYP4A11 (SwissProt accession number: Q02928), CYP4B1 (SwissProt accession number: P13584), CYP4F2 (SwissProt accession number: P78329), CYP4F3 (SwissProt accession number: Q08477), CYP4F8 (SwissProt accession number: P98187), CYP4F11 (SwissProt accession number: Q9HBI6), CYP4F12 (SwissProt accession number: Q9HCS2), CYP4F22 (SwissProt accession number: Q6NT55), CYP4V2 (SwissProt accession number: Q6ZWL3), CYP4X1 (SwissProt accession number: Q8N118), CYP4Z1 (SwissProt accession number: Q86W10), CYP7A1 (SwissProt accession number: P22680), CYP7B1 (SwissProt accession number: 075881), CYP8B1 (SwissProt accession number: Q9UNU6), CYP11B1 (SwissProt accession number: P15538), CYP11B2 (SwissProt accession number: P19099), CYP17A1 (SwissProt accession number: P05093), CYP19A1 (SwissProt accession number: P11511), CYP21A2 (SwissProt accession number: P08686), CYP24A1 (SwissProt accession number: Q07973), CYP26A1 (SwissProt accession number: 043174), CYP26B1 (SwissProt accession number: Q9NR63), CYP26C1 (SwissProt accession number: Q6VOLO), CYP27A1 (SwissProt accession number: Q02318), CYP27B1 (SwissProt accession number: 015528), CYP27C1 (SwissProt accession number: Q4GOS4), CYP39A1 (SwissProt accession number: Q9NYL5), CYP46A1 (SwissProt accession number: Q9Y6A2), CYP51A1 (SwissProt accession number: Q16850), UGT1A1 (SwissProt accession number: P22309), UGT1A3 (SwissProt accession number: P35503), UGT1A4 (SwissProt accession number: P22310), UGT1A5 (SwissProt accession number: P35504), UGT1A6 (SwissProt accession number: P19224), UGT1A7 (SwissProt accession number: Q9HAW7), UGT1A10 (SwissProt accession number: Q9HAW8), UGT2A1 (SwissProt accession number: Q9Y4X1), UGT2B4 (SwissProt accession number: P06133), UGT2B7 (SwissProt accession number: P16662), UGT2B10 (SwissProt accession number: P36537), UGT2B11 (SwissProt accession number: 075310), UGT2B17 (SwissProt accession number: 075795), UGT2B28 (SwissProt accession number: Q9BY64), GSTA1 (SwissProt accession number: P08263), GSTA2 (SwissProt accession number: P09210), GSTA3 (SwissProt accession number: Q16772), GSTA4 (SwissProt accession number: 015217), GSTA5 (SwissProt accession number: Q7RTV2), GSTK1 (SwissProt accession number: Q9Y2Q3), GSTM1 (SwissProt accession number: P09488), GSTM2 (SwissProt accession number: P28161), GSTM3 (SwissProt accession number: P21266), GSTM4 (SwissProt accession number: Q03013), GSTM5 (SwissProt accession number: P46439), GSTP1 (SwissProt accession number: P09211), GSTT1 (SwissProt accession number: P30711), GSTT2 (SwissProt accession number: P30712), MGST1 (SwissProt accession number: P10620), MGST2 (SwissProt accession number: Q99735), MGST3 (SwissProt accession number: 014880), SULT1A2 (SwissProt accession number: P50226), SULT1A3 (SwissProt accession number: P50224), SULT1B1 (SwissProt accession number: 043704), SULT1C2 (SwissProt accession number: P000338), SULT1C3 (SwissProt accession number: Q6IMI6), SULT1C4 (SwissProt accession number: 075897), SULT1E1 (SwissProt accession number: P49888), SULT2A1 (SwissProt accession number: Q06520), SULT2B1 (SwissProt accession number: 000204), SULT4A1 (SwissProt accession number: Q9BR01), SULT4S6 (SwissProt accession number: Q7LFX5), P450R (SwissProt accession number: P16435), MGMT (SwissProt accession number: P16455), dCK (SwissProt accession number: P27707), CDA (SwissProt accession number: P32320), cN-IA (SwissProt accession number: Q9BX13), cN-IB (SwissProt accession number: Q96P26), cN-II (SwissProt accession number: P49902), cN-III (SwissProt accession number: Q9H0P0), dNT-1 (SwissProt accession number: Q8TCD5), dNT-2 (SwissProt accession number: Q9NPB1), Ecto-5'-NT (SwissProt accession number: P21589), RRM1 (SwissProt accession number: P23921), RRM2 (SwissProt accession number: P31350), UMP/CMPK (SwissProt accession number: P30085), dCMPDA (SwissProt accession number: P32321), CTP synthase 1 (SwissProt accession number: P17812), and CTP synthase 2 (SwissProt accession number: Q9NRF8).

The peptide of the present invention is a peptide consisting of any of partial amino acid sequences of the above-mentioned human metabolizing enzyme proteins. Specific examples of the peptide include peptides each consisting of any of amino acid sequences of SEQ ID NOS: 1 to 5, which are partial sequences of CYP1A1; amino acid sequences of SEQ ID NOS: 6 to 8, which are partial sequences of CYP1A2; and amino acid sequences of SEQ ID NOS: 9 to 14, which are partial sequences of CYP1B1. CYP1A1, CYP1A2, and CYP1B1 can be quantified using any of the corresponding peptides.

Similarly, peptides each consisting of any of amino acid sequences of SEQ ID NOS: 15 to 17, which are partial sequences of CYP2A6; amino acid sequences of SEQ ID NOS: 18 and 19, which are partial sequences of CYP2A7; amino acid sequences of SEQ ID NOS: 20 and 21, which are partial sequences of CYP2A13; amino acid sequences of SEQ ID NOS: 22 to 25, which are partial sequences of CYP2B6; amino acid sequences of SEQ ID NOS: 26 to 29, which are partial sequences of CYP2C8; amino acid sequences of SEQ ID NOS: 30 and 31, which are partial sequences of CYP2C9; amino acid sequences of SEQ ID NOS: 32 to 34, which are partial sequences of CYP2C18; amino acid sequences of SEQ ID NOS: 35 to 37, which are partial sequences of CYP2C19; amino acid sequences of SEQ ID NOS: 38 and 39, which are partial sequences of CYP2D6; amino acid sequences of SEQ ID NOS: 40 to 46, which are partial sequences of CYP2E1; an amino acid sequence of SEQ ID NO: 47, which is a partial sequence of CYP2F1; amino acid sequences of SEQ ID NOS: 48 to 55, which are partial sequences of CYP2J2; amino acid sequences of SEQ ID NOS: 56 to 63, which are partial sequences of CYP2R1; amino acid sequences of SEQ ID NOS: 64 to 72, which are partial sequences of CYP2S1; and amino acid sequences of SEQ ID NOS: 73 to 79, which are partial sequences of CYP2W1, are also the peptides of the present invention. CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, and CYP2W1 can be quantified using any of the corresponding peptides.

Similarly, peptides each consisting of any of amino acid sequences of SEQ ID NOS: 80 and 81, which are partial sequences of CYP3A4; amino acid sequences of SEQ ID NOS: 82 to 84, which are partial sequences of CYP3A5; amino acid sequences of SEQ ID NOS: 85 to 88, which are partial sequences of CYP3A7; and amino acid sequences of SEQ ID NOS: 89 to 96, which are partial sequences of CYP3A43, are also the peptides of the present invention. CYP3A4, CYP3A5, CYP3A7, and CYP3A43 can be quantified using any the corresponding peptides.

Similarly, peptides each consisting of any of amino acid sequences of SEQ ID NOS: 97 to 101, which are partial sequences of CYP4A11; amino acid sequences of SEQ ID NOS: 102 to 111, which are partial sequences of CYP4B1; amino acid sequences of SEQ ID NO: 112 to 114, which are partial sequences of CYP4F2; an amino acid sequence of SEQ ID NO: 115, which is a partial sequence of CYP4F3; amino acid sequences of SEQ ID NOS: 116 to 119, which are partial sequences of CYP4F8; amino acid sequences of SEQ ID NOS: 120 to 122, which are partial sequences of CYP4F11; amino acid sequences of SEQ ID NOS: 123 to 127, which are partial sequences of CYP4F12; amino acid sequences of SEQ ID NOS: 128 to 136, which are partial sequences of CYP4F22; amino acid sequences of SEQ ID NOS: 137 to 144, which are partial sequences of CYP4V2; amino acid sequences of SEQ ID NOS: 145 to 154, which are partial sequences of CYP4X1; and amino acid sequences of SEQ ID NOS: 155 to 165, which are partial sequences of CYP4Z1, are also the peptides of the present invention. CYP4A11, CYP4B1, CYP4F2, CYP4F3, CYP4F8, CYP4F11, CYP4F12, CYP4F22, CYP4V2, CYP4X1, and CYP4Z1 can be quantified using any of the corresponding peptides.

Similarly, peptides each consisting of any of amino acid sequences of SEQ ID NOS: 166 to 171, which are partial sequences of CYP7A1; amino acid sequences of SEQ ID NOS: 172 to 178, which are partial sequences of CYP7B1; amino acid sequences of SEQ ID NOS: 179 to 185, which are partial sequences of CYP8B1; amino acid sequences of SEQ ID NOS: 186 to 191, which are partial sequences of CYP11B1; amino acid sequences of SEQ ID NOS: 192 to 195, which are partial sequences of CYP11B2; amino acid sequences of SEQ ID NOS: 196 to 200, which are partial sequences of CYP17A1; and amino acid sequences of SEQ ID NOS: 201 to 207, which are partial sequences of CYP19A1, are also the peptides of the present invention. CYP7A1, CYP7B1, CYP8B1, CYP11B1, CYP11B2, CYP17A1, and CYP19A1 can be quantified using any of the corresponding peptides.

Similarly, peptides each consisting of any of amino acid sequences of SEQ ID NOS: 208 to 210, which are partial sequences of CYP21A2; amino acid sequences of SEQ ID NOS: 211 to 216, which are partial sequences of CYP24A1; amino acid sequences of SEQ ID NOS: 217 to 226, which are partial sequences of CYP26A1; amino acid sequences of SEQ ID NOS: 227 to 238, which are partial sequences of CYP26B1; amino acid sequences of SEQ ID NOS: 239 to 249, which are partial sequences of CYP26C1; amino acid sequences of SEQ ID NOS: 250 to 256, which are partial sequences of CYP27A1; amino acid sequences of SEQ ID NOS: 257 to 265, which are partial sequences of CYP27B1; and amino acid sequences of SEQ ID NOS: 266 to 275, which are partial sequences of CYP27C1, are also the peptides of the present invention. CYP21A2, CYP24A1, CYP26A1, CYP26B1, CYP26C1, CYP27A1, CYP27B1, and CYP27C1 can be quantified using any of the corresponding peptides.

Similarly, peptides each consisting of any of an amino acid sequence of SEQ ID NO: 276, which is a partial sequence of CYP39A1; amino acid sequences of SEQ ID NOS: 277 to 284, which are partial sequences of CYP46A1; and amino acid sequences of SEQ ID NOS: 285 to 296, which are partial sequences of CYP51A1, are also the peptides of the present invention. CYP39A1, CYP46A1, and CYP51A1 can be quantified using any of the corresponding peptides.

Similarly, peptides each consisting of any of an amino acid sequence of SEQ ID NO: 297, which is a partial sequence of UGT1A1; amino acid sequences of SEQ ID NOS: 298 and 299, which are partial sequences of UGT1A3; amino acid sequences of SEQ ID NOS: 300 and 301, which are partial sequences of UGT1A4; an amino acid sequence of SEQ ID NO: 302, which is a partial sequence of UGT1A5; amino acid sequences of SEQ ID NOS: 303 to 305, which are partial sequences of UGT1A6; amino acid sequences of SEQ ID NOS: 306 and 307, which are partial sequences of UGT1A7; and an amino acid sequence of SEQ ID NO: 308, which is a partial sequence of UGT1A10, are also the peptides of the present invention. UGT1A1, UGT1A3, UGT1A4, UGT1A5, UGT1A6, UGT1A7, and UGT1A10 can be quantified using any of the corresponding peptides.

Similarly, peptides each consisting of any of amino acid sequences of SEQ ID NOS: 309 and 310, which are partial sequences of UGT2A1; an amino acid sequence of SEQ ID NO: 311, which is a partial sequence of UGT2B4; amino acid sequences of SEQ ID NOS: 312 and 313, which are partial sequences of UGT2B7; an amino acid sequence of SEQ ID NO: 314, which is a partial sequence of UGT2B10; an amino acid sequence of SEQ ID NO: 315, which is a partial sequence of UGT2B11; an amino acid sequence of SEQ ID NO: 316, which is a partial sequence of UGT2B17; and an amino acid sequence of SEQ ID NO: 317, which is a partial sequence of UGT2B28, are also the peptides of the present invention. UGT2A1, UGT2B4, UGT2B7, UGT2B10, UGT2B11, UGT2B17, and UGT2B28 can be quantified using any of the corresponding peptides.

Similarly, peptides each consisting of any of an amino acid sequence of SEQ ID NO: 318, which is a partial sequence of GSTA1; an amino acid sequence of SEQ ID NO: 319, which is a partial sequence of GSTA2; an amino acid sequence of SEQ ID NO: 320, which is a partial sequence of GSTA3; amino acid sequences of SEQ ID NOS: 321 to 323, which are partial sequences of GSTA4; and an amino acid sequence of SEQ ID NO: 324, which is a partial sequence of GSTA5, are also the peptides of the present invention. GSTA1, GSTA2, GSTA3, GSTA4, and GSTA5 can be quantified using any of the corresponding peptides.

Similarly, peptides each consisting of any of an amino acid sequence of SEQ ID NO: 325, which is a partial sequence of GSTK1; an amino acid sequence of SEQ ID NO: 326, which is a partial sequence of GSTM1; amino acid sequences of SEQ ID NOS: 327 and 328, which are partial sequences of GSTM2; an amino acid sequence of SEQ ID NO: 329, which is a partial sequence of GSTM3; an amino acid sequence of SEQ ID NO: 330, which is a partial sequence of GSTM4; an amino acid sequence of SEQ ID NO: 331, which is a partial sequence of GSTM5; an amino acid sequence of SEQ ID NO: 332, which is a partial sequence of GSTP1; amino acid sequences of SEQ ID NOS: 333 and 334, which are partial sequences of GSTT1; and an amino acid sequence of SEQ ID NO: 335, which is a partial sequence of GSTT2, are also the peptides of the present invention. GSTM1, GSTM2, GSTM3, GSTM4, GSTM5, GSTT1, and GSTT2 can be quantified using any of the corresponding peptides.

Similarly, peptides each consisting of any of an amino acid sequence of SEQ ID NO: 336, which is a partial sequence of MGST1; an amino acid sequence of SEQ ID NO: 337, which is a partial sequence of MGST2; and an amino acid sequence of SEQ ID NO: 338, which is a partial sequence of MGST3, are also the peptides of the present invention. MGST1, MGST2, and MGST3 can be quantified using any of the corresponding peptides.

Similarly, peptides each consisting of any of an amino acid sequence of SEQ ID NO: 339, which is a partial sequence of SULT1A2; an amino acid sequence of SEQ ID NO: 340, which is a partial sequence of SULT1A3; an amino acid sequence of SEQ ID NO: 341, which is a partial sequence of SULT1B1; an amino acid sequence of SEQ ID NO: 342, which is a partial sequence of SULT1C2; an amino acid sequence of SEQ ID NO: 343, which is a partial sequence of SULT1C3; an amino acid sequence of SEQ ID NO: 344, which is a partial sequence of SULT1C4; an amino acid sequence of SEQ ID NO: 345, which is a partial sequence of SULT1E1; an amino acid sequence of SEQ ID NO: 346, which is a partial sequence of SULT2A1; an amino acid sequence of SEQ ID NO: 347, which is a partial sequence of SULT2B1; an amino acid sequence of SEQ ID NO: 348, which is a partial sequence of SULT4A1; and an amino acid sequence of SEQ ID NO: 349, which is a partial sequence of SULT4S6, are also the peptides of the present invention. SULT1A2, SULT1A3, SULT1B1, SULT1C2, SULT1C3, SULT1C4, SULT1E1, SULT2A1, SULT2B1, SULT4A1, and SULT4S6 can be quantified using any of the corresponding peptides.

Similarly, peptides each consisting of any of amino acid sequences of SEQ ID NOS: 350 to 359, which are partial sequences of P450R; amino acid sequences of SEQ ID NOS: 360 and 361, which are partial sequences of MGMT; amino acid sequences of SEQ ID NOS: 362 and 363, which are partial sequences of dCK; and an amino acid sequence of SEQ ID NO: 364, which is a partial sequence of CDA, are also the peptides of the present invention. P450R, MGMT, dCK, and CDA can be quantified using any of the corresponding peptides.

Similarly, peptides each consisting of amino acid sequences of SEQ ID NOS: 365 and 366, which are partial sequences of cN-IA; amino acid sequences of SEQ ID NOS: 367 to 374, which are partial sequences of cN-IB; an amino acid sequence of SEQ ID NO: 375, which is a partial sequence of cN-II; and amino acid sequences of SEQ ID NOS: 376 to 382, which are partial sequences of cN-III, are also the peptides of the present invention. cN-IA, cN-IB, cN-II, and cN-III can be quantified using any of the corresponding peptides.

Similarly, peptides each consisting of any of an amino acid sequence of SEQ ID NO: 383, which is a partial sequence of dNT-1; amino acid sequences of SEQ ID NOS: 384 and 385, which are partial sequences of dNT-2; amino acid sequences of SEQ ID NOS: 386 to 388, which are partial sequences of Ecto-5'-NT; amino acid sequences of SEQ ID NOS: 389 to 392, which are partial sequences of RRM1; amino acid sequences of SEQ ID NOS: 393 and 394, which are partial sequences of RRM2; an amino acid sequence of SEQ ID NO: 395, which is a partial sequence of UMP/CMPK; and an amino acid sequence of SEQ ID NO: 396, which is a partial sequence of dCMPDA, are also the peptides of the present invention. dNT-1, dNT-2, Ecto-5'-NT, RRM1, RRM2, UMP/CMPK, and dCMPDA can be quantified using any of the corresponding peptides.

Similarly, peptides each consisting of any of amino acid sequences of SEQ ID NOS: 397 to 405, which are partial sequences of CTP Synthase 1; and amino acid sequences of SEQ ID NOS: 406 to 412, which are partial sequences of CTP Synthase 2, are also the peptides of the present invention. CTP Synthase 1 and CTP Synthase 2 can be quantified using any of the respective peptides.

### (Peptides consisting of partial amino acid sequences of mouse metabolizing enzyme proteins)

Furthermore, the peptide of the present invention is a peptide consisting of any of partial amino acid sequences of mouse metabolizing enzyme proteins set forth in SEQ ID NOS: 413 to 695. Specific examples of the mouse metabolizing enzyme protein include Cyp11a1 (SwissProt accession number: Q9QZ82), Cyp17a1 (SwissProt accession number: P27786), Cyp19a1 (SwissProt accession number: P28649), Cyp1a1 (SwissProt accession number: P00184), Cyp1a2 (SwissProt accession number: P00186), Cyp21 (SwissProt accession number: P03940), Cyp24a1 (SwissProt accession number: Q64441), Cyp26a1 (SwissProt accession number: 055127), Cyp27a1 (SwissProt accession number: Q9DBG1), Cyp27b1 (SwissProt accession number: 035084), Cyp2a4 (SwissProt accession number: P15392), Cyp2a5 (SwissProt accession number: P20852), Cyp2b19 (SwissProt accession number: 055071), Cyp2c29 (SwissProt accession number: Q64458), Cyp2c39 (SwissProt accession number: P56656), Cyp2c70 (SwissProt accession number: Q91W64), Cyp2d10 (SwissProt accession number: P24456), Cyp2d26 (SwissProt accession number: Q8CIM7), Cyp2d9 (SwissProt accession number: P11714), Cyp2e1 (SwissProt accession number: Q05421), Cyp2f2 (SwissProt accession number: P33267), Cyp2j5 (SwissProt accession number: 054749), Cyp2r1 (SwissProt accession number: Q6VVW9), Cyp2s1 (SwissProt accession number: Q9DBX6), Cyp39a1 (SwissProt accession number: Q9JKJ9), Cyp3a11 (SwissProt accession number: Q64459), Cyp3a13 (SwissProt accession number: Q64464), Cyp3a16 (SwissProt accession number: Q64481), Cyp3a25 (SwissProt accession number: 009158), Cyp46a1 (SwissProt accession number: Q9WVK8), Cyp4a14 (SwissProt accession number: 035728), Cyp4b1 (SwissProt accession number: Q64462), Cyp4f14 (SwissProt accession number: Q9EP75), Cyp4v3 (SwissProt accession number: Q9DBW0), Cyp5a (SwissProt accession number: P36423), Cyp8b1 (SwissProt accession number: 088962), GST01 (SwissProt accession number: 009131), ST2B1 (SwissProt accession number: 035400), ST3A1 (SwissProt accession number: 035403), CHST3 (SwissProt accession number: 088199), SNAT (SwissProt accession number: 088816), UD2B5 (SwissProt accession number: P17717), GSTP1 (SwissProt accession number: P19157), GSTM4 (SwissProt accession number: P19639), GSTA4 (SwissProt accession number: P24472), GSTA3 (SwissProt accession number: P30115), GSTM5 (SwissProt accession number: P48774), ST1E1 (SwissProt accession number: P49891), ARY1 (SwissProt accession number: P50294), ARY2 (SwissProt accession number: P50295), ARY3 (SwissProt accession number: P50296), ST1A1 (SwissProt accession number: P52840), UD12 (SwissProt accession number: P70691), CGT (SwissProt accession number: Q64676), UD17C (SwissProt accession number: Q6ZQM8), ST1C1 (SwissProt accession number: Q80VR3), CHST2 (SwissProt accession number: Q80WV3), MGST3 (SwissProt accession number: Q9CPU4), ST1C2 (SwissProt accession number: Q9D939), GSTK1 (SwissProt accession number: Q9DCM2), CHST7 (SwissProt accession number: Q9EP78), CHST1 (SwissProt accession number: Q9EQC0), CHST5 (SwissProt accession number: Q9QUP4), NAT6 (SwissProt accession number: Q9R123), CHST4 (SwissProt accession number: Q9R1I1), and MAAI (SwissProt accession number: Q9WVLO).

The peptide of the present invention is a peptide consisting of any of partial amino acid sequences of the above-listed proteins. Specific examples of the peptide include peptides each consisting of any of amino acid sequences of SEQ ID NOS: 413 to 422, which are partial sequences of Cyp11a1; amino acid sequences of SEQ ID NOS: 423 to 429, which are partial sequences of Cyp17a1; amino acid sequences of SEQ ID NOS: 430 to 436, which are partial sequences of Cyp19a1; amino acid sequences of SEQ ID NOS: 437 and 438, which are partial sequences of Cyp1a1; and amino acid sequences of SEQ ID NOS: 439 to 443, which are partial sequences of Cypla2. Cyp11a1, Cyp17a1, Cyp19a1, Cyp1a1, and Cyp1a2 can be quantified using any of the corresponding peptides.

Similarly, peptides each consisting of any of amino acid sequences of SEQ ID NOS: 444 to 448, which are partial sequences of Cyp21; amino acid sequences of SEQ ID NOS: 449 to 455, which are partial sequences of Cyp24a1; amino acid sequences of SEQ ID NOS: 456 to 464, which are partial sequences of Cyp26a1; amino acid sequences of SEQ ID NOS: 465 to 470, which are partial sequences of Cyp27a1; and amino acid sequences of SEQ ID NOS: 471 to 478, which are partial sequences of Cyp27b1, are also the peptides of the present invention. Cyp21, Cyp24a1, Cyp26a1, Cyp27a1, and Cyp27b1 can be quantified using any of the corresponding peptides.

Similarly, peptides each consisting of any of an amino acid sequence of SEQ ID NO: 479, which is a partial sequence of Cyp2a4; an amino acid sequence of SEQ ID NO: 480, which is a partial sequence of Cyp2a5; amino acid sequences of SEQ ID NOS: 481 to 483, which are partial sequences of Cyp2b19; an amino acid sequence of SEQ ID NO: 484, which is a partial sequence of Cyp2c29; amino acid sequences of SEQ ID NOS: 485 to 488, which are partial sequences of Cyp2c39; amino acid sequences of SEQ ID NOS: 489 to 492, which are partial sequences of Cyp2c70; an amino acid sequence of SEQ ID NO: 493, which is a partial sequence of Cyp2d10; amino acid sequences of SEQ ID NOS: 494 to 497, which are partial sequences of Cyp2d26; amino acid sequences of SEQ ID NOS: 498 and 499, which are partial sequences of Cyp2d9; an amino acid sequence of SEQ ID NO: 500, which is a partial sequence of Cyp2e1; amino acid sequences of SEQ ID NOS: 501 to 505, which are partial sequences of Cyp2f2; amino acid sequences of SEQ ID NOS: 506 to 511, which are partial sequences of Cyp2j5; amino acid sequences of SEQ ID NO: 512 and 513, which are partial sequences of Cyp2r1; and amino acid sequences of SEQ ID NOS: 514 to 526, which are partial sequences of Cyp2s1, are also the peptides of the present invention. Cyp2a4, Cyp2a5, Cyp2b19, Cyp2c29, Cyp2c39, Cyp2c70, Cyp2d10, Cyp2d26, Cyp2d9, Cyp2e1, Cyp2f2, Cyp2j5, Cyp2r1, and Cyp2s1 can be quantified using any of the corresponding peptides.

Similarly, peptides each consisting of any of an amino acid sequence of SEQ ID NO: 527, which is a partial sequence of Cyp39a1; an amino acid sequence of SEQ ID NO: 528, which is a partial sequence of Cyp3a11; amino acid sequences of SEQ ID NOS: 529 to 534, which are partial sequences of Cyp3a13; amino acid sequences of SEQ ID NOS: 535 and 536, which are partial sequences of Cyp3a16; and amino acid sequences of SEQ ID NOS: 537 and 538, which are partial sequences of Cyp3a25, are also the peptides of the present invention. Cyp39a1, Cyp3a11, Cyp3a13, Cyp3a16, and Cyp3a25 can be quantified using any of the corresponding peptides.

Similarly, peptides each consisting of any of amino acid sequences of SEQ ID NOS: 539 to 545, which are partial sequences of Cyp46a1, are also the peptides of the present invention. Cyp46a1 can be quantified using any of the corresponding peptides.

Similarly, peptides each consisting of any of amino acid sequences of SEQ ID NOS: 546 to 550, which are partial sequences of Cyp4a14; amino acid sequences of SEQ ID NOS: 551 to 554, which are partial sequences of Cyp4b1; amino acid sequences of SEQ ID NOS: 555 to 558, which are partial sequences of Cyp4f14; amino acid sequences of SEQ ID NOS: 559 to 566, which are partial sequences of Cyp4v3; amino acid sequences of SEQ ID NOS: 567 to 570, which are partial sequences of Cyp5a; and amino acid sequences of SEQ ID NOS: 571 to 576, which are partial sequences of Cyp8b1, are also the peptides of the present invention. Cyp4a14, Cyp4b1, Cyp4f14, Cyp4v3, Cyp5a, and Cyp8b1 can be quantified using any of the corresponding peptides.

Similarly, peptides each consisting of any of amino acid sequences of SEQ ID NOS: 577 and 578, which are partial sequences of GST01; an amino acid sequence of SEQ ID NO: 579, which is a partial sequence of ST2B1; amino acid sequences of SEQ ID NOS: 580 to 585, which are partial sequences of ST3A1; amino acid sequences of SEQ ID NOS: 586 to 595, which are partial sequences of CHST3; an amino acid sequence of SEQ ID NO: 596, which is a partial sequence of SNAT; and an amino acid sequence of SEQ ID NO: 597, which is a partial sequence of UD2B5, are also the peptides of the present invention. GST01, ST2B1, ST3A1, CHST3, SNAT, and UD2B5 can be quantified using any of the corresponding peptides.

Similarly, peptides each consisting of any of an amino acid sequence of SEQ ID NO: 598, which is a partial sequence of GSTP1; an amino acid sequence of SEQ ID NO: 599, which is a partial sequence of GSTM4; amino acid sequences of SEQ ID NOS: 600 to 603, which are partial sequences of GSTA4; amino acid sequences of SEQ ID NOS: 604 and 605, which are partial sequences of GSTA3; amino acid sequences of SEQ ID NOS: 606 to 609, which are partial sequences of GSTM5; and amino acid sequences of SEQ ID NOS: 610 to 613, which are partial sequences of ST1E1, are also the peptides of the present invention. GSTP1, GSTM4, GSTA4, GSTA3, GSTM5, and ST1E1 can be quantified using any of the corresponding peptides.

Similarly, peptides each consisting of any of amino acid sequences of SEQ ID NOS: 614 and 615, which are partial sequences of ARY1; amino acid sequences of SEQ ID NOS: 616 to 620, which are partial sequences of ARY2; amino acid sequences of SEQ ID NOS: 621 to 624, which are partial sequences of ARY3; amino acid sequences of SEQ ID NOS: 625 and 626, which are partial sequences of ST1A1; amino acid sequences of SEQ ID NOS: 627 to 629, which are partial sequences of UD12; amino acid sequences of SEQ ID NOS: 630 to 638, which are partial sequences of CGT; amino acid sequences of SEQ ID NOS: 639 to 641, which are partial sequences of UD17C; and amino acid sequences of SEQ ID NOS: 642 and 643, which are partial sequences of ST1C1, are also the peptides of the present invention. ARY1, ARY2, ARY3, ST1A1, UD12, CGT, UD17C, and ST1C1 can be quantified using any of the corresponding peptides.

Similarly, peptides each consisting of any of amino acid sequences of SEQ ID NOS: 644 to 650, which are partial sequences of CHST2; an amino acid sequence of SEQ ID NO: 651, which is a partial sequence of MGST3; amino acid sequences of SEQ ID NOS: 652 to 655, which are partial sequences of ST1C2; and amino acid sequences of SEQ ID NOS: 656 to 659, which are partial sequences of GSTK1, are also the peptides of the present invention. CHST2, MGST3, ST1C2, and GSTK1 can be quantified using any of the corresponding peptides.

Similarly, peptides each consisting of any of amino acid sequences of SEQ ID NOS: 660 to 671, which are partial sequences of CHST7; amino acid sequences of SEQ ID NOS: 672 to 676, which are partial sequences of CHST1; amino acid sequences of SEQ ID NOS: 677 to 683, which are partial sequences of CHST5; amino acid sequences of SEQ ID NOS: 684 to 686, which are partial sequences of NAT6; amino acid sequences of SEQ ID NOS: 687 to 691, which are partial sequences of CHST4; and amino acid sequences of SEQ ID NOS: 692 to 695, which are partial sequences of MAAI, are also the peptides of the present invention. CHST7, CHST1, CHST5, NAT6, CHST4, and MAAI can be quantified using any of the corresponding peptides.

### (Peptides derived from homologues or peptides corresponding to human SNPs)

Examples of the peptide of the present invention can further include peptides for use in simultaneous protein quantification of metabolizing enzymes with a mass spectrometer, consisting of an amino acid sequence including deletion, substitution, or addition of one or two amino acids in any one of amino acid sequences of SEQ ID NOS: 1 to 695. Specific preferred examples of these peptides can include peptides derived from homologues (homologous proteins) of mammals other than humans or mice, consisting of an amino acid sequence including deletion, substitution, or addition of one or two (preferably one) amino acids in any one of amino acid sequences of SEQ ID NOS: 1 to 695, and peptides corresponding to SNPs in human metabolizing enzyme proteins, consisting of an amino acid sequence including deletion, substitution, or addition of one or two (preferably one) amino acids in any one of partial amino acid sequences of a human metabolizing enzyme protein set forth in SEQ ID NOS: 1 to 413. Examples of the above-mentioned mammals other than human or mouse can include primates other than human (for example, monkey, baboon, and chimpanzee), swine, bovine, equine, goat, sheep, dog, feline, rabbits, guinea pig, gerbil, hamster, and rat.

The above-mentioned peptides consisting of partial amino acid sequences of human metabolizing enzyme proteins, the above-mentioned peptides consisting of partial amino acid sequences of mouse metabolizing enzyme proteins, and the above-mentioned peptides derived from homologues or peptides corresponding to human SNPs (hereinafter, these peptides of the present invention may be collectively referred to as "unlabeled peptide of the invention") of the present invention can be produced by common chemical synthesis methods. As such a peptide synthesis method, a stepwise elongation method of extending a chain by binding amino acids one by one based on the amino acid sequence information or a fragment condensation method of synthesizing fragments consisting of several amino acids beforehand and then coupling the fragments can be employed.

### (Stable-isotope-labeled peptide)

Furthermore, when the labeled peptide of the present invention is a stable-isotope-labeled peptide in which one or more amino acids constituting the above-mentioned unlabeled peptide of the invention contain any one or more of ¹⁵N, ¹³C, ¹⁸O, and ²H, the type, the position, and the number of the amino acids are not particularly limited so long as the peptides have differences in mass that can be separated by a liquid chromatograph-tandem massspectrometer (LC-MS/M). Above all, leucine labeled with ¹³C at six sites is more preferably included. Such a stable-isotope-labeled peptide can be chemically synthesized by appropriate measures such as an F-moc method (Amblard M, Fehrentz JA, Martinez J, Subra G. Methods Mol Biol. 298: 3-24 (2005)) using amino acids labeled with a stable isotope. Furthermore, as a stable-isotope-labeled peptide is chemically identical to a peptide to be quantified and shows an identical behavior in LC-MS/MS measurement except that the mass of a labeled amino acid is different, the stable-isotope-labeled peptide can be used as an internal standard peptide in simultaneous protein quantification of metabolizing enzymes with a mass spectrometer.

### (Kit for quantification and use as probes)

The kit of the present invention for simultaneous protein quantification of metabolizing enzymes is not particularly limited so long as the kit comprises the unlabeled peptide of the invention and the stable-isotope-labeled peptide of the present invention. The using method of present invention is not particularly limited so long as the method uses the unlabeled peptide of the invention and the stable-isotope-labeled peptide of the present invention as probes for simultaneous protein quantification of metabolizing enzymes. The unlabeled peptide of the invention, which is also a peptide to be quantified, is used to create a calibration curve for quantification, and the stable-isotope-labeled peptide is used to create a calibration curve for quantification and as an internal standard peptide.

### (Simultaneous protein quantification of metabolizing enzymes)

The method for simultaneous protein quantification of metabolizing enzymes by LC-MS/MS using the stable-isotope-labeled peptide of the present invention is not particularly limited so long as the method comprises the steps of: (a) performing a mass spectrometry by LC-MS/MS using the unlabeled peptide of the invention and the stable-isotope-labeled peptide of the present invention at predetermined concentration levels to create a calibration curve; (b) performing a mass spectrometry by LC-MS/MS by adding the stable-isotope-labeled peptide of the present invention to peptide fragments obtained by fragmenting metabolizing enzyme proteins to be quantified in a sample with trypsin to calculate a mass spectrum area ratio of a metabolizing enzyme protein peptide to be quantified and a stable-isotope-labeled peptide; and (c) obtaining quantitative values from the area ratio using the calibration curve. Examples of the above-mentioned sample as a source of proteins to be quantified can include various tissues such as the liver and cultured cells derived from various tissues. Furthermore, when the amounts of proteins to be quantified contained in a sample are not more than the measurement limit of a mass spectrometer, a sample fractioned by a method of filling a high pressure nitrogen gas or the like is preferably used in measurement. Furthermore, when two or more peptides to be quantified are identified for one target metabolizing enzyme protein, simultaneous protein quantification of metabolizing enzymes at higher precision and reliability can be achieved by quantification using two or more peptides to be quantified.

Furthermore, when a protein to be quantified and a subtype thereof have very similar amino acid sequences, the protein to be quantified can also be quantified by obtaining a difference between a quantitative value of a peptide common to these proteins and a quantitative value of a peptide specific to one protein. For example, the quantitative value of a metabolizing protein CYP3A4 can be obtained by (a quantitative value of a peptide common to CYP3A4 and CYP3A5) - (a quantitative value of a peptide specific to CYP3A5).

Hereafter, the present invention will be specifically described with reference to the following Examples. However, the technical scope of the present invention is not limited to these examples.

### Example 1

### (Detection of peptides (CYP and P450R) by nanoLC-MS/MS)

Measurement by nanoLC-MS/MS was performed using peptides to be quantified, which are partial sequence peptides of human metabolizing enzymes CYP and P450R (unlabeled peptides: CYP1A2, YLPNPALQR (SEQ ID NO: 7) ; CYP2A6, GTGGANIDPTFFLSR (SEQ ID NO: 15) ; CYP2B6, GYGVIFANGNR (SEQ ID NO: 25) ; CYP2C8, GNSPISQR (SEQ ID NO: 28) ; CYP2C9, GIFPLAER (SEQ ID NO: 30) ; CYP2C18, IAENFAYIK (SEQ ID NO: 32); CYP2C19, GHFPLAER (SEQ ID NO: 35); CYP2D6, DIEVQGFR (SEQ ID NO: 39); CYP2E1, GIIFNNGPTWK (SEQ ID NO: 44); CYP2J2, EENGQPFDPHFK (SEQ ID NO: 52) ; CYP3A4, LQEEIDAVLPNK (SEQ ID NO: 81); CYP3A5, DTINFLSK (SEQ ID NO: 83); CYP3A7, FNPLDPFVLSIK (SEQ ID NO: 85); CYP3A43, YIPFGAGPR (SEQ ID NO: 91); CYP4A11, IPIPIAR (SEQ ID NO: 100); CYP51A1, FAYVPFGAGR (SEQ ID NO: 290); and P450R, FAVFGLGNK (SEQ ID NO: 355)) and synthesized stable-isotope-labeled peptides [isotope-labeled peptides: CYP1A2, YLPNPAL(¹³C₆,¹⁵N)QR (SEQ ID NO: 7); CYP2A6, GTGGANIDPTFFL(¹³C₆,¹⁵N)SR (SEQ ID NO: 15); CYP2B6, GYGVIFA(¹³C₆,¹⁵N)NGNR (SEQ ID NO: 25); CYP2C8, GNSPI(¹³C₆,¹⁵N)SQR (SEQ ID NO: 28); CYP2C9, GIFPL(¹³C₆,¹⁵N)AER (SEQ ID NO: 30); CYP2C18, IAENFAYI (¹³C₆,¹⁵N)K (SEQ ID NO: 32); CYP2C19, GHFPL(¹³C₆,¹⁵N)AER (SEQ ID NO: 35); CYP2D6, DIEVQGF(¹³C₆,¹⁵N)R (SEQ ID NO: 39); CYP2E1, GIIFNNGP (¹³C₆,¹⁵N) TWK (SEQ ID NO: 44); CYP2J2, EENGQPFDPHF(¹³C₆,¹⁵N)K (SEQ ID NO: 52); CYP3A4, LQEEIDAVLP(¹³C₆,¹⁵N)NK (SEQ ID NO: 81); CYP3A5, DTINFL (¹³C₆,¹⁵N)SK (SEQ ID NO: 83); CYP3A7, FNPLDPFVLSI (¹³C₆,¹⁵N) K (SEQ ID NO: 85) ; CYP3A43, YIPFGAGP(¹³C₆,¹⁵N)R (SEQ ID NO: 91); CYP4A11, IPIPIA(¹³C₆,¹⁵N)R (SEQ ID NO: 100) ; CYP51A1, FAYVPFGA(¹³C₆,¹⁵N)GR (SEQ ID NO: 290) ; and P450R, FAVFGL (¹³C₆,¹⁵N)GNK (SEQ ID NO: 355)] under the following conditions. 50 fmol each of ¹³C₆,¹⁵N-labeled peptides were added to 1 fmol of unlabeled peptides for measurement by nanoLC-MS/MS. A reverse phase C18 150 µm I.D. x 40 mm was used as a column. 0.1% formic acid and 0.1% formic acid/acetonitrile were used as mobile phases. Analysis was performed with a linear gradient of increasing the concentration of 0.1% formic acid/acetonitrile to 45% over 35 minutes. 4000 Q Trap of Applied Biosystems was used as a mass spectrometer. A peptide was identified when a peptide to be quantified and a labeled peptide were eluted with the same time. The results are shown in Figure 1.

### Example 2

### (Simultaneous quantification of CYP and P450R in human liver tissue sample)

A human liver tissue was shredded with scissors and then suspended in 10 mM Tris-HCl, 10 mM NaCl, and 1.5 mM MgCl₂. The suspension was homogenized with a glass homogenizer, and the solution was centrifuged at 8000 x g for 10 minutes. Further, the supernatant was centrifuged at 100,000 x g for 60 minutes to obtain a crude membrane fraction. The obtained crude membrane fraction was denatured in a buffer containing 7 M guanidine hydrochloride, 0.1 M Tris-HCl, and 10 mM EDTA (pH 8.5) and subjected to reduction with DTT and carbamide methylation with iodoacetamide to protect the SH group of a cysteine residue. Concentration and desalting were performed by a methanol chloroform precipitation method. The fraction was suspended in 1.2 M urea and 10 mM Tris-HCl, then trypsin was added in an amount corresponding to 1/100 of the protein weight, and proteins were digested with the enzyme at 37°C for 16 hours to obtain a peptide sample.

50 fmol of ¹³C₆,¹⁵N-labeled peptides were added to 5 µg of the obtained crude membrane fraction peptide sample, and peptides were measured by LC-MS/MS (Figure 2). After the measurement, MS spectrum area ratios (unlabeled peptide/¹³C₆,¹⁵N-labeled peptide) were calculated, and quantitative values were obtained using a calibration curve.

### (Creation of calibration curve)

A calibration curve was created using a selected peptide to be quantified to examine linearity. 50 fmol each of ¹³C₆,¹⁵N-labeled peptides were added to each of 1, 5, 10, 50, and 100 fmol of an unlabeled peptide, and peptides were similarly measured by nanoLC-MS/MS. MS spectrum area ratios (unlabeled peptide/¹³C₆,¹⁵N-labeled peptide) were calculated to create a calibration curve.

The quantification results CYP and P450R are shown in Table 1.

**[Table 1]**

| Metabolizing enzyme CYPorP450R | Quantitative value (fmol/assay) |
|---|---|
| CYP1A2 | 40.8 |
| CYP2A6 | 70.0 |
| CYP2B6 | 2.50 |
| CYP2C9 | 435 |
| CYP2C18 | 5.00 |
| CYP2C19 | 21.1 |
| CYP2D6 | 39.1 |
| CYP2E1 | 33.9 |
| CYP3A4 | 95.1 |
| CYP3A5 | 6.50 |
| CYP3A43 | 89.0 |
| CYP4A11 | 13.5 |
| CYP51A1 | 11.3 |
| P450R | 100 |

### Example 3

### (Detection of peptides (Ara-C metabolizing enzymes) by conventional LC-MS/MS)

Measurement by conventional LC-MS/MS was performed using peptides to be quantified, which are partial sequence peptides of human Ara-C metabolizing enzymes, (unlabeled peptides: dCK, AQLASLNGK (SEQ ID NO: 362); CDA, AVSEGYK (SEQ ID NO: 364) ; cN-IA, GFLEALGR (SEQ ID NO: 366); cN-IB, GFLEDLGR (SEQ ID NO: 369); cN-II, VFLATNSDYK (SEQ ID NO: 375); cN-III, GELIHVFNK (SEQ ID NO: 379); dNT-1, TVVLGDLLIDDK (SEQ ID NO: 383); Ecto-5'-NT, VPSYDPLK (SEQ ID NO: 388); RRM1, LNSAIIYDR (SEQ ID NO: 390); RRM2, ENTPPALSGTR (SEQ ID NO: 393); UMP/CMPK, FLIDGFPR (SEQ ID NO: 395); dCMPDA, LIIQAGIK (SEQ ID NO: 396); CTPS1, FVGQDVEGER (SEQ ID NO: 402); CTPS2, ADGILVPGGFGIR (SEQ ID NO: 409)) and synthesized stable-isotope-labeled peptides [isotope-labeled peptides: dCK, AQLASL (¹³C₆,¹⁵N) NGK (SEQ ID NO: 362); CDA, AV(¹³C₆,¹⁵N)SEGYK (SEQ ID NO: 364); cN-IA, GFLEAL(¹³C₆,¹⁵N)GR (SEQ ID NO: 366); cN-IB, GFLEDL(¹³C₆,¹⁵N)GR (SEQ ID NO: 369); cN-II, VFLA(¹³C₆,¹⁵N)TNSDYK (SEQ ID NO: 375); cN-III, GELIHVF(¹³C₆,¹⁵N)NK (SEQ ID NO: 379); dNT-1, TVVLGDLLI(¹³C₆,¹⁵N) DDK (SEQ ID NO: 383); Ecto-5' -NT, VPSYDPL(¹³C₆,¹⁵N)K (SEQ ID NO: 388); RRM1, LNSAII (¹³C₆,¹⁵N) YDR (SEQ ID NO: 390); RRM2, ENTPPAL(¹³C₆,¹⁵N)SGTR (SEQ ID NO: 393) ; UMP/CMPK, FLIDGFP(¹³C₆,¹⁵N)R (SEQ ID NO: 395); dCMPDA, LIIQAGI(¹³C₆,¹⁵N) K (SEQ ID NO: 396) ; CTPS1, FVGQDV (¹³C₆,¹⁵N) EGER (SEQ ID NO: 402); and CTPS2, ADGILVPGGFGI(¹³C₆,¹⁵N)R (SEQ ID NO: 409)] under the following conditions.

500 fmol each of ¹³C₆,¹⁵N-labeled peptides were added to 10 fmol of unlabeled peptides for measurement by conventional LC-MS/MS. A reverse phase C18 0.5 mm I.D. x 150 mm was used as a column. 0.1% formic acid and 0.1% formic acid/acetonitrile were used as mobile phases. Analysis was performed with a linear gradient of increasing the concentration of 0.1% formic acid/acetonitrile to 45% over 60 minutes. API 5000 of Applied Biosystems was used as a mass spectrometer. A peptide was identified when a peptide to be quantified and a labeled peptide are eluted with the same time. The results are shown in Figure 7.

### Example 4

### (Simultaneous quantification of Ara-C metabolizing proteins in human cultured cells)

5.0 x 10⁸ cells of human leukemic cell strain K562 were cultured in an RPMI-1640 medium containing 10% FBS and then crushed by a cavitation method. This solution was centrifuged at 10,000 x g for 10 minutes. Then, the supernatant was centrifuged at 100,000 x g for 60 minutes to obtain a soluble fraction. The obtained soluble fraction was denatured in a buffer containing 7 M guanidine hydrochloride, 0.1 M Tris-HCl, and 10 mM EDTA (pH 8.5) and subjected to reduction with DTT and carbamide methylation with iodoacetamide to protect the SH group of a cysteine residue. Concentration and desalting were performed by a methanol chloroform precipitation method. The fraction was suspended in 1.2 M urea and 10 mM Tris-HCl, then trypsin was added in an amount of 1/100 corresponding to the protein weight, and proteins were digested with the enzyme at 37°C for 16 hours to obtain a peptide sample.

500 fmol of ¹³C₆,¹⁵N-labeled peptides were added to 50 µg of the obtained crude membrane fraction peptide sample and measured by LC-MS/MS. After measurement, MS spectrum area ratios (¹³C₆,¹⁵N-labeled peptide) were calculated, and quantitative values were obtained using the calibration curve.

### (Creation of calibration curve)

A calibration curve was created using a selected peptide to be quantified. 500 fmol each of ¹³C₆,¹⁵N-labeled peptides were added to 1, 5, 10, 50, 100, 500, and 1000 fmol of an unlabeled peptide and measured by conventional LC-MS/MS in the same manner as in Example 1. The MS spectrum area ratios (unlabeled peptide/¹³C₆,¹⁵N-labeled peptide) were calculated to create a calibration curve.

The results of Ara-C metabolizing enzyme quantification are shown in Table 2.

**[Table 2]**

| Ara-C-related metabolizing enzyme | Quantitative value (fmol/assay) |
|---|---|
| dCK | 73.5 |
| cN-II | 89.9 |
| RRM1 | 195 |
| UMP/CMPK | 544 |
| dCMPDA | 334 |
| CTPS1 | 216 |
| CTPS2 | 104 |

### [Comparative Example 1]

LC-MS/MS measurement was performed under the following conditions using a peptide to be quantified (unlabeled peptide: ENT2, VALTLDLDLEK (SEQ ID NO: 696)), which is a partial sequence peptide of a human transporter ENT2 (SLC29A2), and a synthesized stable-isotope-labeled peptide [isotope-labeled peptide: ENT2, VALTLDLDL(¹³C₆,¹⁵N)EK (SEQ ID NO: 696)], but the peptide could not be quantified due to the low detection sensitivity.

500 fmol of the ¹³C₆,¹⁵N-labeled peptide was added to 500 fmol of the unlabeled peptide and measured by conventional LC-MS/MS. A reverse phase C18 0.5 mm I.D. x 150 mm was used as a column. 0.1% formic acid and 0.1% formic acid/acetonitrile were used as mobile phases. Analysis was performed with a linear gradient of increasing the concentration of 0.1% formic acid/acetonitrile to 45% over 60 minutes. API 5000 of Applied Biosystems was used as a mass spectrometer. The peptide was identified under a condition that the peptide to be quantified and the labeled peptide were eluted with the same time, but no peaks were detected for either the peptide to be quantified or the labeled peptide due to the low sensitivity.

### [Comparative Example 2]

LC-MS/MS measurement was performed under the following conditions using a peptide to be quantified (unlabeled peptide: BGT1, QELIAWEK (SEQ ID NO: 697)), which is a partial sequence peptide of a human transporter BGT1 (SLC6A12), and a synthesized stable-isotope-labeled peptide [isotope-labeled peptide: BGT1, QELIA(¹³C₆,¹⁵N)WEK (SEQ ID NO: 697)], but the peptide could not be quantified because peptides were not retained on the reverse phase column and therefore the sensitivity was low.

500 fmol of the ¹³C₆,¹⁵N-labeled peptide was added to 500 fmol of the unlabeled peptide and measured by conventional LC-MS/MS. A reverse phase C18 0.5 mm I.D. x 150 mm was used as a column. 0.1% formic acid and 0.1% formic acid/acetonitrile were used as mobile phases. Analysis was performed with a linear gradient of increasing the concentration of 0.1% formic acid/acetonitrile to 45% over 60 minutes. API 5000 of Applied Biosystems was used as a mass spectrometer. The peptide was identified under a condition that the peptide to be quantified and the labeled peptide were eluted with the same time, but no peaks were detected for either the peptide to be quantified or the labeled peptide.

## Claims

1. A method for simultaneous protein quantification of two or more of human metabolizing enzymes with a liquid chromatograph-tandem mass spectrometer (LC-MS/MS) using stable-isotope-labeled peptides, comprising the following steps (a) to (c):
(a) performing a mass spectrometry by LC-MS/MS using unlabeled peptides each consisting of an amino acid sequence set forth in any of SEQ ID NOS : 1 to 412, which are partial amino acid sequences of the human metabolizing enzymes, and stable-isotope-labeled peptides having the same amino acid sequence as the unlabeled peptides, wherein one or more peptide-constituting amino acids contain any one or more of ¹⁵N, ¹³C, ¹⁸O, and ²H in the stable-isotope-labeled peptides at respective predetermined concentration levels to create a calibration curve;
(b) performing a mass spectrometry by LC-MS/MS by adding the stable-isotope-labeled peptides to a peptide fragment obtained by fragmenting metabolizing enzyme proteins to be quantified in a sample with trypsin to calculate a mass spectrum area ratio of a metabolizing enzyme protein peptide to be quantified to the stable-isotope-labeled peptide; and
(c) calculating a quantitative value from the area ratio using the calibration curve,
wherein the human metabolizing enzymes are two or more selected from CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2W1, CYP3A4, CYP3A5, CYP3A7, CYP3A43, CYP4A11, CYP4B1, CYP4F2, CYP4F3, CYP4F8, CYP4F11, CYP4F12, CYP4F22, CYP4V2, CYP4X1, CYP4Z1, CYP7A1, CYP7B1, CYP8B1, CYP11B1, CYP11B2, CYP17A1, CYP19A1, CYP21A2, CYP24A1, CYP26A1, CYP26B1, CYP26C1, CYP27A1, CYP27B1, CYP27C1, CYP39A1, CYP46A1, CYP51A1, UGT1A1, UGT1A3, UGT1A4, UGT1A5, UGT1A6, UGT1A7, UGT1A10, UGT2A1, UGT2B4, UGT2B7, UGT2B10, UGT2B11, UGT2B17, UGT2B28, GSTA1, GSTA2, GSTA3, GSTA4, GSTA5, GSTK1, GSTM1, GSTM2, GSTM3, GSTM4, GSTM5, GSTP1, GSTT1, GSTT2, MGST1, MGST2, MGST3, SULT1A2, SULT1A3, SULT1B1, SULT1C2, SULT1C3, SULT1C4, SULT1E1, SULT2A1, SULT2B1, SULT4A1, SULT4S6, P450R, MGMT, dCK, CDA, cN-IA, cN-IB, cN-II, cN-III, dNT-1, dNT-2, Ecto-5' -NT, RRM1, RRM2, UMP/CMPK, dCMPDA, CTP synthasel, and CTP synthase2.

2. A kit for the method according to claim 1, comprising peptides described in the following (i) and (ii):
(i) unlabeled peptides each consisting of an amino acid sequence set forth in any of SEQ ID NOS: 1 to 412, which are partial amino acid sequences of the human metabolizing enzymes;
(ii) stable-isotope-labeled peptides wherein one or more of peptide-constituting amino acids contain any one or more of ¹⁵N, ¹³C, ¹⁸O, any ²H in the peptides of (i) .

3. A method for simultaneous protein quantification of two or more of mouse metabolizing enzymes with a liquid chromatograph-tandem mass spectrometer (LC-MS/MS) using stable-isotope-labeled peptides, comprising the following steps (a) to (c):
(a) performing a mass spectrometry by LC-MS/MS using unlabeled peptides each consisting of an amino acid sequence set forth in any of SEQ ID NOS: 413 to 695, which are partial amino acid sequences of the mouse metabolizing enzymes, and stable-isotope-labeled peptides having the same amino acid sequence as the unlabeled peptides, wherein one or more of peptide-constituting amino acids contain any one or more of ¹⁵N, ¹³C, ¹⁸O, and ²H in the stable-isotope-labeled peptides at respective predetermined concentration levels to create a calibration curve;
(b) performing a mass spectrometry by LC-MS/MS by adding the stable-isotope-labeled peptides to a peptide fragment obtained by fragmenting metabolizing enzyme proteins to be quantified in a sample with trypsin to calculate a mass spectrum area ratio of a metabolizing enzyme protein peptide to be quantified to the stable-isotope-labeled peptide; and
(c) calculating a quantitative value from the area ratio using the calibration curve,
wherein the mouse metabolizing enzymes are two or more selected from Cyp11a1, Cyp17a1, Cyp19a1, Cyp1a1, Cyp1a2, Cyp21, Cyp24a1, Cyp26a1, Cyp27a1, Cyp27b1, Cyp2a4, Cyp2a5, Cyp2b19, Cyp2c29, Cyp2c39, Cyp2c70, Cyp2d10, Cyp2d26, Cyp2d9, Cyp2e1, Cyp2f2, Cyp2j5, Cyp2r1, Cyp2s1, Cyp39a1, Cyp3a11, Cyp3a13, Cyp3a16, Cyp3a25, Cyp46a1, Cyp4a14, Cyp4b1, Cyp4f14, Cyp4v3, Cyp5a, Cyp8b1, GSTO1, ST2B1, ST3A1, CHST3, SNAT, UD2B5, GSTP1, GSTM4, GSTA4, GSTA3, GSTM5, ST1E1, ARY1, ARY2, ARY3, ST1A1, UD12, CGT, UD17C, ST1C1, CHST2, MGST3, ST1C2, GSTK1, CHST7, CHST1, CHST5, NAT6, CHST4, and MAAI.

4. A kit for the method according to claim 3, comprising peptides described in the following (i) and (ii):
(i) peptides each consisting of an amino acid sequence set forth in any of SEQ ID NOS: 413 to 695 which are partial amino acid sequences of the mouse metabolizing enzymes;
(ii) stable-isotope-labeled peptides wherein one or more of peptide-constituting amino acids contain any one or more of ¹⁵N, ¹³C, ¹⁸O, any ²H in the peptides of (i) .

## Patentansprüche

1. Verfahren zur simultanen Proteinquantifizierung von zwei oder mehr Enzymen des menschlichen Stoffwechsels mit einem LC-MS/MS (Liquid Chromatograph-Tandem Mass Spectrometer) unter Verwendung mit stabilen Isotopen markierter Peptide, umfassend die folgenden Schritte (a) bis (c):
(a) Durchführen einer Massenspektrometrie mit LC-MS/MS unter Verwendung unmarkierter Peptide, die jeweils aus einer Aminosäuresequenz gemäß einer der SEQ ID NO: 1 bis 412, bei denen es sich um Aminosäureteilsequenzen der Enzyme des menschlichen Stoffwechsels handelt, bestehen, und mit stabilen Isotopen markierter Peptide mit der gleichen Aminosäuresequenz wie die unmarkierten Peptide, wobei eine oder mehrere peptidbildende Aminosäuren ein oder mehrere von ¹⁵N, ¹³C, ¹⁸O und ²H in den mit stabilen Isotopen markierten Peptiden bei jeweiligen vorbestimmten Konzentrationsniveaus enthalten, um eine Eichkurve zu erstellen;
(b) Durchführen einer Massenspektrometrie mit LC-MS/MS durch Zugeben der mit stabilen Isotopen markierten Peptide zu einem Peptidfragment, das durch Fragmentieren von zu quantifizierenden Stoffwechselenzym-Proteinen in einer Probe mit Trypsin erhalten wurde, so dass ein Massenspektrum-Flächenverhältnis eines zu quantifizierenden Stoffwechselenzym-Protein-Peptids zum mit stabilen Isotopen markierten Peptid berechnet wird; und
(c) Berechnen eines quantitativen Werts aus dem Flächenverhältnis unter Verwendung der Eichkurve, wobei es sich bei den Enzymen des menschlichen Stoffwechsels um zwei oder mehr aus CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2W1, CYP3A4, CYP3A5, CYP3A7, CYP3A43, CYP4A11, CYP4B1, CYP4F2, CYP4F3, CYP4F8, CYP4F11, CYP4F12, CYP4F22, CYP4V2, CYP4X1, CYP4Z1, CYP7A1, CYP7B1, CYP8B1, CYP11B1, CYP11B2, CYP17A1, CYP19A1, CYP21A2, CYP24A1, CYP26A1, CYP26B1, CYP26C1, CYP27A1, CYP27B1, CYP27C1, CYP39A1, CYP46A1, CYP51A1, UGT1A1, UGT1A3, UGT1A4, UGT1A5, UGT1A6, UGT1A7, UGT1A10, UGT2A1, UGT2B4, UGT2B7, UGT2B10, UGT2B11, UGT2B17, UGT2B28, GSTA1, GSTA2, GSTA3, GSTA4, GSTA5, GSTK1, GSTM1, GSTM2, GSTM3, GSTM4, GSTM5, GSTP1, GSTT1, GSTT2, MGST1, MGST2, MGST3, SULT1A2, SULT1A3, SULT1B1, SULT1C2, SULT1C3, SULT1C4, SULT1E1, SULT2A1, SULT2B1, SULT4A1, SULT4S6, P450R, MGMT, dCK, CDA, cN-IA, cN-IB, cN-II, cN-III, dNT-1, dNT-2, Ecto-5'-NT, RRM1, RRM2, UMP/CMPK, dCMPDA, CTP-Synthase 1 und CTP-Synthase 2 ausgewählte Enzyme handelt.

2. Kit für das Verfahren nach Anspruch 1, umfassend im Folgenden unter (i) und (ii) beschriebene Peptide:
(i) unmarkierte Peptide, die jeweils aus einer Aminosäuresequenz gemäß einer der SEQ ID NO: 1 bis 412, bei denen es sich um Aminosäureteilsequenzen der Enzyme des menschlichen Stoffwechsels handelt, bestehen;
(ii) mit stabilen Isotopen markierte Peptide, wobei eine oder mehrere peptidbildende Aminosäuren ein oder mehrere von ¹⁵N, ¹³C, ¹⁸O und ²H in den Peptiden unter (i) enthalten.

3. Verfahren zur simultanen Proteinquantifizierung von zwei oder mehr Enzymen des Maus-Stoffwechsels mit einem LC-MS/MS (Liquid Chromatograph-Tandem Mass Spectrometer) unter Verwendung mit stabilen Isotopen markierter Peptide, umfassend die folgenden Schritte (a) bis (c):
(a) Durchführen einer Massenspektrometrie mit LC-MS/MS unter Verwendung unmarkierter Peptide, die jeweils aus einer Aminosäuresequenz gemäß einer der SEQ ID NO: 413 bis 695, bei denen es sich um Aminosäureteilsequenzen der Enzyme des Maus-Stoffwechsels handelt, bestehen, und mit stabilen Isotopen markierter Peptide mit der gleichen Aminosäuresequenz wie die unmarkierten Peptide, wobei eine oder mehrere peptidbildende Aminosäuren ein oder mehrere von ¹⁵N, ¹³C, ¹⁸O und ²H in den mit stabilen Isotopen markierten Peptiden bei jeweiligen vorbestimmten Konzentrationsniveaus enthalten, um eine Eichkurve zu erstellen;
(b) Durchführen einer Massenspektrometrie mit LC-MS/MS durch Zugeben der mit stabilen Isotopen markierten Peptide zu einem Peptidfragment, das durch Fragmentieren von zu quantifizierenden Stoffwechselenzym-Proteinen in einer Probe mit Trypsin erhalten wurde, so dass ein Massenspektrum-Flächenverhältnis eines zu quantifizierenden Stoffwechselenzym-Protein-Peptids zum mit stabilen Isotopen markierten Peptid berechnet wird; und
(c) Berechnen eines quantitativen Werts aus dem Flächenverhältnis unter Verwendung der Eichkurve, wobei es sich bei den Enzymen des Maus-Stoffwechsels um zwei oder mehr aus Cyp11a1, Cyp17a1, Cyp19al, Cyp1a1, Cyp1a2, Cyp21, Cyp24a1, Cyp26a1, Cyp27a1, Cyp27b1, Cyp2a4, Cyp2a5, Cyp2b19, Cyp2c29, Cyp2c39, Cyp2c70, Cyp2d10, Cyp2d26, Cyp2d9, Cyp2e1, Cyp2f2, Cyp2j5, Cyp2r1, Cyp2s1, Cyp39a1, Cyp3a11, Cyp3a13, Cyp3a16, Cyp3a25, Cyp46a1, Cyp4a14, Cyp4b1, Cyp4f14, Cyp4v3, Cyp5a, Cyp8b1 GST01, ST2B1, ST3A1, CHST3, SNAT, UD2B5, GSTP1, GSTM4, GSTA4, GSTA3, GSTM5, ST1E1, ARY1, ARY2, ARY3, ST1A1, UD12, CGT, UD17C, ST1C1, CHST2, MGST3, ST1C2, GSTK1, CHST7, CHST1, CHST5, NAT6, CHST4 und MAAI ausgewählte Enzyme handelt.

4. Kit für das Verfahren nach Anspruch 3, umfassend im Folgenden unter (i) und (ii) beschriebene Peptide:
(i) Peptide, die jeweils aus einer Aminosäuresequenz gemäß einer der SEQ ID NO: 413 bis 695, bei denen es sich um Aminosäureteilsequenzen der Enzyme des Maus-Stoffwechsels handelt, bestehen;
(ii) mit stabilen Isotopen markierte Peptide, wobei eine oder mehrere peptidbildende Aminosäuren ein oder mehrere von ¹⁵N, ¹³C, ¹⁸O und ²H in den Peptiden unter (i) enthalten.

## Revendications

1. Procédé de quantification protéique simultanée de deux ou plusieurs enzymes métabolisantes humaines avec un spectromètre de masse couplé à la chromatographie liquide (LC-MS/MS), en utilisant des peptides marqués par des isotopes stables, comprenant les étapes (a) à (c) suivantes :
(a) exécuter une spectrométrie de masse par LC-MS/MS en utilisant des peptides non marqués, constitués chacun par une séquence d'acides aminés indiquée dans l'une quelconque des SEQ ID n° : 1 à 412, qui sont des séquences d'acides aminés partielles des enzymes métabolisantes humaines, ainsi que des peptides marqués par des isotopes stables ayant la même séquence d'acides aminés que les peptides non marqués, dans lequel un ou plusieurs acide(s) aminé(s) constituant les peptides contient (contiennent) un quelconque élément parmi les ¹⁵N, ¹³C, ¹⁸O et ²H ou plusieurs d'entre eux dans les peptides marqués par des isotopes stables à des niveaux de concentration prédéterminés respectifs, pour créer une courbe d'étalonnage ;
(b) exécuter une spectrométrie de masse par LC-MS/MS en ajoutant les peptides marqués par des isotopes stables à un fragment peptidique, obtenu en fragmentant avec de la trypsine des protéines d'enzymes métabolisantes à quantifier dans un échantillon, pour calculer un rapport des surfaces de spectre de masse d'un peptide de protéine d'enzyme métabolisante à quantifier au peptide marqué par un isotope stable ; et
(c) calculer une valeur quantitative à partir du rapport des surfaces en utilisant la courbe d'étalonnage,
dans lequel les enzymes métabolisantes humaines sont deux ou plusieurs choisies parmi les CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2W1, CYP3A4, CYP3A5, CYP3A7, CYP3A43, CYP4A11, CYP4B1, CYP4F2, CYP4F3, CYP4F8, CYP4F11, CYP4F12, CYP4F22, CYP4V2, CYP4X1, CYP4Z1, CYP7A1, CYP7B1, CYP8B1, CYP11B1, CYP11B2, CYP17A1, CYP19A1, CYP21A2, CYP24A1, CYP26A1, CYP26B1, CYP26C1, CYP27A1, CYP27B1, CYP27C1, CYP39A1, CYP46A1, CYP51A1, UGT1A1, UGT1A3, UGT1A4, UGT1A5, UGT1A6, UGT1A7, UGT1A10, UGT2A1, UGT2B4, UGT2B7, UGT2B10, UGT2B11, UGT2B17, UGT2B28, GSTA1, GSTA2, GSTA3, GSTA4, GSTA5, GSTK1, GSTM1, GSTM2, GSTM3, GSTM4, GSTM5, GSTP1, GSTT1, GSTT2, MGST1, MGST2, MGST3, SULT1A2, SULT1A3, SULT1B1, SULT1C2, SULT1C3, SULT1C4, SULT1E1, SULT2A1, SULT2B1, SULT4A1, SULT4S6, P450R, MGMT, dCK, CDA, cN-IA, cN-IB, cN-II, cN-III, dNT-1, dNT-2, Ecto-5' -NT, RRM1, RRM2, UMP/CMPK, dCMPDA, CTP synthase1 et CTP synthase2.

2. Trousse destinée au procédé selon la revendication 1, comprenant des peptides décrits dans les paragraphes (i) et (ii) suivantes :
(i) des peptides non marqués constitués chacun par une séquence d'acides aminés indiquée dans l'une quelconque des SEQ ID n° : 1 à 412, qui sont des séquences d'acides aminés partielles des enzymes métabolisantes humaines ;
(ii) des peptides marqués par des isotopes stables, dans lesquels un ou plusieurs des acides aminés constituant les peptides contient (contiennent) un quelconque élément parmi les ¹⁵N, ¹³C, ¹⁸O et ²H ou plusieurs d'entre eux dans les peptides du paragraphe (i) .

3. Procédé de quantification protéique simultanée de deux ou plusieurs enzymes métabolisantes de souris avec un spectromètre de masse couplé à la chromatographie liquide (LC-MS/MS), en utilisant des peptides marqués par des isotopes stables, comprenant les étapes (a) à (c) suivantes :
(a) exécuter une spectrométrie de masse par LC-MS/MS en utilisant des peptides non marqués, constitués chacun par une séquence d'acides aminés indiquée dans l'une quelconque des SEQ ID n° : 413 à 695, qui sont des séquences d'acides aminés partielles des enzymes métabolisantes de souris, ainsi que des peptides marqués par des isotopes stables ayant la même séquence d'acides aminés que les peptides non marqués, dans lequel un ou plusieurs des acides aminés constituant les peptides contient (contiennent) un quelconque élément parmi les ¹⁵N, ¹³C, ¹⁸O et ²H ou plusieurs d'entre eux dans les peptides marqués par des isotopes stables à des niveaux de concentration prédéterminés respectifs, pour créer une courbe d'étalonnage ;
(b) exécuter une spectrométrie de masse par LC-MS/MS en ajoutant les peptides marqués par des isotopes stables à un fragment peptidique, obtenu en fragmentant avec de la trypsine des protéines d'enzymes métabolisantes à quantifier dans un échantillon, pour calculer un rapport des surfaces de spectre de masse d'un peptide de protéine d'enzyme métabolisante à quantifier au peptide marqué par un isotope stable ; et
(c) calculer une valeur quantitative à partir du rapport des surfaces en utilisant la courbe d'étalonnage, dans lequel les enzymes métabolisantes de souris sont deux ou plusieurs choisies parmi les Cyp11a1, Cyp17a1, Cyp19a1, Cyp1a1, Cyp1a2, Cyp21, Cyp24a1, Cyp26a1, Cyp27a1 Cyp27b1, Cyp2a4, Cyp2a5, Cyp2b19, Cyp2c29, Cyp2c39, Cyp2c70, Cyp2d10, Cyp2d26, Cyp2d9, Cyp2e1, Cyp2f2, Cyp2j5, Cyp2r1, Cyp2s1, Cyp39a1, Cyp3a11, Cyp3a13, Cyp3a16, Cyp3a25, Cyp46a1, Cyp4a14, Cyp4b1, Cyp4f14, Cyp4v3, Cyp5a, Cyp8b1, GSTO1, ST2B1, ST3A1, CHST3, SNAT, UD2B5, GSTP1, GSTM4, GSTA4, GSTA3, GSTM5, ST1E1, ARY1, ARY2, ARY3, ST1A1, UD12, CGT, UD17C, ST1C1, CHST2, MGST3, ST1C2, GSTK1, CHST7, CHST1, CHST5, NAT6, CHST4 et MAAI.

4. Trousse destinée au procédé selon la revendication 3, comprenant des peptides décrits dans les paragraphes (i) et (ii) suivantes :
(i) des peptides constitués chacun par une séquence d'acides aminés indiquée dans l'une quelconque des SEQ ID n° : 413 à 695, qui sont des séquences d'acides aminés partielles des enzymes métabolisantes de souris ;
(ii) des peptides marqués par des isotopes stables, dans lesquels un ou plusieurs des acides aminés constituant les peptides contient (contiennent) un quelconque élément parmi les ¹⁵N, ¹³C, ¹⁸O et ²H ou plusieurs d'entre eux dans les peptides du paragraphe (i) .
